(19) Europäisches Patentamt
European Patent Office
Office européen des brevets

(11) EP 1 392 803 B1

(12) # EUROPEAN PATENT SPECIFICATION

(45) Date of publication and mention of the grant of the patent:
**07.02.2007 Bulletin 2007/06**

(21) Application number: **02728057.7**

(22) Date of filing: **15.05.2002**

(51) Int Cl.:
***C10M 105/38*** *(2006.01)* ***C10M 169/04*** *(2006.01)*
***G01N 33/28*** *(2006.01)* ***G11B 25/04*** *(2006.01)*
***F16C 33/10*** *(2006.01)*

(86) International application number:
**PCT/JP2002/004679**

(87) International publication number:
**WO 2002/092732 (21.11.2002 Gazette 2002/47)**

(54) **LUBRICANT COMPOSITION for spindle motor**

SCHMIERMITTELZUSAMMENSETZUNG für Spindelmotor

COMPOSITION LUBRIFIANTE pour moteur à broche

(84) Designated Contracting States:
**DE FR GB**

(30) Priority: **15.05.2001 JP 2001144405**
**15.05.2001 JP 2001144406**
**05.11.2001 JP 2001339115**
**05.11.2001 JP 2001339116**

(43) Date of publication of application:
**03.03.2004 Bulletin 2004/10**

(73) Proprietor: **MATSUSHITA ELECTRIC INDUSTRIAL CO., LTD.**
**Kadoma-shi, Osaka 571-8501 (JP)**

(72) Inventors:
- **TOJOU, Fumiyo**
**Gose-shi, Nara 639-2304 (JP)**
- **MATSUOKA, Kaoru**
**Osaka-shi,**
**Osaka 353-0013 (JP)**

(74) Representative: **TER MEER - STEINMEISTER & PARTNER GbR**
**Patentanwälte,**
**Mauerkircherstrasse 45**
**81679 München (DE)**

(56) References cited:
**EP-A- 0 485 979** **EP-A- 0 913 457**
**WO-A-97/16827** **DE-A- 2 152 666**
**FR-A- 2 774 286** **GB-A- 2 063 909**
**US-A- 5 973 878**

- **BARNES; FAINMAN: "Synthetic ester lubricants" LUBRICATION ENGINEERING, vol. 13, August 1957 (1957-08), pages 454-457, XP001098167**
- **PATENT ABSTRACTS OF JAPAN vol. 2002, no. 03, 3 April 2002 (2002-04-03) & JP 2001 316687 A (JAPAN ENERGY CORP;NIPPON DENSAN CORP), 16 November 2001 (2001-11-16)**
- **ABE Y., SHIBAYAMA M.; MATSUO T: "Characterization of lubricant films on magnetic recording disks by ToF-SIMS" SURFACE AND INTERFACE ANALYSIS, vol. 30, no. 1, August 2000 (2000-08), pages 632-634, XP009001825**
- **MATSUOKA KAORU ET AL: "Development of FDB spindle motors for HDD use" IEEE TRANS MAGN;IEEE TRANSACTIONS ON MAGNETICS MARCH 2001, vol. 37, no. 2 I, March 2001 (2001-03), pages 783-788, XP002223134**
- **CAMPANA J E ET AL: "ADVANCED MASS SPECTOMETRY OF LUBRICANTS" ASLE TRANSACTIONS, SOCIETY OF TRIBOLOGISTS AND LUBRICATION, US, vol. 29, no. 2, April 1986 (1986-04), pages 235-245, XP001118816 ISSN: 0569-8197**
- **OHCHI Y ET AL: "The characterization of lubricant on ME tape by TOF-SIMS analysis" JOURNAL OF MAGNETISM AND MAGNETIC MATERIALS, ELSEVIER SCIENCE PUBLISHERS, AMSTERDAM, NL, vol. 235, no. 1-3, October 2001 (2001-10), pages 183-186, XP004308561 ISSN: 0304-8853**
- **ZEMAN, A. ET AL: "Mass spectrometric fragmentation of neopentylpolyol esters, part III: Fatty acid diesters of neopentylglycol" FETTE, SEIFEN, ANSTRICHM. (1978), 80(10), 388-92 , XP009001823**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **PATENT ABSTRACTS OF JAPAN vol. 2000, no. 03, 30 March 2000 (2000-03-30) & JP 11 344027 A (MATSUSHITA ELECTRIC IND CO LTD), 14 December 1999 (1999-12-14)**
- **PATENT ABSTRACTS OF JAPAN vol. 2000, no. 15, 6 April 2001 (2001-04-06) & JP 2000 336383 A (SEIKO INSTRUMENTS INC), 5 December 2000 (2000-12-05)**

## Description

FIELD OF THE INVENTION

The present invention primarily relates to a lubricant composition used for lubricating parts that slide in relation to each other in a mechanical system, in particular, to a lubricant composition (hereinafter referred to as simply lubricant, if necessary) suitable for a dynamic pressure hydraulic bearing such as in a spindle motor.

BACKGROUND OF THE INVENTION

At present, a spindle motor used in an information recording and reproduction device, such as a hard disk device, has a rotational speed of approximately 10,000 rpm and is targeted to have a rotational speed of 15, 000 rpm at the next stage. Under the conditions for such a high-speed rotational operation, the motor is expected to produce quite a large amount of heat. When an simulation is actually carried out, the result is gained that the temperature of the motor exceeds 100°C. Then, a dynamic pressure hydraulic bearing tends to be used.' The characteristics of the lubricant have a great influence on the bearing performance of a dynamic pressure hydraulic bearing. It is required to restrict metal contact between the rotational axis part and the bearing part immediately after the startup or immediately before the stoppage of the motor. In addition, at the time of continuous operation, stable hydraulic lubricant characteristics having little deterioration, such as oxidation, decomposition and vaporization of the lubricant, are' required in spite of the great generation of heat due to rotation. Furthermore, a lubricant of a low viscosity, of which the friction coefficient is low, is required in order to reduce the driving power. The lowering of the torque can be implemented by lowering the viscosity of the lubricant while the problem wherein the lowering of the viscosity allows the deterioration of the heat resistance and of the vaporization characteristics must be solved.

The lubricants that have conventionally been used with dynamic pressure hydraulic bearings include di-esters represented by DOS (sebacic acid di-2-ethylhexyl) and triesters of trimethylolpropane and monovalent fatty acids. In addition, there are lubricants of which the base oil is a mixed ester of a capryl acid of neopentylglycol and capric acid.

When the viscosity of a di-ester is lowered, the heat resistance and vaporization characteristics deteriorate and hydrolysis more easily occurs. A mixed ester has a property wherein its characteristics may vary after utilization for a long period of time under high temperature and high-speed conditions and wherein viscosity becomes higher through polymerization. As the rotational speed of a spindle motor becomes higher, the amount of heat generation increases and, in some cases, the temperature of the bearing part exceeds 100°C. When a lubricant including a di-ester or a tri-ester is used under such temperature conditions, hydrolysis or polymerization of the lubricant is promoted and, therefore, a dynamic pressure hydraulic bearing cannot be stably utilized for a long period of time under high-speed rotational conditions as described above.

On the other hand, as for a lubricant wherein a mixed ester of capryl acid of neopentylglycol and a capric acid is used as a base oil, overall deterioration of the lubricant advances due to the decomposition of the low viscosity ester component so that change in viscosity occurs. As a result an abnormal rotation of the motor or an increase in torque occurs and, therefore, not only is reliability lowered but, also, a reduction in power consumption cannot be implemented.

Bearing loss increases at low temperatures wherein the viscosity increases and the lubricant film breaks down at high temperatures wherein viscosity decreases so that seizure, or the like, easily occurs due to metal contact caused by breakdown of the lubricant film. Under conditions wherein temperature variation is great, such as utilization outdoors during the cold season and in after being left in a car during the hot season, the attainment of a stable rotational performance is required.

EP 0 913 457 A2 relates to a liquid refrigeration composition comprising at least one fluorine-containing hydrocarbon, at least one ester of a carboxylic acid and polyhydroxy compound and a performance additive.

The publication LUBRICATION ENGINEERING, volume 13. August 1957; XP-001098167 relates to a general article of lubrication engineering describing in detail synthetic ester lubricants.

FR 2 774 286 relates to cosmetic compositions produced by forming a lipophilic phase and a process for its preparation.

Zeman. A. et al: Fette. Seifen. Anstrichm. (1978); XP-009001823 is a general article of fragmentation of homologeous diesters of fatty acids of neopentyl glycols (C2 to C18) in the MS-identification of specific fatty acid esters.

Matsuoka Kaoru et al: IEEE Trans Magn, 2001; XP 002223134 relates to a hard spindle motor using a fluid dynamic bearing (FDB) for being used in hard disk drives (HDD).

Campana J E et al: Asle Transactions; XP001118816 is a general article of Advanced Mass Spectrometry of Lubricants.

US 5,973,878 relates to recording disk apparatus and rotational supporting structure thereof, having a magnetic lubricant seal which is inclined.

WO 97/16827 relates to a disk drive spindle motor having hydro bearing with lubricant optimized with disc drive

compatible additives.

JP 11344027 relates to a fluid bearing free of a rub or seizure by increasing the accuracy and surface hardness of a sleeve having small wall thickness, and the improvement of the sliding with respect to a shaft.

JP 2000336383 relates to a hydrodynamic bearing having long durability and a reduced potential different between a shaft and a bearing by using a neopentyl glycol mixed ester of caprylic acid and capric acid as the base oil of a lubricationg oil for a hydrodynamic bearing.

SUMMARY OF THE INVENTION

Accordingly, a major object of the present invention is to provide a lubricant having a low viscosity even at low temperatures and having a small variation in viscosity and a small vaporization loss wherein oxidation and decomposition are restricted even at high temperatures and of which the characteristics are stable.

Other objects, characteristics and benefits of the present invention will become clear from the descriptions below.

In order to achieve the above described object, a lubricant composition according to claim 1 is provided.

A base oil having the structure of (chemical formula) is a single ester made of a polyol and a fatty acid. "tert" indicates the third class.

A hindered phenol-based antioxidant has the function of a radical scavenger and has the nature of prevention of oxidation of the base oil through self-oxidation when heated. Accordingly, even in the case that the lubricant becomes of a high temperature due to the heating of the dynamic pressure hydraulic bearing at the time of high-speed rotation, oxidation is not allowed to occur so that deterioration of the characteristics of the lubricant can be prevented.

According to the above description the viscosity characteristics required in the case wherein the lubricant is used with a dynamic pressure hydraulic bearing, that is to say, a viscosity of 48 mPa · s' or less at 0°C and of 12 mPa · s or less at 40°C can be secured. In particular, the lubricant is suitable for a dynamic pressure hydraulic bearing in a spindle motor for rotationally driving, at a high-speed, an information recording medium that is mounted to an information recording and reproduction device, such as a magnetic disk device, an optical disk device and an magneto-optical disk device. In addition, the lubricant is' suitable for a dynamic pressure hydraulic bearing in a motor that rotationally drives, at a high speed, a polygonal mirror for a scanner of an LBP (laser beam printer).

According to the experiments of the present inventors, 46.7 mPa • s was gained (in the case wherein R = $C_9H_{19}$) as a high value of viscosity at 0°C, which makes possible the implementation of 48 mPa·s or less including a margin in accordance with empirical observation. In addition, 9.50 mPa·s was gained (in the case wherein R = $C_9H_{19}$) as a high value of viscosity at 40°C, which makes possible the implementation of 12 mPa • s or less, and, more preferably, 10 mPa·s or less, including a margin in accordance with empirical observation.

In addition, according to the experiments of the present inventors, 36.1 mPa·s was gained (in the case wherein R = $C_8H_{17}$) as a viscosity at 0°C, which makes possible the implementation of 38 mPa•s or less including a margin in accordance with empirical observation. In addition, 7.7 mPa • s was gained (in the case wherein R = $C_8H_{17}$) as a viscosity at 40°C, which makes possible the implementation of 10 mPa • s, and more preferably 8 to 9 mPa·s or less, including a margin in accordance with empirical observation.

In regard to the above description, it is preferable for the antioxidant content to be 0.1 wt.% or more. In the case that the content is lower than this, the antioxidant effect cannot be expected to a great degree. The optimal amount to be added differs according to the purpose of utilization and when, at least, 0.1 wt.% or more is added, it produces the effect of antioxidant prevention. In addition, an excessive content allows the performance of the base oil to deteriorate and, therefore, it is desirable for the upper limit value of the content to be set at 10 wt.% and, moreover, it is more preferable for the upper limit value to be set at 8 wt.% or less, because the occurrence of deterioration of the performance of the base oil is minimal.

By adding a triglyceride, slidability is increased under the metal contact condition that occurs when the dynamic pressure becomes low immediately after start-up or immediately before stoppage. Accordingly, in the.case that a spindle motor is frequently started up or stopped, such as in a hard disk device, friction and wear of the rotational axis part and of the bearing part can be reduced so that a dynamic pressure hydraulic bearing of a high reliability can be implemented.

In addition, low viscosity can be maintained even in low temperature conditions and a lubricant wherein decomposition or oxidation does not occur, even at high temperatures, can be gained. Bearing loss is small even at the time of utilization at low temperatures and the bearing can be stably utilized even at the time of high-speed rotation wherein the temperature of the lubricant becomes high. Accordingly, a spindle motor wherein a dynamic pressure hydraulic bearing is mounted exercises a stable rotational performance of a high reliability even under conditions wherein change in temperature is great, such as utilization outdoors during the winter or utilization after being left in a car in summer. In addition, the change in quality in the lubricant does not easily occur in a dynamic pressure hydraulic bearing of a copper alloy material wherein vaporization loss is small. In particular, change in quality does not occur in a dynamic pressure hydraulic bearing formed of a copper alloy material on which nickel phosphorus plating has been carried out.

In the above description, it is according to the invention for CxHyOz, which are R1, R2 and R3 of the triglyceride,

to have an integer value of x in the range of from 15 to 21, an integer value of y in the range of from 29 to 43 and an integer value of z in the range of from 0 to 1, respectively.

In the case that the value of z is 0, a structure of an unsaturated or a saturated straight chain alkyl base or a branched alkyl base is provided. In the case that the value of z is 1, an unsaturated or a saturated straight chain structure or a branched structure having an OH base in the structure is provided. In the case that the structure has an OH base, wettability with metal forming the dynamic pressure hydraulic bearing is improved so that the lubricant characteristics can be improved. In addition, in the case that the values of x and y are made greater than the.upper limit of the above described ranges, the triglyceride becomes a solid having a poor compatibility with the base oil. Furthermore, the viscosity also becomes great. In addition, in the case that the values of x and y are smaller than the lower limit of the above described ranges, the lubricant characteristics at the time of start-up are lowered. As for conditions that can obviate the above problems, the above described values of x and y are in the desired ranges.

In addition, in the above description, in the case that the triglyceride content exceeds 5 wt.%, the performance of the base oil is lowered. The allowance of the addition of triglyceride for maintaining performance is 5 wt.% while the addition of triglyceride can increase the slidability in the metal contact condition that occurs when the dynamic pressure is low immediately after start-up or immediately before stoppage. In the case that the triglyceride content is 3 wt.% or less, the lifetime of the lubricant can be prevented from being lowered and this is more desirable.

Another aspect of the above described lubricant composition according to the present invention can be described as follows.

This is a lubricant composition according to claim 1 wherein peaks of the secondary ion intensities exist at "127," "213" and "357" of the mass numbers of positive secondary ions per unit charge when a base oil included in the lubricant is analyzed by means of secondary ion mass spectrometry in a manner corresponding to the case of the lubricant including a base oil that is a single ester having the structure of (chemical formula) when alkyl base R = $C_7H_{15}$.

This is a single ester having the structure of (chemical formula) wherein the mass numbers for specifying the base oil in the case wherein alkyl base R = $C_7H_{15}$ are a combination of "127," "213" and "357."

In addition, this is a lubricant composition wherein the peaks of secondary ion intensities exist at "141," "227" and "385" of the mass numbers of positive secondary ions per unit charge when the base oil included in the lubricant is analyzed by means of secondary ion mass spectrometry so as to correspond to the case of a lubricant that includes a base oil of a single ester having the structure of (chemical formula) in the case wherein the alkyl base R = $C_8H_{17}$.

This is a single ester having the structure of (chemical formula) wherein the mass numbers for identifying the base oil in the case wherein the alkyl base R = $C_8H_{17}$ are a combination of "141," "227" and "385."

Furthermore, this is a lubricant composition wherein the peaks of secondary ion intensities exist at "155," "241" and "413" of the mass numbers of positive secondary ions per unit charge when the base oil included in the lubricant is analyzed by means of secondary ion mass spectrometry so as to correspond to the case of a lubricant that includes a base oil of a single ester having the structure of (chemical formula) in the case wherein the alkyl base R = $C_9H_{19}$.

This is a single ester having the structure of (chemical formula) wherein the mass numbers for identifying the base oil in the case wherein the alkyl base R = $C_9H_{19}$ are a combination of "155," "241" and "413."

All of these describe one means in the case that a lubricant composition according to the present invention is practically specified. The grounds for the above described mass numbers will become clear from the descriptions in from paragraph (Section 3) to paragraph (Section 4) in the explanation in the below described third embodiment.

It is possible to understand the present invention according to the following expanded considerations. That is, as a reference aspect concerning an analysis method of a lubricant.

That is to say, in an analysis method of a lubricant according to the reference aspect, a base oil included in the lubricant is analyzed by means of secondary ion mass spectrometry and whether or not the peaks of the secondary ion intensities exist in the mass spectrum at "127," "213" and "357" of the mass numbers of positive secondary ions per unit charge is determined so that a lubricant having a base oil of a single ester of (chemical formula 3) is identified in the case where the peaks exist.

[chemical formula 3]

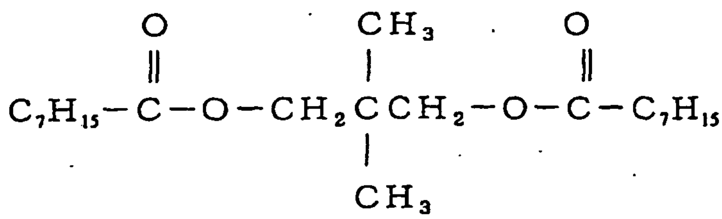

In addition, in another analysis method of a lubricant according to the reference aspect, a base oil included in

the lubricant is analyzed by means of secondary ion mass spectrometry and whether or not the peaks of the secondary ion intensities exist in the mass spectrum at "141," "227" and "385" of the mass numbers of positive secondary ions per unit charge is determined so that a lubricant having a base oil of a single ester of (chemical formula 4) is identified in the case that the peaks exist.

[chemical formula 4]

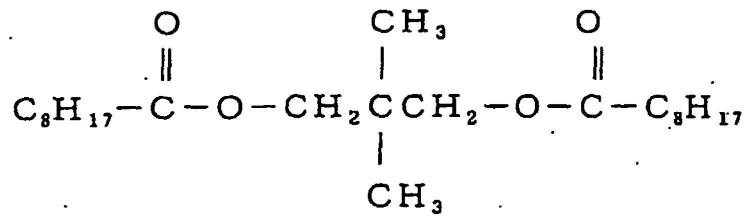

Furthermore, in another analysis method of a lubricant according to the reference aspect, a base oil included in the lubricant is analyzed by means of secondary ion mass spectrometry and whether or not the peaks of the secondary ion intensities exist in the mass spectrum at "155, " "241" and "413" of the mass numbers of positive secondary ions per unit charge is determined so that a lubricant having a base oil of single ester of (chemical formula 5) is identified in the case that the peaks exist.

[chemical formula 5]

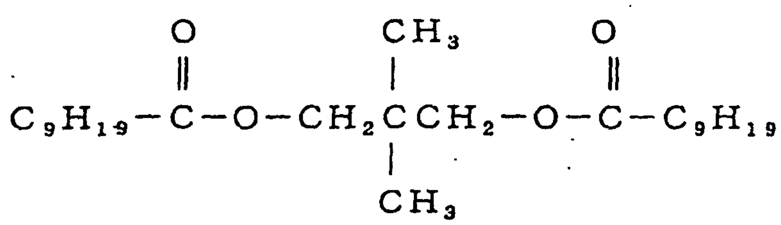

It is possible to understand the present invention according to the following expanded considerations. That is, as a reference aspect concerning a deterioration analysis method of a lubricant composition.

That is to say, in a deterioration analysis method of a lubricant composition according to the reference aspect, after a lubricant composition in an initial condition is analyzed by means of secondary ion mass spectrometry so as to find secondary ion intensities at "127," "213" and "357" of the mass numbers of positive secondary ions per unit charge in the mass spectrum, the lubricant composition after utilization for a predetermined period of time is analyzed in the same manner so as to find secondary ion intensities at the same mass numbers and wherein a ratio of a secondary ion intensity of the initial condition to a secondary ion intensity after the utilization for the predetermined period is found so as to evaluate the deterioration condition of the lubricant composition.

This is effective in the deterioration analysis of a lubricant having a base oil of a single ester of (chemical formula 3) in the case of the alkyl group R = $C_7H_{15}$.

In addition, in another deterioration analysis method of a lubricant composition according to the reference aspect, after a lubricant composition in an initial condition is analyzed by means' of secondary ion mass spectrometry so as to find the secondary ion intensities at "141, " "227" and "385" of the mass numbers of positive secondary ions per unit charge in a mass spectrum, the lubricant composition after utilization for a predetermined period of time is analyzed in the same manner so as to find secondary ion intensities at the same mass numbers and wherein a ratio of a secondary ion intensity in the initial condition to a secondary ion intensity after the utilization for the predetermined period is found so as to evaluate a deterioration condition of the lubricant composition.

This is effective in the deterioration analysis of a lubricant having a base oil of a single ester of (chemical formula 4) in the case of the alkyl group R = $C_8R_{17}$.

Further, in still another deterioration analysis method of a lubricant composition according to the reference aspect, after a lubricant composition in an initial condition is analyzed by means of secondary ion mass spectrometry so as to find secondary ion intensities at "155", "241," and "413" of the mass numbers of positive secondary ions per unit charge in a mass spectrum, the lubricant composition after utilization for a predetermined period of time is analyzed in the same manner so as to find secondary ion intensities at the same mass numbers and wherein a ratio of a secondary ion intensity in the initial condition to a secondary ion intensity after the utilization for the predetermined period is found

so as to evaluate the deterioration condition of the lubricant composition.

This is effective in the deterioration analysis of a lubricant having a base oil of a single ester of (chemical formula 5) in the case wherein the alkyl base R = $C_9H_{19}$.

In all of the above described deterioration analysis methods of a lubricant composition, a ratio of the mass spectrum of the lubricant before utilization to the mass spectrum of the lubricant after utilization is gained by means of secondary ion mass spectrometry and, thereby, the deterioration of the lubricant, even of a sample of an extremely small amount, can be analyzed with a high precision.

A lubricant composition gained in the above described manner is favorably applied with the following dynamic pressure hydraulic bearing or in the following motor.

That is to say, a dynamic pressure hydraulic bearing according to the present invention is provided with a bearing part and a rotational axis part engaged with each other so as to be freely rotatable; a dynamic pressure generation trench created in, at least, one of the two surfaces facing each other that form a gap between the above described bearing part and the above described rotational axis part; and a lubricant composition filled in the gap between the surfaces facing each other, wherein any of the above described lubricant compositions is used as the above described lubricant composition.

In addition, a motor according to the present invention is provided with a base part; a stator for generating a magnetic field secured to the above described base part; a rotor having a rotational magnet opposed to the above described stator; a rotational axis part provide in the above described rotor; a bearing part provided in the above described base part and with which the above described rotational axis part engaged so as to be freely rotatable; a dynamic pressure generation trench created in at least, one of the two surfaces facing each other that form a gap between the above described bearing part and the above described rotational axis part; and a lubricant composition filled in the gap between the above described surfaces facing each other, wherein any of the above described lubricant compositions is used as the above described lubricant composition.

There are a variety of modes, such as the following, of the configurations of the above described motor.

A motor is provided wherein the above described bearing part on the base part side is made in a cylindrical form and the above described rotational axis part on the rotor side is engaged with the inside of the above described bearing part.

In addition, a motor is provided wherein the above described rotational axis part on the rotor side is made in a cylindrical form and wherein the above described rotational axis part is engaged with the outside of the above described bearing part on the base part side.

In addition, a motor is provided so as to have a radial bearing part wherein the above described bearing part and the above described rotational axis part face each other in the radial direction and the above described lubricant composition is filled in the gap between the above described bearing part and the above described rotational axis part and wherein a dynamic pressure generation trench is created in, at least, one of the two surfaces facing each other that form the gap in the above described radial bearing part.

In addition, a motor is provided so as to have a thrust bearing part wherein the above described bearing part and the above described rotational axis part face each other in the axis direction and the above described lubricant composition is filled in the gap between the above described bearing part and the above described rotational axis part and wherein a dynamic pressure generation trench is created in, at least, one of the two surfaces facing each other that form the gap in the above described thrust bearing part.

In addition, a motor is provided wherein a thrust bearing part is formed of an end surface on the side having an opening of the above described cylindrical bearing part and of an annular region in the above described rotor facing the above described end surface on the side having the opening of the above described bearing part and wherein a dynamic pressure generation trench is formed in, at least, one of the above described end surface on the side having the opening and the above described annular region that faces the end surface in the above described thrust bearing part.

In addition, a motor is provided wherein a thrust bearing part is formed of an end surface on the side having an opening of the above described cylindrical rotational axis part and of an annular region in the above described base part facing the above described end surface on the side having the opening of the above described rotational axis part and wherein a dynamic pressure generation trench is formed in, at least, one of the above described end surface on the side having the opening and the above described annular region that faces the end surface in the above described thrust bearing part.

In addition, a motor is provided wherein nickel phosphorus plating film is applied to the two surfaces facing each other that form the gap between the above described bearing part and the above described rotational axis part. It is preferable for the above described nickel phosphorus plating film to be an electroless plated film of which the phosphorus concentration is 15 wt.% or less.

Thus, a motor integrated device to which any of the above described motors is mounted is also an effective invention.

BRIEF DESCRIPTION OF THE DRAWINGS

These and other objects as well as advantages of the invention will become clear by the following description of preferred embodiments of the invention with reference to the accompanying drawings, wherein:

Fig. 1 is a graph showing the result of an accelerated high temperature test carried out on the lubricants of the examples reference examples and of the comparative examples used with a nickel phosphorous plated copper alloy with respect to the lubricant composition according to the first embodiment of the present invention;
Fig. 2 is a graph showing the result of an accelerated high temperature test carried out on the lubricants of the examples and reference examples used with a copper alloy with respect to the lubricant component according to the first embodiment of the present invention;
Fig. 3 is a mass spectrum graph as -a result of secondary ion mass spectrometry of the lubricant of the first example with respect to the lubricant composition according to the second embodiment of the present invention;
Fig. 4 is a mass spectrum graph as a result of secondary ion mass spectrometry of the lubricant of the second example with respect to the lubricant composition according to the second embodiment of the present invention;
Fig. 5 is a mass spectrum graph as a result of secondary ion mass spectrometry of the lubricant of the third example with respect to the lubricant composition according to the second embodiment of the present invention;
Fig. 6 is a mass spectrum graph as a result of secondary ion mass spectrometry of the lubricant of the fourth example with respect to the lubricant composition according to the second embodiment of the present invention;
Fig. 7 is a mass spectrum graph as a result of secondary ion mass spectrometry of the lubricant of a comparative example with respect to the lubricant composition according to the second embodiment of the present invention;
Fig. 8 is a cross sectional view showing an example wherein a motor to which a dynamic pressure hydraulic bearing according to the fourth embodiment of the present invention is applied in a hard disk device;
Fig. 9 is a cross sectional view of an enlarged portion in Fig. 9;
Fig. 10 is a view showing a pattern of dynamic pressure trench in the thrust surface of the dynamic pressure hydraulic bearing in Fig. 9;
Fig. 11 is a graph showing the result of an accelerate high temperature test carried out on the lubricant of a reference example and the lubricants of comparative examples with respect to the lubricant composition according to the fourth embodiment of the present invention;
Fig. 12A is a plan view showing the configuration of the main parts of a device in which a motor is integrated according to the fifth embodiment of the present invention;
Fig. 12B is a cross sectional view corresponding to Fig. 12A;
Fig. 13 is a cross sectional view of the main parts of the motor according to the fifth embodiment of the present invention;
Fig. 14A is a plan view of a rotor of the motor according to the fifth embodiment of the present invention;
Fig. 14B is a plan view of a rotor of another motor according to the fifth embodiment of the present invention;
Fig. 15 is a cross sectional view showing the structure of an outer rotor-type motor according to the sixth embodiment of the present invention;
Fig. 16 is a cross sectional view showing the structure of an axial gap-type motor according to the seventh embodiment of the present invention;
Fig. 17 is a cross sectional view of main parts of a motor according to the eighth embodiment of the present invention;
Fig. 18A is a plan view of a rotor of a motor according to the eighth embodiment of the present invention;
Fig. 18B is a plan view of a base part of a motor according to the eighth embodiment of the present invention;
Fig. 19 is a graph showing variations over time in viscosity of lubricants used in a motor according to the fifth to eighth embodiments of the present invention;
Fig. 20 is another graph showing variations over time in viscosity of lubricants used in a motor according to the fifth to eighth embodiments of the present invention;
Fig. 21 is a graph showing the effects of oil agents added to a lubricant used in a motor according to the fifth to eighth embodiments of the present invention; and
Fig. 22 is a graph showing the effects of nickel phosphorous plating and of lubricants used with a dynamic pressure hydraulic bearing part provided in a motor according to the fifth to eighth embodiments of the present invention.

In all these figures, like components are indicated by the same numerals.

DETAILED DESCRIPTION OF THE PREFERRED EMBODIMENTS

In the following, the present embodiments of the present invention are described in reference to the drawings.

(First Embodiment)

Lubricant compositions (lubricant) according to the first embodiment of the present invention include at least a base oil, an antioxidant and an additive and is filled into the gap between two opposing faces of two members that shift relative to each other wherein the lubricant is composed as follows.

First, a substance having the structure of (chemical formula 6) is adopted as the base oil.

[chemical formula 6]

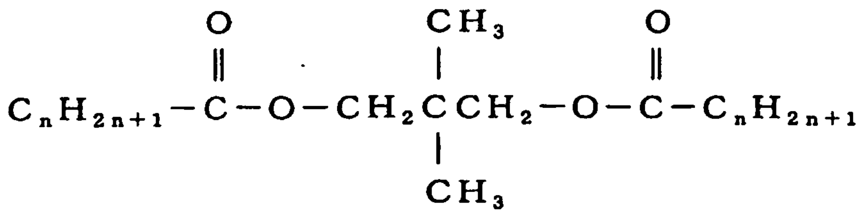

Here, n = 7, n = 8 or n = 9 in (chemical formula 6). Concretely, they are base oil (A) having the structure of (chemical formula 7), base oil (B) having the structure of (chemical formula 8) or base oil (C) having the structure of (chemical formula 9), all of which are single esters.

[chemical formula 7]

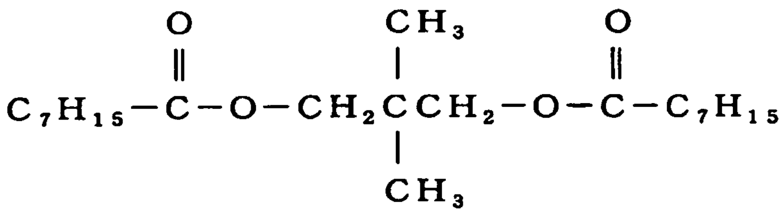

[chemical formula 8]

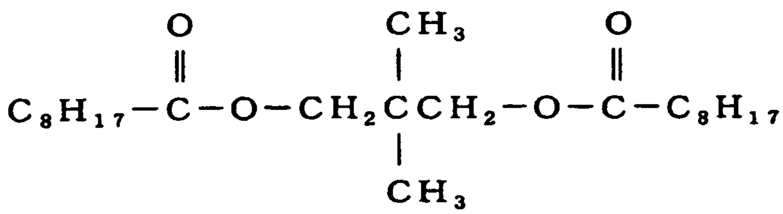

[chemical formula 9]

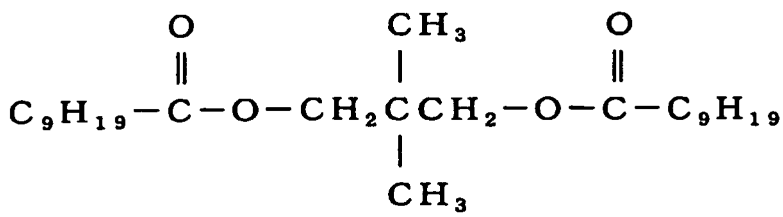

Any one type from among the above three types is used as the base oil of the lubricant. Here, all of base oils (A) to (C) are esters made up of a polyol and a fatty acid.

In one lubricant the alkyl bases $R=C_nH_{2n+1}$ on both ends of the base oil are the same. In addition, in one lubricant the above described three types of base oils are not mixed but, rather, the base oil is limited to any one type. In this sense, the base oil in a lubricant of the present invention is a single ester. Base oil (A), base oil (B) and base oil

(C) have alkyl bases R on each end that differ from each other and, due to this, these three types of base oils have viscosities differing from each other. Unlike the mixed ester in the case of the prior art, a plural number of types of base oils having differing viscosities are not mixed in the present invention. That is to say, the base oil in a lubricant of the present invention is a single ester. Here, a lubricant wherein the above described base oil is used, according to the present invention, has the appropriate application object of a dynamic pressure hydraulic bearing.

In the case that a mixed ester is used, the low viscosity component in the mixed component decomposes earlier than the other components when utilized for a long period of time and, due to this, a phenomenon occurs wherein the deterioration of the lubricant is accelerated. In contrast to this, exactly the same alkyl base is used in the structure and, in addition, a base oil consisting of only one type is used according to the present invention and, thereby, the viscosity characteristics can be adjusted to have a predetermined value, that is to say, 48 mPa·s or less at 0°C and 12 mPa·s or less at 40°C.

A lubricant having a low viscosity, even in low temperature conditions, and of which the variation in viscosity is small in response to change in temperature from low to high can be realized by using a base oil having the structure represented by (chemical formula 6) and having the above described viscosity characteristics. In the case that this lubricant is utilized for a dynamic pressure hydraulic bearing, bearing loss is small when utilized at a low temperature and the bearing can be utilized stably at a high rotation speed wherein the temperature of the lubricant becomes high.

Here, a viscosity of 48 mPa·s or less at 0°C is a value wherein the.bearing loss is allowable and the bearing loss can be further reduced in the case that the viscosity is 40 mPa·s or less at 0°C. In addition, the viscosity at a temperature of 40°C is 12 mPa·s or less and, in the case that the viscosity is 48 mPa·s at 0°C and 12 mPa·s at 40°C, variation in viscosity due to temperature fluctuation can be restricted to the range of allowable values and, therefore, the bearing loss can be reduced over a broad temperature range. The above has been discovered by the present inventors.

Next, the antioxidant is described and a lubricant of the present invention has a main body of the above described base oil and, in addition, includes an additive of hindered phenol-based antioxidants. The total amount of this additive is made to be, at least, 0.1 wt.% or more. At least one (3, 5-di-tert-butyl-4-hydroxyphenyl) is included in the structure thereof. Here, at least one (3, 5-di-tert-butyl-4-hydroxyphenyl) may be included and two, three or four thereof may be included.

In summary, a hindered phenol-based antioxidant including (3, 5-di-tert-butyl-4-hydroxyphenyl) is added or both of these antioxidants are added.

Oxidation can be prevented from occurring even when the lubricant becomes of a high temperature so that deterioration of the characteristics of the lubricant can be prevented by adding 0.1 wt.% or more, of a hindered phenol-based antioxidant to the above described based oil. This hindered phenol-based antioxidant antioxidant has the function of radical scavenger and is of a nature that prevents oxidation of the base oil through self-oxidation when heated. That is to say, in the case that a lubricant is exposed to high temperature, unstable peroxide is generated as an oxidization product generated at the initial stage. This peroxide decomposes so as to generate new free radicals and, thereby, the oxidation progresses in an accelerated manner. However, a hindered phenol-based antioxidant has the effect of reacting with and deactivating these free radicals. Accordingly, oxidation can be prevented. Though the optimal amount of these antioxidants to be added differs depending on the purpose of utilization, the addition of, at least, 0.1 wt.% or more, gains an anti-oxidation effect.

Here, if the added amount is excessive, the performance of the base oil deteriorates and, therefore, it is desirable for the upper limit value of the added amount to be set at 10 wt.% and, furthermore, 8 wt.% or less, is more desirable because it is in the range wherein performance deterioration of the base oil hardly occurs.

In addition, an additive for reduction of the friction coefficient is required for the lubricant and a lubricant of the present invention includes a triglyceride shown in the structure of (chemical formula 10).

[chemical formula 10]

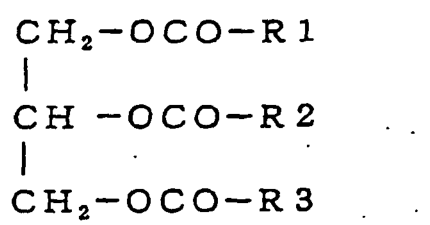

Here, R1, R2 and R3 are, respectively, an unsaturated or saturated straight chain structure or branched structure consisting of CxHyOz and, furthermore, R1, R2 and R3 preferably have the same structure or at least one of them has a differing structure. Moreover, it is according to the invention for the value of x to be an integer value in the range of

from 15 to 21, for the value of y to be an integer value in the range of from 29 to 43 and for the value of z to be an integer value in the range of from 0 to 1.

More concretely, when the value of z is 0, a structure of an unsaturated or saturated straight chain alkyl base or branched alkyl base is gained while when the value of z is 1 an unsaturated or saturated straight chain structure or branched structure having an OH base in the structure is gained. In the case that an OH base is in the structure, the wettability of the bearing member vis-à-vis a metal is improved so that the friction coefficient is reduced and, therefore, the characteristics of the lubricant can be improved.

Here, in the case that the values of x and y are greater than the upper limit of the above described range, the triglyceride becomes a solid and not only the miscibility with the base oil becomes worse but, also, the viscosity becomes greater. In addition, in the case that the values are made smaller than the lower limit of the above described range, the characteristics of the lubricant at the time of start up are lowered. Accordingly, the above described values indicate desirable ranges as the conditions for overcoming both of these limitations.

Then, the total amount of the additive, which includes, at least, triglyceride, is made to be 5 wt.%, or less. In the case that the amount of the triglyceride to be added is excessive, the performance or the base oil is lowered and a limit Of 5 wt.% of additive is within the performance allowance while in the case of 3 wt.% or less, the lifetime of the lubricant can be prevented from being lowered, which is more desirable.

A metal contact condition occurs in the dynamic pressure hydraulic bearing when the dynamic pressure becomes small immediately after start up or immediately before stoppage and in the case that a lubricant, to which 5 wt.% or less, of triglyceride having the above described structure is added, is used, the slidability at that time can be increased. Accordingly, in the case that a spindle motor is frequently started up or stopped such as in a hard disk device, the friction and wear of the rotation axis and the bearing part can be reduced so that a dynamic pressure hydraulic bearing of a high reliability can be implemented.

As described above, a lubricant (lubricant compositions) of the present embodiment is characterized in that the viscosity characteristics are adjusted so that the viscosity at 0°C is 48 mPa·s or less and the viscosity at 40°C is 12 mPa s or less by adding a hindered phenol-based antioxidant, or a hindered phenol-based antioxidant and a hindered amine-based antioxidant and a triglyceride to a base oil of a single ester having the above described structure of (chemical formula 7), (chemical formula 8) or (chemical formula 9) so as to gain a substitute for DOS (sebacic acid di-2-ethylhexyl) that is a representative example of conventional diesters, (trimethylolpropane + monovalent fatty acid) that is a representative example of triesters or a mixed ester of a capryl acid of neopentylglycol and capric acid and, thereby, a lubricant is provided wherein the low viscosity can be maintained even in low temperature conditions and decomposition or oxidation does not occur at a high temperature and, therefore, the friction coefficient is low. Accordingly, in the case that the lubricant is utilized for a dynamic pressure hydraulic bearing, bearing loss is small at the time of low temperature utilization and, when the temperature of the lubricant becomes high, the bearing can be utilized stably even at high rotation speed. Furthermore, a lubricant for a dynamic pressure hydraulic bearing can be provided, of which the characteristics are very stable, having a small variation in viscosity and a small vaporization loss and having good heat resistance so that oxidation or decomposition hardly occurs.

[Examples of First Embodiment]

In the following, the present invention is described in detail by using examples of the first embodiment.

(First Reference Example)

A lubricant of the first reference example was prepared as follows. This is a lubricant that includes a single ester base oil having the structure shown in (chemical formula 6) wherein the alkyl base $R=C_nH_{2n+1}$ is a straight chain saturated alkyl base of $C_7H_{16}$ in the case of n = 7. 1 wt.% of hindered phenol-based antioxidant having four (3, 5-di-tert-butyl-4-hydroxyphenyl) was added to the above base oil as an antioxidant..

(Second Reference Example)

A lubricant of the second reference example was prepared as follows. This is a lubricant that includes a single ester bas.e oil having the structure shown in (chemical formula 6) wherein the alkyl base $R=C_nH_{2n+1}$ is a straight chain saturated alkyl base of $C_8H_{17}$ in the case of n = 8. 1 wt.% of hindered phenol-based antioxidant having four (3, 5-di-tert-butyl-4-hydroxyphenyl) was added to the above base oil as an antioxidant.

(Third Reference Example)

A lubricant of the third reference example was prepared as follows. This is a lubricant that includes a single

ester base oil having the structure shown in (chemical formula 6) wherein the alkyl base $R=C_nH_{2n+1}$ is a straight chain saturated alkyl base of $C_9H_{19}$ in the case of n = 9. 1 wt.% of hindered phenol-based antioxidant having four (3, 5-di-tert-butyl-4-hydroxyphenyl) was added to the above base oil as an antioxidant.

(Fourth Example)

A lubricant of the fourth example was prepared as follows. This is a lubricant that includes a single ester base oil having the structure shown in (chemical formula 6) wherein the alkyl base $R=C_nH_{2n+1}$ is a straight chain saturated alkyl base of $C_8H_{17}$ in the case of n = 8. 1 wt. % of hindered phenol-based antioxidant having four (3, 5-di-tert-butyl-4-hydroxyphenyl) was added to the above base oil as an antioxidant. In addition, 1 wt.% of a triglyceride shown in (chemical formula 10) was added. Here, R1, R2 and R3 have the same structure and have, respectively, the structure of CxHyOz wherein the value of x is 17, the value of y is 33 and the value of z is 1.

The first reference example, the second reference example and the third reference example are the same except that the structure of the alkyl base $R=C_nH_{2n+1}$ in the base oil differs. The fourth example corresponds to the case wherein a triglyceride is added in the second reference example.

(First Comparative Example)

DOS (sebacic acid di-2-ethylhexyl), which is a lubricant that has been used with dynamic pressure hydraulic bearings, was used as the first comparative example.

(Second Comparative Example)

A lubricant having a mixed ester as the base oil wherein two types of esters, an ester of neopentylglycol and capryl acid and an ester of neopentylglycol and a capric acid, are mixed was used as the second comparative example-.

Here, the amounts of main components of the base oils in the lubricants of from first to fourth reference examples and examples are shown in Table 1.

[Table 1]

| lubricant component | First Reference Example | Second Reference Example | Third Reference Example | Fourth Example |
|---|---|---|---|---|
| amount of base oil as main component (wt. %) | 98.5 | 85.3 | 97.7 | 96.7 |

As can be seen from (Table 1), the amounts of the main components of the base oil of the lubricants of from first to fourth examples, respectively, are 85 wt.% or more.

The results of measuring the viscosity, the stability of the viscosity, the amount of vaporization, the antioxidation, the stability at high temperature and the friction coefficient with respect to the above described lubricants of the four examples and reference examples and the two comparative examples are described in the following.

Table 2 shows the viscosity at 0°C and at 40°C, the rates of change according to temperature (B value) and the amounts of vaporization.

[Table 2]

| | First Reference Example | Second Reference Example | Third Reference Example | Fourth Example | first Comparative Example | Second Comparative Example |
|---|---|---|---|---|---|---|
| Viscosity at 0°C (mPa · s) | 27.3 | 36.13 | 46.71 | 36.52 | 52.72 | 37.39 |
| Viscosity at 40°C (mPa· s) | 6.44 | 7.72 | 9.50 | 7.75 | 10.42 | 8.00 |

(continued)

| | First Reference Example | Second Reference Example | Third Reference Example | Fourth Example | first Comparative Example | Second Comparative Example |
|---|---|---|---|---|---|---|
| rate of change accordi ng to tempera ture: B value | 2925 | 2757 | 2960 | 2914 | 3256 | 2975 |
| amount of vaporiz ation (wt.%) | 0.58 | 0.29 | 0.09 | 0.25 | 0.61 | 0.50 |

As for the viscosity at 0°C and the viscosity at 40°C, all the lubricants of from first to fourth examples and reference examples have a viscosity lower than that of the lubricant (characterized by DOS) of the first comparative example. In addition, all the lubricants of the first, second and fourth examples and reference examples except for the lubricant (characterized by $C_9H_{19}$) of the third reference example, have a viscosity lower than that of the lubricant (characterized the mixed ester) of the second comparative example.

The lubricant (characterized by DOS) of the first comparative example has been conventionally utilized in spindle motors of hard disk devices. Each example differs greatly from the first comparative example and the following drop in viscosity was recognized. That is to say, the results of
48% drop at 0°C and 38% drop at 40°C in the lubricant (characterized by $C_7H_{15}$) of the first reference example,
31% drop at 0°C and 26% drop at 40°C in the lubricant (characterized by $C_8H_{17}$) of the second reference example,
11% drop at 0°C and 8% drop at 40°C in the lubricant (characterized by $C_9H_{19}$) of the third reference example, and
31% drop at 0°C and 25% drop at 40°C in the lubricant (characterized by $C_8H_{17}$ + triglyceride) of the fourth example, are gained.

In addition, in comparison with the lubricant (characterized by the mixed ester) of the second comparative example, the results of
26% drop at 0°C and 19% drop at 40°C in the first reference example,
3% drop at 0°C and 3% drop at 40°C in the second reference example, and
2% drop at 0°C and 3% drop at 40°C in the fourth example, are gained.

Accordingly, in comparison with the lubricant (characterized by DOS) of the first comparative example that has been conventionally utilized, each of the lubricants of the present examples has a lower viscosity, both under low temperature and under high temperature conditions so that bearing loss of the dynamic pressure hydraulic bearing can be reduced.

In addition, in comparison with the lubricant (characterized by the mixed ester) of the second comparative example that is ester-based in the same manner, the viscosity is lower in the first, second and fourth examples and reference examples so that the bearing loss can be reduced.

It is also important for the variation in viscosity of the lubricant to be small over a broad temperature range in order to maintain, in a highly precise manner, the rotational speed of a spindle motor and, therefore, this variation in viscosity is evaluated. As for the method of evaluation, data of the viscosity shown in Table 2 is used so as to approximate the relationship between the viscosity Y and the reciprocal of the absolute temperature T to an exponential function shown in (equation 1) and the variation in viscosity is compared by using the value of a constant B.

[equation 1] $$Y \doteq A \cdot e^{-B/T}$$

In (equation 1), A and B are constants. The constant B shows the slope of the curve which represents the variation ratio of the viscosity according to the temperature. That is to' say, the curve represents wherein the smaller the value of the constant B is, the smaller is the variation in viscosity of the lubricant according to temperature. This result is shown as B values in Table 2.

The B values of the lubricants of the first to fourth examples and reference examples were smaller than the respective B values of the first comparative example and the second comparative example so that the result was gained that fluctuation in viscosity according to change in temperature does not easily occur.

Large variation in viscosity means that bearing loss becomes large on the lower temperature side wherein the

viscosity becomes high and that seizure easily occurs due to contact with metal when the lubricant film is broken on the high temperature side wherein the viscosity becomes low. Each of the lubricants of the present examples has a smaller change in viscosity than that in the comparative examples even under the conditions wherein change in temperature is great such that an apparatus, to which a motor wherein such a lubricant is used is mounted, is utilized outdoors in winter or is utilized after being left in.a car in summer and, therefore, a dynamic pressure hydraulic bearing with which such a lubricant is used or a spindle motor wherein such a dynamic pressure hydraulic bearing is used can gain a stable rotational performance over a broad temperature range and in a high-speed rotation region.

Next, the results of measurements of vaporization loss are described. As for the vaporization loss, each of the lubricants of the first to fourth examples and reference examples as well as the first and second comparative examples is put in a glass beaker and the beaker is left in a thermostatic bath at 100°C for 120 hours and, after that, the amount of decrease in weight (wt.%) is found as vapor loss. This result is shown as the amount of vaporization in Table 2.

The results are gained that the vaporization losses of the second, third and fourth examples and reference examples are lower than the vaporization losses of the first and second comparative examples. In particular, the result of significantly low vaporization loss .is gained in the third reference example. That is to say, the lubricants of the second, third and fourth examples and reference examples have small vaporization losses so that lubricant does not run short after a long period of operation and the lifetime of a spindle motor can be increased. In particular, the lubricant of the third reference example has a remarkable stability.

Next, heat resistance stability of a lubricant when the lubricant contacts a rotating shaft and a metal that is a bearing member under a high temperature for a long period of time is evaluated -according to an accelerated high temperature test. The accelerated high temperature test is carried out according to the following procedure by taking into consideration the condition of the lubricant at the time of actual drive of a spindle motor and by using an oil heating bath with a shaker.

First, each of the lubricants of the first to fourth examples and reference examples as well as the first and second comparative examples is poured into a test tube made of stainless steel. The same metal material as that of the rotating shaft and the bearing member is soaked in the lubricant in each of the test tubes into which the lubricant has been poured. As for this metal material, a columnar rod made of a copper alloy is used that is frequently utilized as a bearing member, on the surface of which a coating film of nickel phosphor plating, which is generally used for the hardening of the surface, is formed. These test tubes are soaked in the oil heating bath with a shaker that has been heated to 150°C and are shaken in order to carry out the accelerated high temperature test. A test tube made of stainless steel and a copper alloy rod on which nickel phosphorus plating is carried out are in constant contact with each other due to oscillation by means of the shaker and, therefore, newly created surfaces are always formed on the surfaces of the respective metal so that the interface lubrication condition can be implemented.

As for the evaluation method, a constant amount of each of the lubricants is sampled and the viscosity is measured whenever a constant period of time elapses during the above described accelerated high temperature test and, thereby, the variation in viscosity is evaluated and the total oxidation number after 627 hours (26 days and 3 hours) has elapsed is measured for the evaluation. In the case of a lubricant of which the heat resistance is inferior or in the case of a lubricant that decomposes or deteriorates when metal works as a catalyst, the viscosity thereof changes under high temperature conditions or due to catalysis when a metal is added and, therefore, the heat resistance of the lubricant can be evaluated by measuring the change in viscosity.

Here, the total oxidation number is the amount, indicated in milligram units, of potassium hydroxide that is required for neutralizing the acid components included in the measured sample of 1 gram. In the case that the value of the total oxidation number is great, it means that the lubricant is oxidized under high temperature conditions and, furthermore, decomposition, or the like, has progressed and, as a result, the acid components have been generated. Accordingly, a great value for the total oxidation number indicates that deterioration, such as oxidation or decomposition, has occurred in the lubricant and, thereby, the heat resistance can be evaluated.

The results of the measurement of the change in viscosity according to the accelerated high temperature test are shown in Fig. 1. The lateral axis shows the period of time during which high temperature conditions are maintained and the longitudinal axis the viscosity after every constant period of time has elapsed. Here, the measurement of the viscosity is carried out at 40°C. P1 is the lubricant of the first reference example, P2 is the lubricant of the second reference example, P3 is the lubricant of the third reference example, P4 is the lubricant of the fourth example, Q1 is the lubricant of the first comparative example and Q2 is the lubricant of the second comparative example.

In the case of the lubricant Q1 (characterized by DOS) of the first comparative example, the viscosity increases in an approximately linear manner as time elapses and is saturated at the point in time when approximately 340 hours has elapsed. In the case that the change in viscosity occurs in such a manner, bearing loss becomes large under the utilization condition wherein the bearing part becomes of .a high temperature so that utilization of the lubricant becomes difficult. In addition, the change in viscosity has occurred after approximately 170 hours have elapsed in the lubricant Q2 (characterized by the mixed ester) of the second comparative example, which has a good heat resistance in comparison with the lubricant Q1 of the first comparative example but which, however, is recognized to have a problem of

reliability over long periods of time.

On the other hand, the change in viscosity of the lubricant of the first to fourth examples and reference examples is barely detectable as time elapses during the accelerated high temperature test and, therefore, it is confirmed that the heat resistance stability is excellent.

Next, the results of the measurement of the total oxidation numbers of the above described six types of lubricants after 627 hours has elapsed are shown in (Table 3).

[Table 3]

| | First Reference Example | Second Reference Example | Third Reference Example | Fourth Example | First Comparative Example | Second Comparative Example |
|---|---|---|---|---|---|---|
| total oxidati on number (mgKOH/ g) | 0.00 | 0.91 | 0.00 | 0.37 | 18.16 | 1.00 |

All of the lubricants of the first to fourth examples and reference examples have values of the total oxidation number smaller than those of the first and second comparative examples so that a good heat resistance is confirmed. In particular, the values of the total oxidation number of the lubricant (characterized by $C_7H_{15}$) of the first reference example and the lubricant (characterized by $C_9H_{19}$) of the third reference example are 0 and it is recognized that they are very stable lubricants wherein decomposition does not occur even under high temperature conditions.

On the other hand, in the case of the lubricant (characterized by DOS) of the first comparative example, the value of the total oxidation number is great and, therefore, it can be seen that decomposition has occurred during the accelerated high temperature test. That is to say, it has become clear that the lubricant of the first comparative example has a poor heat resistance stability and, accordingly, has a great change in viscosity.

In addition, in the case of the lubricant (characterized by mixed ester) of the second comparative example, the value of the total oxidation number is slightly greater than that of the lubricant (characterized by $C_8H_{17}$) of the second reference example while change in viscosity is clearly greater than that of the second example. As a result of this, it was observed that change in viscosity occurred in the lubricant of the second comparative example not because the mixed ester, which is a composition, decomposed but,' rather, because the mixed ester was chemically polymerized. That is to say, a chemical polymerization occurs under high temperature conditions in the lubricant of the second comparative example wherein an base oil made of the mixed ester is used while the lubricant wherein a base oil of a single ester, in which only one type from among $C_7H_{15}$, $C_8H_{17}$ or $C_9H_{19}$ is used as the alkyl base $R=C_nH_{2n+1}$ in (chemical formula 6) of the present invention, was observed to be stable even at high temperatures.

Here, when the lubricant (characterized by $C_8H_{17}$) of the second reference example and the lubricant (characterized by $C_8H_{17}$ + a triglyceride) of the fourth example are compared, the result is gained such that the value of the total oxidation number of the lubricant of the fourth example is approximately 60% smaller than the that of the lubricant of the second reference example. It has become clear from this that the triglyceride has the effect of suppression of the decomposition of the base oil.

Here, an accelerated high temperature test using a copper alloy material, on which nickel phosphorus plating is not carried out, is performed on the lubricants of the first to fourth examples and reference examples wherein change in viscosity has not occurred with respect to the copper alloy material on which nickel phosphorus plating is carried out. This result is shown in Fig. 2. The test conditions are similar to the above described accelerated high temperature test.

As can be seen from Fig. 2, it becomes clear that change in viscosity does not occur when a copper alloy material on which nickel phosphorus plating is not carried out is used and a good heat resistance stability is observed.

Here, as for the metal material utilized for a rotating shaft and for a bearing part, not only the above described copper alloys on which nickel phosphorus plating is carried out or on which nickel phosphorus plating is not carried out but, also, there is a possibility that a variety of other materials may be utilized and in such a case a coating by means of plating, or the like, may be applied to the surface in accordance with the material or an inhibitor for inhibiting metal corrosion or metal deactivator may be added to the lubricant.

Next, the result of evaluation of slidability characteristics when contact with metal has occurred. Contact with metal occurs when the oil film is broken immediately after the start up of, or immediately before stoppage of, the motor to which a dynamic pressure hydraulic bearing is mounted. When contact with metal has occurred, the friction coefficient becomes large and, therefore, a large amount of wear is caused.

An evaluation method of the slidability characteristics when contact with metal has occurred is carried out by measuring the friction coefficient using a pinion disk testing apparatus. A pin made of stainless steel generally used for a rotating shaft and a disk made of a copper alloy on which nickel phosphorus plating coating film is formed and which

is, in some cases, utilized for a bearing part are utilized in the pinion disk test. As for test conditions, the speed of the disk relative to the pin is set at 0.16 m/sec and the load added to the pin is set at 624 mN. The result of the test is shown in Table 4.

[Table 4]

| | First Reference Example | Second Reference Example | Third Reference Example | Fourth Example | First Comparative Example | Second Comparative Example |
|---|---|---|---|---|---|---|
| Friction coefficient | 0.15 | 0.14 | 0.14 | 0.12 | 0.16 | 0.15 |

The result is gained that all of the friction coefficients of the lubricants of the first to fourth examples and reference examples are smaller than those of the lubricants of the first and second comparative examples. Among these, the lubricant of the fourth example has the lowest friction coefficient and the effects of the addition of triglyceride are clearly confirmed.

The results of the comparison of the performance of lubricants of the first to fourth examples and reference examples with the lubricants of the first and second comparative examples are summarized as follows.

That is to say, though the lubricant (characterized by $C_7H_{15}$) of the first reference example has a low viscosity and heat resistance is good, the amount of vaporization is slightly greater than that of the lubricant of the second comparative example having the mixed ester as the base oil. However, heat generation due to friction of the lubricant itself is small because of its low viscosity and, therefore, the generation of heat can be restrained in comparison with the second comparative example. Accordingly, even in the case that the amount of vaporization is slightly greater than that of the second comparative example, the lubricant of the first reference example has an overall stability at a high temperature in comparison with the second comparative example.

The lubricant (characterized by $C_8H_{17}$) of the second reference example and the lubricant ($C_8H_{17}$ + a triglyceride) of the fourth example have better characteristics from an overall point of view than those of the first and second comparative examples and it can be seen that they are the lubricants having a high heat resistance and small bearing loss.

On the other hand, the lubricant (characterized by $C_9H_{19}$) of the third embodiment has a viscosity greater than that of the lubricant made up of the mixed ester of the second comparative example. However, the amount of vaporization is very small and the value of the total oxidation number is 0 so that the heat resistance is excellent. As a result of this, a dynamic pressure hydraulic bearing of a high reliability can be gained wherein oxidation, decomposition, or the like, does not occur even though the viscosity is great so that the friction of the lubricant itself easily causes heat generation.

As described above, the lubricants of the present examples all have, overall, superior characteristics in comparison to the comparative examples and, therefore, lubricants, having excellent reliability at high temperature could be achieved.

Here, a variety of additives such as, for example, oil agent, metal corrosion inhibitor or metal deactivator may further be added in accordance with the environment or conditions wherein the dynamic pressure hydraulic bearing will be utilized.

Moreover, the lubricant (characterized by $C_7H_{15}$) of the first example has the lowest viscosity and has excellent heat resistance and, therefore, this is appropriate as a lubricant for, for example, a motor for driving a rotational drum-shaped head of a video recorder in which a camera is integrated or for a dynamic pressure hydraulic bearing of a spindle motor for a mobile apparatus.

In addition, the lubricant (characterized by $C_7H_{15}$) of the second reference example and the lubricant (characterized by $C_8H_{17}$ + a triglyceride) of the fourth example have overall superior performance to in comparison with the comparative examples and are well-balanced lubricants. In addition, the lubricant of the fourth example has a small friction coefficient and, therefore, is appropriate for the applications wherein a motor is frequently started up or stopped.

Furthermore, though the viscosity is greater than that of the lubricant (characterized by the mixed ester) of the second comparative example, the lubricant (characterized by $C_9H_{19}$) of the third reference example has a small vaporization loss and has an excellent resistance to oxidation and decomposition and an excellent heat resistance so that no deterioration occurs even when the bearing part is exposed to a high temperature and, therefore, is suitable as a lubricant for dynamic pressure hydraulic bearings requiring a high reliability and long life.

The above described lubricant of the first' embodiment can be summarized as follows.

A base oil of a single ester having any of the structures of (chemical formula 7), (chemical formula 8) or (chemical formula 9) is used as the base oil and at least one type from among hindered phenol-based antioxidants (having at least one (3, 5-di-tert-butyl-4-hydroxyphenyl)) or hindered amine-based antioxidants is included and, furthermore, a triglyceride is included for the reduction of the friction coefficient in the configuration and, thereby, the viscosity characteristics are

adjusted to be 48 mPa·s or less at 0°C and 12 mPa·s or less at 40°C in these lubricants. Then these lubricants are mainly utilized for dynamic pressure hydraulic bearings.

Since the lubricants have such compositions, the low viscosity can be maintained under low temperature conditions decomposition or oxidation does not occur at high temperatures so that high heat resistance stability can be achieved. Furthermore, vaporization loss is small and, at least, copper alloy materials on which nickel phosphorus plating has been carried out, or on which nickel phosphorus plating has .not been carried out, do not cause change in quality of the lubricant. Accordingly, the bearing loss is small even when the lubricant is utilized at low temperatures and the lubricant can be utilized so as to have a high reliability even at the time of high-speed rotation wherein the temperature becomes high and, therefore, significant effects are gained such that a dynamic pressure hydraulic bearing that can be utilized at the time of high-speed rotation or over a broad temperature range can be achieved.

(§2)

(Second Embodiment)

The second embodiment of the reference aspect relates to an analysis method of a lubricant (base oil). This analysis method of a lubricant is a technology for identifying a lubricant, from among other lubricants, and the like, of which the base oil is a single ester having the above described structure of (chemical formula 6) and having only one type of alkyl base $R=C_nH_{2n+1}$ wherein n = 7, n = 8 or n = 9.

(§3)

An analysis method of a lubricant of the reference aspect is based on the following principle.

[chemical formula 11]

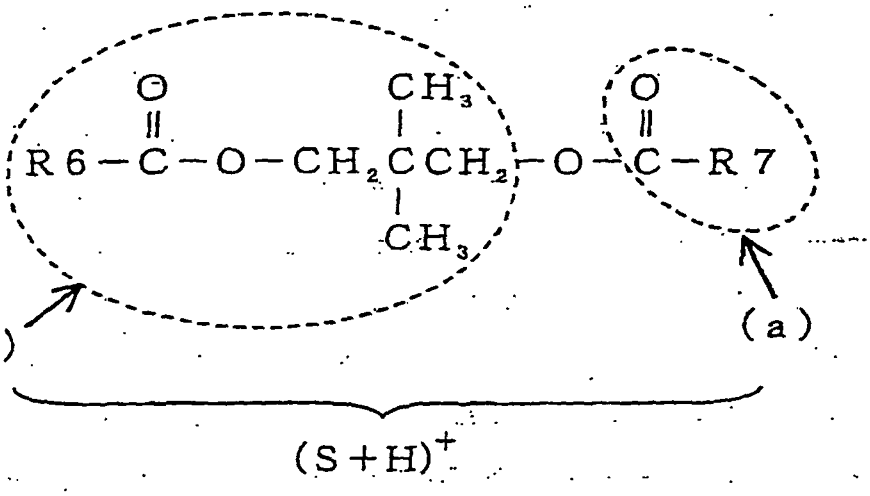

One molecule of the base oil that is a single ester molecule having- a'molecular structure shown by (chemical formula 11) is represented by the symbol S (here, S is not sulfur). An H atom- is joined to this base oil molecule S which is ionized so as to be represented as $(S + H)^+$. In the case that the base oil molecule S decomposes, an (a) portion is ionized or a (b) portion is ionized. When the mass numbers are assumed such that H = 1, C = 12 and O = 16, the respective mass numbers of the (a) portion, the (b) portion and the $(S + H)^+$ portion are calculated.

[1] If $R6 = R7 = C_7H_{15}$,

In the (a) portion in (chemical formula 11),the number of H, C and O are 15, 8 and 1, respectively, because R7 is $C_7H_{15}$, and the mass number is

$$1 \times 15 + 12 \times 8 + 16 \times 1 = 127.$$

In the (b) portion, the number of H, C, and O are 25, 13 and 2, respectively, because R6 is also $C_7H_{15}$ and the mass number is

$$1 \times 25 + 12 \times 13 + 16 \times 2 = 213.$$

The oil molecule S is gained by adding O(of which the number is one) into the (a) portion and the (b) portion. One H atom is joined to this base oil molecule S which is ionized so as to become $(S + H)^+$. The mass number of $(S + H)^+$ becomes

$$127 + 213 + 16 + 1 = 357.$$

Accordingly, the mass number for specifying the base oil that is a single having the structure of (chemical formula 6) in the case of alkyl base R=$C_7H_{15}$ is a combination of "127," "213" and "357." Then, "127," "213" and "357" are assumed to be the first group with respect to the combination of masses of positive secondary ions per charge unit at the time of analysis by means of a secondary ion mass spectrometer.

In the case that a peak is gained in the secondary ion intensity at the mass numbers of "127," "213" and "357" of the first group, a structure can be specified that corresponds to the case wherein R6 and R7 are both the alkyl base of $C_7H_{15}$ in (chemical formula 11).

[2] case wherein R6 = R7 = $C_8H_{17}$

Since R7 is $C_8H_{17}$, the (a) portion in (chemical formula 11) has two additional H and one additional C in comparison with the first group according to the calculation and, therefore, the mass number is

$$127 + 1 \times 2 + 12 \times 1 = 127 + 14 = 141.$$

Since R6 is also $C_8H_{17}$, the (b) portion in (chemical formula 11) has two additional H and one additional C in comparison with the first group according to the calculation and, therefore, the mass number is

$$213 + 14 = 227.$$

Then, the $(S + H)^+$ portion has two additional sets of two H and one C in comparison with the first group according to the calculation and, therefore, the mass number becomes

$$357 + 14 \times 2 = 385.$$

Accordingly, the mass number for specifying the base oil that is a single ester having the structure of (chemical formula 6) in the case of alkyl base R = $C_8H_{17}$ is a combination of "141," "227" and "385." Then, "141," "227" and "385" are assumed to be the second group with respect to the combination of masses of positive secondary ions per charge unit at the time of analysis by means of a secondary ion mass spectrometer.

In the case that a peak is gained in the secondary ion intensity at the mass numbers of "141," "227" and "385" of the second group, a structure can be specified that corresponds to the case where R6 and R7 are both the alkyl base of $C_8H_{17}$ in (chemical formula 11).

[3] case wherein R6 = R7 = $C_9H_{19}$

Since R7 is $C_9H_{19}$, the (a) portion in (chemical formula 11) has two additional H and one additional C in comparison with the second group according to the calculation and, therefore, the mass number is

$$141 + 14 = 155.$$

Since R6 is also $C_9H_{19}$, the (b) portion in (chemical formula 11) has two additional H and one additional C in comparison with the second group according to the calculation and, therefore, the mass number is

$$227 + 14 = 241.$$

Then, the $(S + H)^+$ portion has two additional sets of two H and one C in comparison with the second group and, therefore, the mass number becomes

$$385 + 14 \times 2 = 413.$$

Accordingly, the mass number for specifying the base oil that is a single ester having the structure of (chemical formula 6) in the case that the alkyl base R = $C_9H_{19}$ is a combination of "155," "241" and "413." Then, "155," "241" and "413" are assumed to be the third group with respect to the combination of the masses of the positive secondary ions per charge unit at the time of analysis by means of a secondary ion mass spectrometer.

In the case that a peak is gained in the secondary ion intensity at the mass numbers of "155," "241" and "413" of the third group, a structure can be specified that corresponds to the case wherein R6 and R7 are both the alkyl base of $C_9H_{19}$ in (chemical formula 11).

The above descriptions are summarized as follows. That is to say, whether or not a peak in each of the mass numbers of "127," "213" and "357" of the first group is gained is determined, in an analysis method of a lubricant according to the reference aspect, with respect to the peak of the positive secondary ion intensity per unit charge when secondary ion mass spectrometry is utilized. In the case that this determination becomes affirmative, the lubricant, of which the base oil is a. single ester having the structure of (chemical formula 6) wherein alkyl base R = $C_7H_{15}$, can be identified.

In addition, whether or not a peak in each of the mass numbers of "141," "227" and "385" of the second group is gained is determined, in an analysis method of a lubricant according to another embodiment of the reference aspect, with respect to the peak of the positive secondary ion intensity per unit charge when secondary ion mass spectrometry is utilized. In the case that this determination becomes affirmative, the lubricant, of which the base oil is a single ester having the structure of (chemical formula 6) wherein alkyl base R = $C_8H_{17}$, can be identified.

In addition, whether or not a peak in each of the mass numbers of "155," "241" and "413" of the third group is gained is determined, in an analysis method of a lubricant according to still another embodiment of the reference aspect, with respect to the peak of the positive secondary ion intensity per unit charge when secondary ion mass spectrometry is utilized. In the case that this determination becomes affirmative, the lubricant, of which the base oil is a single ester having the structure of (chemical formula 6) wherein alkyl base R = $C_9H_{19}$, can be identified.

Here, in any case of the above described analysis method of a lubricant, when the mass number of the base oil molecule S, which is the object of determination, is M and the difference between the mass number of the base oil molecule and the adjoining mass number gained by static secondary ion mass spectrometry is $\Delta M$, an analysis may be carried out under the conditions of $(M + \alpha)$ or less, with respect to the mass number resolution represented by $M/\Delta M$ when $\alpha$ is a positive number.

In the case that the mass number per charge unit of the base oil molecule S is "357," the mass number resolution may be $(357 + \alpha)$ or less, in order to significantly identify the mass number of "1." In the case that the mass number per charge unit of the base oil molecule S is "385," the mass number resolution may be $(385 + \alpha)$, or less. In the case that the mass number per charge unit of the base oil molecule S is "413," the mass number resolution may be $(413 + \alpha)$ or less. Accordingly, the conditions can be set such that the mass number resolution is, for example, 500 or less.

Here, the base oil is allowed to have a peak in only one group from among the first to third groups. In the case that the base oil has peaks in two, or more, groups, the base oil includes a mixed ester and, therefore, this case is excluded. (§4)

Such a reaction of decomposition as is seen in di-esters, represented by DOS (sebacic acid di-2-ethylhexyl) that is a conventional base oil, does not easily occur. Di-esters, represented by DOS, have, in general, the structure represented by (chemical formula 12). R4 and R5, respectively, shown herein indicate a straight chain or a branched alkyl base formed of CmHn (m and n are integers).

[chemical formula 12]

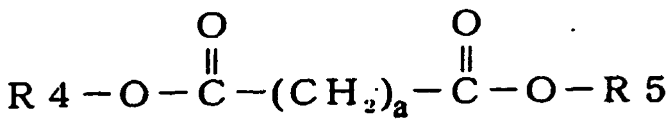

In the case that R4 and R5 have the same structure and are straight chain alkyl bases formed of $CH_2CH_2R$, molecular motion of the base oil becomes violent when exposed to high temperature so that the structure of (chemical formula 12) easily changes to the structure of (chemical formula 13) and pseudo-bonds 1 and 2 of O-H are created.

[chemical formula 13]

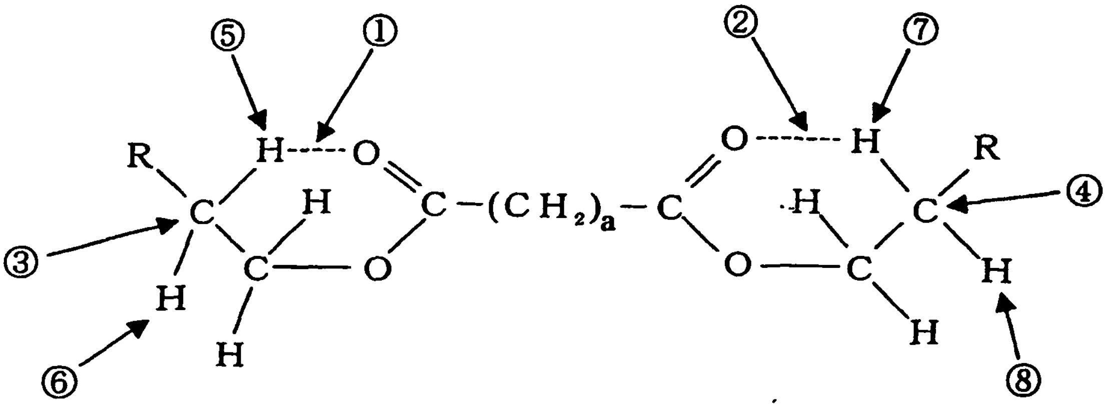

That is to say, at least one of H (of which the number is 5, 6, 7 or 8) that are bonded to C (of which the number is 3) or C (of which the number is 4) in (chemical formula 13) is shifted by means of a given thermal energy and, thereby, a reaction as in (chemical formula 14) occurs so that the di-ester decomposes comparatively easily.

[chemical formula 14]

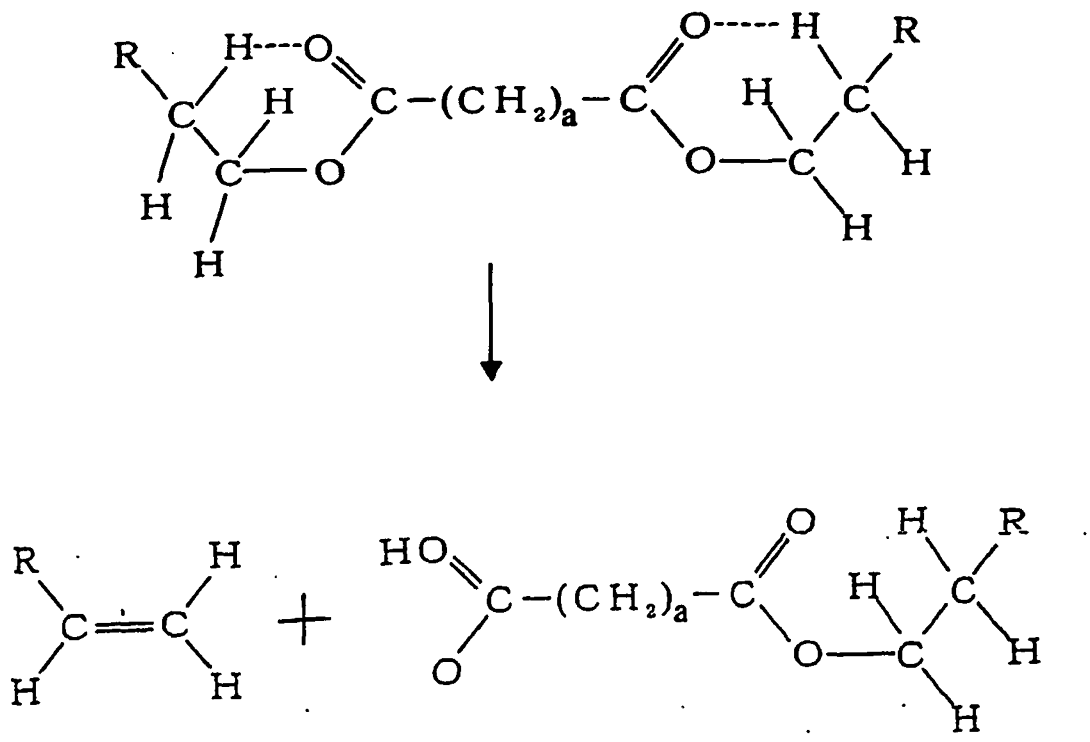

Here, (chemical formula 14) shows a case of the structure wherein H atom 5 or H atom 7, shown in (chemical formula 13), converts to a transition state. As described above, di-esters having R4 and R5 as straight chain alkyl bases are poor in thermal stability.

It is possible that at least one of H atoms 5, 6, 7 or 8 that are bonded to C atom 3 or C atom 4, shown in (chemical formula 13), is used in a branched alkyl base R4 or R5 formed of a plurality of C atoms, such as a methyl base or an ethyl base. In this case the reaction shown in (chemical formula 14) can be prevented due to the three-dimensional blocking effect. However, when such a branched alkyl base is used, the viscosity of the lubricant becomes high. Accordingly, it is difficult for good heat resistance and low viscosity to be simultaneously attained in a di-ester. Thus, though the stability and the viscosity of the lubricant are greatly restricted depending on the characteristics of the base oil, it is difficult to easily evaluate whether or not the base oil has a specific characteristic.

In contrast to this, by carrying out static secondary ion mass spectrometry as in the reference aspect, the base oil component, even of a microscopic amount, can be easily specified with a high precision. The base oil identified in the above described manner does not easily cause a reaction, as shown in (chemical formula 14).

Then, a lubricant is prepared by using a base oil that is determined in the above described manner. The component of this lubricant is the same as in the case of the first embodiment. That is to say, the above described base oil is used as the main body and, furthermore, at least one type of antioxidant from among hindered phenol-based antioxidants or hindered amine-based antioxidants is included as an additive and an additive for the reduction of the friction coefficient is required and a lubricant of the present invention includes a triglyceride, of which the structure is shown in (chemical formula 10), as an additive. Qualities requirements and qualitative requirements of the above are the same as in the case of the first embodiment.

As described above, the lubricant (lubricant composition) of the present embodiment is characterized in that a hindered phenol-based antioxidant or a hindered amine-based antioxidant, or both of these, as well as a triglyceride are added to the base oil of a single ester having the structure of (chemical formula 7), (chemical formula 8) or (chemical formula 9) that is identified by means of an analysis method, including the above described secondary ion mass spectrometry, as substitutes for DOS (sebacic acid di-2-ethylhexyl) that is a representative example of a conventional diester, (trimethylolpropane + monovalent fatty acid) that is a representative example of a triester or a mixed ester of a capryl acid of neopentylglycol and capric acid and, thereby, the viscosity characteristics are adjusted so that the viscosity at 0°C is 48 mPa · s or less and the viscosity at 40°C is 12 mPa · s or less. Thereby, the low viscosity can be maintained even in low temperature conditions and decomposition or oxidation does not occur at high temperatures and, then, a

lubricant of which the friction coefficient is low can be provided. Accordingly, in the case that the lubricant is utilized for a dynamic pressure hydraulic bearing, bearing loss is small even when it is utilized at a low temperature and the lubricant can be stably utilized at the time of high-speed rotation wherein the temperature becomes high. Furthermore, a lubricant for a dynamic pressure hydraulic bearing can be provided having very stable characteristics wherein change in viscosity and vaporization loss are low and heat resistance is excellent so that oxidation or decomposition is not easily caused even at high temperatures.

[Reference Examples of the Second Embodiment]

In the following, the reference examples of the second embodiment are used to describe the present invention in detail.
The measurement conditions in Table 5. $Ga^+$ is used as a primary ion, the irradiation energy is 12 keV, the polarity of the measured secondary ion is positive and the detection region is 40 $\mu m^2$.

[Table 5]

| | |
|---|---|
| primary ion | Ga+ |
| primary ion irradiation energy | 12kev |
| secondary ion polarity | Positive |
| detection region | 40μm×40μm |

(First Reference Example)

A lubricant of the first reference example was prepared as follows. The following was used as the base oil. That is to say, when the base oil is analyzed by using a secondary ion mass spectrometer, the secondary ion intensity has peaks at, respectively, the positions of mass numbers of "127," "213" and "357" of the first group. This base oil is single ester having the structure of (chemical formula 6), which corresponds to the lubricant in the case of alkyl base R = $C_7H_{15}$-1 wt.% of a hindered phenol-based antioxidant having four (3, 5-di-tert-butyl-4-hydroxyphenyl) is added to this base oil as an antioxidant. This is substantially the same as (the first reference example) of the first embodiment.
The measurement result with respect to the base oil is shown in Fig. 3. (a), (b) and $(S + H)^+$ in Fig. 3 are peaks corresponding to the fragments shown in (chemical formula 11), of which the mass numbers are "127," "213" and "357," respectively.

(Second Reference Example)

A lubricant of the second reference example was prepared as follows. The following was used as the base oil. That is to say, when the base oil is analyzed by using a secondary ion mass spectrometer, the secondary ion intensity has peaks at, respectively, the positions of mass numbers of "141," "227" and "385" of the second group. This base oil is single ester having the structure- of (chemical formula 6), which corresponds to the lubricant in the case of alkyl base R = $C_8H_{17}$. 1 wt.% of a hindered phenol-based antioxidant having four (3, 5-di-tert-butyl-4-hydroxyphenyl) is added to this base oil as an antioxidant. This is substantially the same as (the second reference example) of the first embodiment.
The measurement result with respect to the base oil is shown in Fig. 4. (a), (b) and $(S' + H)^+$ in Fig. 4 are peaks corresponding to the fragments shown in (chemical formula 11), of which the mass numbers are "141," "227" and "385," respectively.

(Third Reference Example)

A lubricant of the third reference example was prepared as follows. The following was used as the base oil. That is to say, when the base oil is analyzed by using a secondary ion mass spectrometer, the secondary ion intensity has peaks at, respectively, the positions of mass numbers of "155," "241" and "413" of the third group. This base oil is single ester having the structure of (chemical formula 6), which corresponds to the lubricant in the case of alkyl base R = $C_9H_{19}$. 1 wt.% of a hindered phenol-based antioxidant having four (3, 5-di-tert-butyl-4-hydroxyphenyl) is added to this base oil as an antioxidant. This is substantially the same as (the third reference example) of the first embodiment.
The measurement result with respect to the base oil is shown in Fig. 5. (a), (b) and $(S + H)^+$ in Fig. 5 are peaks corresponding to the fragments shown in (chemical formula 11), of which the mass numbers are "155," "241" and "413," respectively.

(Fourth Reference Example)

A lubricant of the third example was prepared as follows. That is to say, when the base oil is analyzed by using a secondary ion mass spectrometer, the secondary ion intensity has peaks at, respectively, the positions of mass numbers of "141," "227" and "385" of the second group. This base oil is single ester having the structure of (chemical formula 6), which corresponds to the lubricant in the case of alkyl base R = $C_8H_{17}$. 1 wt.% of a hindered phenol-based antioxidant having four (3, 5-di-tert-butyl-4-hydroxyphenyl) is added to this base oil as an antioxidant. In addition to this, 1 wt.% of a triglyceride, shown in (chemical formula 10) is added. Here, R1, R2 and R3 have the same structure and have the structure of CxHyOz wherein the value of x is 17, the value of In the above described aspect, is 33 and the value of z is 1. This is substantially the same as (the fourth example) of the first embodiment.

The measurement result with respect to the base oil is shown in Fig. 6. (a), (b) and $(S + H)^+$ in Fig. 6 are peaks corresponding to the fragments shown in (chemical formula 11), of which the mass numbers are "141," "227" and "385," respectively. In the case of the fourth reference example, it can be confirmed that the composition of the base oil is of the same structure as that of the second reference example by comparing the peaks in Fig. 4 and in Fig. 6.

(Comparative Example)

DOS (sebacic acid di-2-ethylhexyl), which is a lubricant that has been conventionally used with dynamic pressure hydraulic bearings, was used as a comparative example. This is substantially the same as the first comparative example in the first embodiment. The spectrum measured by a mass spectrometer under the conditions described in the first reference example is shown in- Fig. 7. Here, CO- $(CH_2)_8$-COOH in (chemical formula 12) corresponds to the mass number "185."

The peak positions and the intensities thereof of this comparative example are totally different from the ester-based base oil in the first to fourth reference examples and the difference thereof can be clearly determined.

The explanations in from paragraph (§1) to paragraph (§2) in the descriptions of the above first embodiment correspond to the descriptions of the reference examples in this second embodiment. Here, the descriptions with respect to the second comparative example are excluded because the present embodiment does not have a portion corresponding to the second comparative example.

(Third Embodiment)

The third embodiment of the reference aspect relates to a deterioration analysis method of a lubricant.

Here, static secondary ion mass spectrometry is described.

In secondary ion mass spectrometry, an accelerated primary ion is irradiated into the surface of a sample so as to generate a secondary ion and mass analysis is carried out on the generated secondary ion by using a mass spectrometer and, thereby, an element existing in the sample is identified. The acceleration energy,for the primary ion is from a few to several tens keV.

Static secondary ion mass spectrometry is a type of secondary ion mass spectrometry and the total amount of radiated primary ions is adjusted to be $10^{12}/cm^2$ or less. Thereby, the molecules existing on the surface of the sample have a portion of their bonds cut off by the irradiated primary ions so as to be ejected as molecular ions. These ejected molecular ions are detected. As a result, a mass spectrum representing the characteristics of the chemical bonds of the molecules existing on the surface of the sample can be gained. Time of flight secondary ion mass spectrometry (TOF-SIMS), wherein time of flight is used in mass spectrometry, is utilized in the present embodiment.

The potential gap applied between the sample and the detector is utilized so that the generated secondary ions fly from the sample to the detector so as to be detected by the detector and, thereby, mass segregation is carried out in TOF-SIMS. The energy E given to a monovalent ion by a voltage gap is represented by (equation 2) wherein the mass of the ion is M and the velocity thereof is V.

[equation 2]

$$E = M \cdot V^2 / 2$$

When the distance between the sample and the detector is denoted as L and the time required for the ion generated on the surface of the sample to reach the detector is denoted as t, the velocity V is represented by (equation 3).

[equation 3]

$$V = L / t$$

(equation 4) is led out from the above (equation 2) and (equation 3) so that the mass of an ion can be determined by measuring the time required for the ion to reach the detector.

[equation 4]

$$M = 2 \cdot E \cdot t^2 / L^2$$

As is seen from (equation 4), the smaller the mass of an ion is, the sooner the ion reaches the detector. The amount of detected ions is monitored as the secondary ion intensity.

In the case that a lubricant utilized with a dynamic pressure hydraulic bearing is evaluated, the amount of oil used as the lubricant is extremely small, such as approximately several μL (microliter). According to TOF-SIMS, however, the identification of an element existing at the level of ppm and analysis of an organic molecule can be simultaneously carried out even in the case that the sample is of an extremely small amount. In addition, an organic substance or an inorganic impurity at the level of one molecular layer that exists on the surface of the electrode can be analyzed with a high sensitivity and, in particular, status analysis is possible. Accordingly, deterioration of the base oil of the lubricant can be analyzed by analyzing change in intensity of the mass spectrum. In addition, the identification of a metal element that has been mixed into the lubricant composition and the amount of the mixed in element can be analyzed. Here, a general secondary ion mass spectrometer may, of course, be used in the present invention without being limited to TOF-SIMS.

In the following, a concrete deterioration evaluation method is described. The intensity of secondary ions having a specific mass number is denoted as $(I_R)_{ref}$ and the intensity of the secondary ions having the mass number of interest is denoted as $(I_n)_{ref}$ in the spectrum gained by carrying out a secondary ion mass spectrometry on the lubricant in the initial condition. In addition, the intensities of secondary ions of the same mass number as that gained in the lubricant after utilization for a specific period of time under specific conditions are, respectively, denoted as $(I_R)_{after}$ and $(I_n)_{after}$.

The ratio of the secondary ion intensity of the mass number of interest to the secondary ion intensity of the specific mass number before the test is

[equation 5]

$$IR_{ref} = \frac{(I_n)_{ref}}{(I_R)_{ref}}$$

In addition, the ratio of the secondary ion intensity of the mass number of interest to the secondary ion intensity of the specific mass number after the test is

[equation 6]

$$IR_{after} = \frac{(I_n)_{after}}{(I_R)_{after}}.$$

A variety of combinations exist concerning which ion is assigned as the ion $(I_R)$ of the specific mass number and which ion is designated as the ion $(I_n)$ of the mass number of interest. That is to say, in (equation 5) and (equation 6) any of (a), (b) or $(S + H)^+$ in (chemical formula 11) may be placed as denominator and any of (a), (b) or $(S + H)^+$ in (chemical formula 11) may be placed as the numerator under the condition that it is different from the denominator.

Then, the ratio IR (intensity ratio) of change of a fragment in the lubricant is described by (equation 7).

[equation 7]

$$IR = \frac{IR_{after}}{IR_{ref}} = \frac{\dfrac{(I_n)_{after}}{(I_R)_{after}}}{\dfrac{(I_n)_{ref}}{(I_R)_{ref}}}$$

The above described specific mass number and the mass number of interest are concretely described below.

In the case of the first group, the mass numbers are a combination of any of two from among the values of "127," "213" and "357." In the case of the second group, they are a combination of any of two from among the values of "141," "227" and "385." In the case of the third group, they are a combination of any of two from among the values of "155," "241" and "413."

In the case that the ratio IR of change is "1," the fragmented ionic species of interest has not changed and, therefore, it is shown that deterioration of the lubricant has not occurred. In addition, in the case that the ratio IR of change is greater than "1," it is shown that this ionic species has increased, while in the case that the ratio IR of change is smaller than "1," it is shown that this ionic species has decreased so that in either case it can be determined that that greater is the difference between the ratio IR of change and 1, the more the deterioration of the lubricant has progressed. As described above, deterioration of a lubricant can be analyzed from the fluctuation of the peaks the fragmented ion of interest.

[Reference Example of the Third Embodiment]

This reference example is a lubricant that includes a single ester base oil having the structure shown in (chemical formula 6) wherein alkyl base R = $C_nH_{2n+1}$ is a straight chain saturated alkyl base of $C_8H_{17}$ in the case wherein n = 8. This is a lubricant wherein one wt.% of a hindered phenol-based antioxidant having four (3, 5-di-tert-butyl-4-hydroxyphenyl) as an antioxidant is added to the above base oil. The result of the analysis of deterioration at the time of the utilization of the lubricant of this composition is described below.

A starting and stopping test of a motor having a dynamic pressure hydraulic bearing wherein a lubricant of this reference example is used is carried out for 1000 hours at room temperature. The test is carried out on two types of motors having a bearing part wherein brass, which is a copper alloy including zinc, is partially used and having a bearing part wherein nickel phosphorus plating is carried out on the surface of this brass. The lubricants after the test are extracted so as to analyze the deterioration thereof by means of TOF-SIMS. The analysis conditions of TOF-SIMS are the same as the conditions described in Table 5.

A ratio of the secondary ion intensity of the peak of $(S + H)^+$, wherein an H atom is attached to one molecule, from among the spectrum gained by the measurement to the secondary ion intensity of peak of (b) gained by the same measurement:

$$IR_{after} = (S + H)^{+}_{after} / (b)_{after}$$

is found. In addition, the same ratio with respect to the lubricant before the test:

$$IR_{ref} = (S + H)^{+}_{ref} / (b)_{ref}$$

is found and, then, the ratio IR of change in the ratio of $IR_{after}$ to this $IR_{ref}$, that is to say,

$$IR = IR_{after} / IR_{ref}$$

is used to evaluate the degree of deterioration. This value is shown in Table 6.

[Table 6]

| | | Brass bearing part | NiP plating bearing part |
|---|---|---|---|
| $IR_{after}/IR_{ref}$. | | 0.59 | 0.90 |
| metal mixed into lubricant | Cu | 196 | 1 |
| | Pb | 586 | 1 |
| | Fe | 103 | 1 |

As is seen in Table 6, it is recognized that the degree of deterioration is small in the case of the motor having the bearing part on which nickel phosphorus plating has been carried out and deterioration of the lubricant is great in the motor having the bearing part wherein brass is used without change.

Table 6 shows metal components mixed into the lubricants, which are simultaneously analyzed, in relative ratios to the respective components in the lubricants before the test. In the case that the relative ratio is "1," it indicates that the metal component in the lubricant has not changed after the test in comparison to the condition before the test. In the case that the relative ratio is "196," for example, it indicates that an amount of the metal component 196 times greater than in the lubricant before the test has been extracted. In the case that brass was used, Pb, which is an impurity component of brass, and Fe, which is a component of stainless steel, used as the material of the bearing part, greatly increased in the same manner as Cu. It has become clear that when these metal components have increased, deterioration of the lubricant has occurred.

Thus, TOF-SIMS is used and, thereby, the deterioration of the lubricant utilized in an actual motor and the metal components that have been mixed in can be analyzed. Furthermore, deterioration of the lubricant used for a bearing part having even a very small diameter can be analyzed with a high precision because the sample amount required for measurement may be an extremely small amount, to the degree of several $\mu$L (microliters).

Here, though secondary ions having a positive polarity were used in the static secondary ion mass spectrometry in the present example, secondary ions having a negative polarity may be used. Here, in this case the detected peaks differ and, therefore, negative peaks corresponding to those of the positive case may be used. In addition, though $(S + H)^+$ and (b) are used as the values of $IR_{after}$ and $IR_{ref}$ in the present example, a ratio of any combination from among (a), (b) and $(S + H)^+$ may be used without being limited to the above.

The above described deterioration analysis method of a lubricant according to the third embodiment can be summarized as follows.

The ratio $IR_{ref}$ of the secondary ion intensity of the mass number of interest in the mass spectrum gained in secondary ion mass spectrometry to the secondary ion intensity of a specific mass number of a lubricant before utilization is found and, then, the ratio $IR_{after}$ of the secondary ion intensity of the mass number of interest to the secondary ion intensity of a specific mass number of the lubricant that has been utilized for a predetermined period of time in a dynamic pressure hydraulic bearing part is found in the same manner. Furthermore, the ratio $IR = IR_{after}/ IR_{ref}$ of a fragment in the lubricant, which is a ratio of these before and after utilization, is found. The above described mass number of interest and specific mass number are two of any mass numbers selected from among three mass numbers in only one of any group from among the above described three groups. The deterioration condition of the lubricant is evaluated based on the above described ratio IR of change. Thereby, the deterioration of the lubricant can be analyzed with a high precision even in the case that the sample is of an extremely small amount.

(Fourth Embodiment)

The fourth embodiment of the present invention relates to one modification of a lubricant according to the first embodiment. That is to say, it relates to a lubricant that includes a single ester base oil having the structure represented by (chemical formula 6) wherein the alkyl base $R = C_nH_{2n+1}$ is a straight chain saturated alkyl base of $C_8H_{17}$ in the case wherein n = 8 and, furthermore, that a mixed antioxidant formed of two types of antioxidants, that is to say, of a hindered phenol-based antioxidant and a hindered amine-based antioxidant, is added to the above base oil as an antioxidant. The above described hindered phenol-based antioxidant has at least one (3, 5-di-tert-butyl-4-hydroxyphenyl).

In the above description, the ratios in the mixture of the hindered phenol-based antioxidant and of the hindered amine-based antioxidant to the mixed antioxidant are approximately the same. The hindered phenol-based antioxidant or the hindered amine-based antioxidant has the function of a radical scavenger so as to prevent the oxidation of the base oil through self-oxidation at the time of heating.

These two types of antioxidants have antioxidation effects even in the case that they are used individually,

such as in the case of the first embodiment, and the antioxidation effects can be further increased so that that a sufficient effect can be gained in the case of the present embodiment wherein both of the antioxidants are added.

Then, in the case that the above described two types of antioxidants are added in approximately equal amounts to the base oil having the structure of (chemical formula 6) wherein the alkyl base R = $C_nH_{2n+1}$ is $C_8H_{17}$, the heat resistance stability can be improved without increasing viscosity. Accordingly, when the lubricant is utilized with a dynamic pressure hydraulic bearing it is stable even at a high temperature so that a reduction in the amount of power consumed can be implemented.

Furthermore, in the above description the mixed antioxidant content in the lubricant is adjusted between 0.1 wt.% or more and 8 wt.% or less. The base oil having the structure of (chemical formula 6), wherein the alkyl base R = $C_nH_{2n+1}$ is $C_8H_{17}$, is improved in regard to oxidation and deterioration accompanying the increase in the mixed antioxidant content so that the heat resistance stability increases while, on the other hand, the viscosity increases. Contrarily, in the case that the mixed antioxidant is not added at all, deterioration occurs for a very short period of time and the lubricant loses its function. Though the heat resistance stability depends on the utilization conditions of the dynamic pressure hydraulic bearing, a level for practical used can be secured by adding 0.1 wt.% or more of the mixed antioxidant. In addition, by adjusting the amount of the mixed antioxidant to 8 wt.% or less, the viscosity characteristics required for the case wherein the lubricant is used with a dynamic pressure hydraulic bearing, that is to say, a viscosity of 48 mPa · s or less at 0°C and a viscosity of 12 mPa · s or less at 40°C can be secured.

In the case that a motor wherein a dynamic pressure hydraulic bearing is used is mounted to a portable apparatus having a possibility of being utilized in low temperature conditions, such as' outdoors, it is important to lower the viscosity at low temperature conditions and, in this case, it is desirable for the added amount of the mixed antioxidant to be 5 wt.% or less.

Furthermore, it is according to the invention to add a triglyceride represented by the structure of (chemical formula 10) of an amount of 3 wt.% or less in order to reduce the friction coefficient. As for the triglyceride, the structures R1, R2 and R3, as well as such terms as x, y and z described in the first embodiment, are utilized.

Next, an example of a motor with a dynamic pressure hydraulic bearing wherein a lubricant according to the present invention is utilized is described.

Fig. 8 is a cross sectional view showing one example wherein the above described motor is applied in a hard disk device. Fig. 9 is an enlarged view of a portion thereof.

A sleeve member 20 formed of a bearing sleeve 8 and a thrust support plate 9 is attached to the center portion of a housing 7 and an axis member 3 of a rotational disk 1 is mounted to the sleeve member 20 so that it is freely rotatable. As shown in Fig. 9, lubricant 10 according to the present invention is sealed in a microscopic gap between the sleeve member 20 and the axis member 3. The rotational center is denoted as 4.

A stator 16 formed of a coupling part 13, an iron core 14 and a coil 15 is attached to the housing 7. On the other hand, a rotor yoke 11 is attached to the rotational disk 1 and a rotational magnet 12 is attached to the rotor yoke. The rotational disk 1, the rotor yoke 11 and the rotational magnet 12 form a rotor 21.

The annular bearing sleeve 8 in the sleeve member 20 is engaged with the center portion of the housing 7. The opening at one end of the sleeve member 20 is sealed by the thrust support plate 9. The annular coupling part 13 is secured to the housing 7 by being compressively inserted so as to be integrated. The coil 15 is wound around the iron core 14 extending toward the center from the coupling part 13.

The rotational disk 1 is formed of a disk part 2 and axis member 3 in a columnar form that is integrated with the center portion of the disk 2. A recording medium layer 6 made of a magnetic material is formed on the smooth main surface 5 of the disk part 2. This recording medium layer 6 is formed as a film of the magnetic material having a predetermined thickness by using thin film formation technology, such as vacuum deposition or sputtering.

The annular rotor yoke 11 in the rotor 21 is engaged and secured to a circular step portion 2a in the disk part 2. The annular rotational magnet 12, which has been magnetized in a plurality of places, is secured to the rotor yoke 11 by means of adhesive, or the like. An annular thrust suction plate 17 is secured to the housing 7 so as to be opposed to the rotational magnet 12.

A dynamic pressure generation trench is created on the thrust surface 18, which is the end surface of the axis member 3 opposed to the thrust support plate 9. In addition, a dynamic pressure generation trench (not shown) is created on the inner surface of the bearing sleeve 8 opposed to the external surface of the axis member 3. The form of this dynamic pressure generation trench is in a herringbone form or in a spiral form. For example, a trench 22 of a herringbone form may be created, or a trench of a spiral form may be created, on the thrust surface 18 of the axis member 3 in the thrust bearing part. Fig. 10 shows, as an example, the trench 22 in the herringbone form created in the thrust surface 18 of axis member 3.

When the rotor 21 is rotated by activating the coil 15 in the motor having the dynamic pressure hydraulic bearing applied in the hard disk device, as described above, dynamic pressure is generated by the effect of the lubricant 10 in the portion of the dynamic pressure generation trench so that the rotor 21 smoothly rotates.

A very thin type motor with a high rotational precision can be realized in the motor configuration using a dynamic

pressure hydraulic bearing in such a manner. For example, a hard disk device having a thickness of approximately 5 mm, or less, can be implemented.

Here, the present invention is not limited to the type in Fig. 8 but, rather, is applicable to a variety of motors. It may also be applied to a spindle motor configuration similar to that according to the prior art. In addition, it may also be applied to a motor for the rotation of a polygonal mirror, or the like. It is possible to be utilized with a dynamic pressure hydraulic bearing used in other rotational parts.

As described above, a dynamic pressure hydraulic bearing wherein a lubricant according to the present invention is used has an axis member and a sleeve member that engage with each other so as to be freely rotatable, the above described lubricant filled into a bearing gap between the two and a rotatable member integrally coupled to the axis member (or sleeve member) so as to form the configuration wherein dynamic pressure is generated due to the above described lubricant through the effect of dynamic pressure generation trench created in the axis member or in the sleeve member in the direction of thrust and in a radial direction so as to support the rotational member in the non-contact condition.

Furthermore, it is possible to form a thin type motor by using such a dynamic pressure hydraulic bearing. That is to say, this motor is provided with a housing, a stator secured to this housing and that generates a magnetic field in order to provide rotational force to a rotor, the rotor having a rotational magnet that is magnetized to a plurality of magnetic poles and that is secured in order to be opposed to the magnetic field generation part of the stator and a bearing part for support of the axis of the rotor so that it is freely rotatable and has the configuration such that this bearing part corresponds to the above described dynamic pressure hydraulic bearing.

A dynamic pressure hydraulic bearing or a motor formed in such a manner can be formed not only in a thin type form but can also be stably operated in a manner that power consumption is low over a broad temperature range and wherein vaporization loss is small. The vaporization of the lubricant thereof is low and the lubricant has a low viscosity and the fluctuation of viscosity change in temperature is small and, moreover, stabilization against oxidation is excellent. Accordingly, a dynamic pressure hydraulic bearing and a motor of which bearing loss is small over a range of temperatures from low to high can be implemented.

[Examples and Reference Examples of the Fourth Embodiment]

In the following, the present invention is described in detail using examples of the fourth embodiment.

(First Reference Example)

A lubricant of the first reference example is prepared as follows. This is a lubricant that includes a single ester base oil having the structure represented by (chemical formula 6), wherein the alkyl base $R = C_nH_{2n+1}$ is a straight chain saturated alkyl base of $C_8H_{17}$ in the case that wherein n = 8. This base oil is made to contain a mixed antioxidant made up of a hindered phenol-based antioxidant and hindered amine-based antioxidant as an antioxidant. The content of each antioxidant is 1 wt.%. The hindered phenol-based antioxidant has four (3, 5-di-tert-butyl-4-hydroxyphenyl).

(second Example)

A lubricant of the second example is prepared as follows. The above described composition of the first reference example is made to additionally contain 1 wt.% of the triglyceride represented by (chemical formula 10). Here, R1, R2 and R3 have the same structure and, respectively, have the structure of CxHyOz wherein the value of x is 17, the value of y is 33 and the value of z is 1.

(First Comparative Example)

Two types of base oils are used in the first comparative example wherein the carbon number n in the alkyl base $R = C_nH_{2n+1}$ differ as follows in the structure represented by (chemical formula 6). In either case, 1 wt.% of a hindered phenol-based antioxidant having four (3, 5-di-tert-butyl-4-hydroxyphenyl) is included.

First Comparative Example A: $R = C_7H_{15}$
First Comparative Example B: $R = C_8H_{17}$
First Comparative Example C: $R = C_9H_{19}$

(Second Comparative Example)

The three types shown below are prepared as the second comparative example in order to examine the effects

due to the hindered phenol-based antioxidant content. The base oil has the structure represented by (chemical formula 6) wherein R is a straight chain saturated alkyl base formed of $C_8H_{17}$. In addition, a hindered phenol-based antioxidant having four (3, 5-di-tert-butyl-4-hydroxyphenyl) is used.

Second Comparative Example D: hindered phenol-based antioxidant content: 0 wt.%
Second Comparative Example E: hindered phenol-based antioxidant content: 2 wt.%

Here, the first comparative example B of the first comparative example can also be utilized in the case wherein the hindered phenol-based antioxidant content is 1 wt.%.

(Third Comparative Example)

A lubricant, shown in the third comparative example F, is prepared in order to examine the effect due to the triglyceride content.

Third Comparative Example F: the base oil has the structure represented by (chemical formula 6) wherein R is straight chain saturated alkyl base formed of $C_8H_{17}$. Furthermore, the base oil is made to contain 1 wt.% of the hindered phenol-based antioxidant and 1 wt.% of the triglyceride.

(Fourth Comparative Example)

Lubricants that have conventionally been used with dynamic pressure hydraulic bearings are used as the fourth comparative example.

Fourth Comparative Example G: DOS (sebacic acid di-2-ethylhexyl)

Fourth Comparative Example H: a lubricant containing a mixed ester made of two types of ester, ester of neopentylglycol and capryl acid and ester of neopentylglycol and capric acid.

The viscosities at 40°C and at 0°C, the amount of vaporization, the stability at high temperatures in the condition of contact with a metal of a bearing part and the total oxidation number are, respectively, measured with respect to these lubricants. These measurements and evaluations are carried out in the same manner as in the case of the first embodiment, of which the detailed descriptions are omitted.

The results of the measurements of the viscosities at 40°C and at 0°C and the amount of vaporization with respect to these lubricants are shown in Table 7. In addition, the results concerning the viscosity fluctuation'in the accelerated high temperature test are shown in Fig. 11. In addition, the results of the measurements of the total oxidation numbers are shown in Table 8.

[Table 7]

| | Total acid number (mgKOH/g) |
|---|---|
| First Reference Example | 0.37 |
| Second Example | 0.15 |
| First Comparative Example A | 0.00 |
| First Comparative Example B | 0.91 |
| First Comparative Example C | 0.00 |
| Second Comparative Example D | Measurement impossible |
| Second Comparative Example E | 0.50 |
| Third Comparative Example F | 0.37 |
| Fourth Comparative Example G | 18.16 |
| Fourth Comparative Example H | 1.00 |

[Table 8]

| | viscosity (0°C) (mPa·s) | Viscosity (40°C) (mPa · s) | amount of vaporization (wt. %) |
|---|---|---|---|
| First Reference Example | 38.74 | 8.18 | 0.30 |
| Second Example | 39.16 | 8.21 | 0.26 |
| First Comparative Example A | 27.30 | 6.44 | 0.58 |
| First Comparative Example B | 36.13 | 7.72 | 0.29 |
| First Comparative Example C | 46.71 | 9.50 | 0.09 |
| Second Comparative Example D | 33.31 | 7.41 | 0.39 |
| Second Comparative Example E | 39.54 | 8.12 | 0.30 |
| Third Comparative Example F | 36.52 | 7.75 | 0.25 |
| Fourth Comparative Example G | 52.72 | 10.42 | 0.61 |
| Fourth Comparative Example H | 37.39 | 8.00 | 0.50 |

As for the results of the accelerated high temperature test shown in Fig. 11, the lateral axis indicates a period of time wherein the high temperature condition is maintained and the longitudinal axis indicates the viscosity after a constant period of time has elapsed.

First, the influence of the carbon number n of the alkyl base R = $C_nH_{2n+1}$ in a single ester base oil formed of (chemical formula 6) on the lubricant characteristics is described.

The first comparative example A, wherein a base oil of which the alkyl base R is formed of $C_7H_{15}$ is used, has a low viscosity and its amount of vaporization is great. Here, as is seen from Fig. 11 and Table 8, fluctuation in viscosity and the total oxidation number are low and stable, even in the accelerated high temperature test. Accordingly, low torque can be implemented and lubricant deterioration in an axis member or in a sleeve member does not occur. However, there is a possibility that the lubricant will be vaporized in a comparatively short period of time because the amount of vaporization is great so that the function of the dynamic pressure hydraulic bearing may deteriorate. Since it is comparatively difficult to control the amount of vaporization by means of additives, utilization is limited to fields wherein low rotation speeds are used in environments not of high temperature.

On the other hand, in the first comparative example C wherein a base oil, of which the R is formed of $C_9H_{19}$ is used, the amount of vaporization becomes very small while the viscosity has a high value in comparison with the fourth comparative example H, which is a conventional lubricant. In the accelerated high temperature test, however, the fluctuation in viscosity and the total oxidation number both show excellent characteristics. Since the reduction of power consumption is strongly required in portable apparatuses, a lubricant of a low viscosity is essential in order to mount a hard disk device in a portable apparatus. In order to reduce the viscosity using this base oil, an ester-based material of a lower viscosity needs to be added. In this case, however, the amount of vaporization increases.

The first comparative example B, wherein a base oil, of which the R is formed of $C_8H_{17}$ is used, has a viscosity and an amount of vaporization superior to the fourth comparative example G and to the fourth comparative example H, which are both conventional lubricants. Furthermore, though fluctuation in viscosity in the accelerated high temperature test is barely detectable, the total oxidation number is 0.91 and is slightly superior to the fourth comparative example H (1.00), which is a conventional lubricant.

According to the above results, it was found that the base oil having the structure formed of (chemical formula 6), wherein the alkyl base R is $C_8H_{17}$, is the most well-balanced from the viewpoint of the overall characteristics. In the case that the total oxidation number is further reduced, better characteristics can be gained.

In addition, the hindered phenol-based antioxidant content is described in reference to the second comparative example D, the second comparative example E and the first comparative example B.

As the hindered phenol-based antioxidant content increases, the viscosity increases. Though the amount of vaporization is somewhat great when the content is 0 wt.%, there is almost no difference when the content is 1 wt.% or more. On the other hand, it was found in the accelerated high temperature test that a great fluctuation in viscosity occurred in an extremely short period of time when the content was 0 wt.% and the heat resistance stability increased when the content was increased and, thereby, the total oxidation number was reduced. That is to say, the heat resistance stability of the hindered phenol-based antioxidant increases together with the increase in the hindered phenol-based antioxidant content and, at the same time, the viscosity thereof also increases to a comparatively great degree.

In contrast to this, in the case of the lubricant of the first example, it was found that though the total of the hindered phenol-based antioxidant content and the hindered amine-based antioxidant content was 2 wt.%, which is the same as of the second comparative example E, the total oxidation number was further reduced so that the heat resistance stability was increased. In addition, the viscosity of the first example at 0°C is lower and, contrarily, the viscosity at 40 °C is slightly higher than of the lubricant of the second comparative example E and, therefore, change in viscosity according to fluctuation in temperature is rather small. Accordingly, low power consumption can be achieved at low temperatures and fluctuation in power consumption can be suppressed over a wide range of temperatures.

Here, though not shown in the examples, it was found in another test that heat resistance stability, when the hindered phenol-based antioxidant content and the hindered amine-based antioxidant content are approximately equal, improved to a great degree with the content up to 0.1 wt.%, or more, and that the heat resistance stability improved in a gradual manner when the content was increased to 0.1 wt.% or more.

Next, the effect of the inclusion of a triglyceride is described.

The inclusion of triglyceride slightly increases the viscosity and slightly reduces the amount of vaporization while greatly improving the total oxidation number judging from the comparison of the third comparative example F with the first comparative example B. In the same manner, as a result of the inclusion of a triglyceride, the viscosity increased slightly while the amount of vaporization and the total oxidation number both improved judging from a comparison of the first example with the second example.

Furthermore, the effects of the inclusion of triglyceride were derived according to the friction coefficient in a pinion disk test wherein a stainless steel pin and a disk made of a copper alloy, on which is formed nickel phosphorus plating coating, is used. As a result, it was found that the friction coefficient can be reduced and heat resistance stability can also be improved by including triglyceride.

It was found from the above results that the friction coefficient can be reduced by adding triglyceride. Accordingly, seizure, or the like, can be prevented from occurring even when a drive mounted in a portable apparatus is repeatedly turned on and off. Furthermore, the amount of vaporization and the total oxidation number are both improved by adding triglyceride so that the lubricant stability can be improved. The addition of triglyceride up to 3 wt.% is allowable as is seen from the improvement in the amount of vaporization and in the total oxidation number, with a small increase in the viscosity, by adding 1 wt.% of triglyceride. The lower limit of the triglyceride content is 0.1 wt.% judging not only from the experimental data described herein but, also, from the additional experiments. In particular, in the case that the lubricant is utilized in a portable apparatus under the condition of frequent intermittent operation, it is desirable for the triglyceride content to be 1 wt.% or more.

Here, depending on the type of metal utilized for the axis member and for the sleeve member, a case may result wherein the surface of the metal is corroded due to reaction with the lubricant. In such a case, a metal corrosion inhibitor or a metal deactivator which serves to protect the metal surface may be added in advance to a lubricant of the present example.

In addition, the components of the lubricant of the above described example can be changed depending on the above described spindle motor, dynamic pressure hydraulic bearing and hard disk device. That is to say, a variety of additives, such as an oiliness improver, a metal corrosion inhibitor, a metal deactivator, or the like, may be utilized in accordance with the structure of the spindle motor or the environment of utilization.

As described above, it was found that the best overall characteristics can be gained in a lubricant having the composition wherein a mixed antioxidant, of a hindered phenol-based antioxidant and a hindered amine-based antioxidant, as well as a triglyceride are added to a single ester base oil having the structure formed of (chemical formula 6) wherein the alkyl base R is $C_8H_{17}$.

(Fifth Embodiment)

Figs. 12A and 12B show an information recording and reproduction device (hard disk device) according to the fifth embodiment of the present invention. Fig. 12A is a schematic plan view of the major portion of this device and Fig. 12B is a schematic cross sectional view along line A-O-A in Fig. 12A. The spindle motor in this information recording and reproduction device is an inner rotor-type .

A base part 33 and a cover 49 are secured to a housing 48 of an information recording and reproduction device 100 so as to form a sealed space inside thereof. A cylindrical bearing part 39 with a bottom is engaged and secured to

the base part 33. A rotational axis part 42, which is integrated with a disk part 41, is inserted into the bearing part 39 and is supported so as to freely rotate. As shown in Fig. 13, the end surface of the rotational axis part 42 in the axis direction and the inner surface of a thrust support part 40, which becomes the bottom of the bearing part 39, face each other and lubricant 44 according to the present invention is filled in into the gap between the two surfaces and, thereby, a thrust bearing part 50 is formed. A dynamic pressure generation trench (not shown) is created in, at least, one of the surfaces including the end surface of the rotational axis part 42 in the axis direction and the inner surface of the thrust support part 40 which face each other. In addition, the outer peripheral surface of the rotational axis part 42 and the inner peripheral surface of the bearing part 39 face each other and the lubricant 44 is filled in into the gap between the two surfaces and, thereby, a radial bearing part 51 is formed. A dynamic pressure generation trench (not shown) is created in, at least, one of the surfaces including the example peripheral surface of the rotational axis part 42 and the inner peripheral surface of the bearing part 39, which face each other. Furthermore, the lubricant 44 is filled in into the gap between the end surface on the side having an opening of the bearing part 39 and an annular region 41a opposed to the above end surface on the side having the opening in the rear surface of the disk part 41, which face each other. A dynamic pressure generation trench (not shown) is created in, at least, one of the surfaces including the end surface on the side having the opening of the bearing part 39 and the annular region 41a of the disk part 41, which face each other.

A dynamic pressure hydraulic bearing is formed of the thrust bearing part 50, the radial bearing part 51 and the thrust bearing part 52. The thrust bearing part 52 may be omitted.

A stator 38 formed of an iron core 36 and a coil 37 is attached the base part 33 by being compressively inserted or by means of adhesive, or the like. On the other hand, a rotor yoke 34 is attached to the disk part 41 and a rotational magnet 35 is attached to the rotor yoke 34. The rotor yoke 34 is an annular form and the rotational magnet 35 is magnetized to have a plurality of poles. The rotational magnet 35, the rotor yoke 34 and a portion of the disk part 41 and the rotational axis part 42 form a rotor 32. The spindle motor formed in such a manner has the rotor 32 inside of the stator 38 and, thereby, becomes of the inner rotor-type.

A recording medium layer, made of a magnetic material, is grown on the smooth main surface of the disk part 41. A magnetic head for carrying out read-out and write-in of an information signal is arranged so as to be opposed to the disk part 41 and a head arm 46 to which this magnetic head 45 is attached is formed so as to be driven by an actuator 47. A thrust suction plate 43 is secured to the base part 33 so as to be opposed to the rotational magnet 35.

Next, a dynamic pressure generation trench is described. Figs. 14A and 14B are bottom views of the lower side of the disk part 41 as viewed in separation.

In the case of Fig. 14A, a dynamic pressure generation trench 50a in a herringbone form is created on the end surface of the rotational axis part 42 in the axis direction that forms the thrust bearing part 50. In the case of Fig. 14B, a dynamic pressure generation trench 52a in a herringbone form is created in the annular region 41a of the disk part 41 that forms the thrust bearing part 52. One of the dynamic pressure generation trenches of Fig. 14A or 14B is necessary for generating a dynamic pressure in the thrust direction and it is more preferable for both of them to be provided.

In this spindle motor, when the rotor 32 is rotated by activating the coil 37, a dynamic pressure is generated through the effects of the lubricant 44 in the dynamic pressure generation trench portions in a radial bearing part 51 and in thrust bearing parts 50 and 52 so that the rotor 32 rotates smoothly around the center axis 30. During rotation, the dynamic pressure of the lubricant 44 that occurs due to rotation is balanced with the weight of the rotor 32 itself and the magnetic suction power between the rotational magnet 35 and the thrust suction plate 43 and, thereby, the rotor 32 smoothly rotates regardless of the position of the information recording and reproduction device 100.

In the above description, a metal material such as stainless steel, an aluminum alloy or a copper alloy, glass, a liquid crystal polymer or a thermoplastic material, such as a PPS (polyphenylene sulfide) is utilized as the material for forming the bearing part 39 and the rotational axis part 42. In the case that an aluminum alloy or a copper alloy is utilized, it is preferable to harden the surface by carrying out nickel phosphorus plating, for example, in order to increase the resistance to abrasion.

Since the rotational magnet 35 and the thrust suction plate 43 mutually attract magnetically, the rotational axis part 42 is prevented from being withdrawn. In addition, the lubricant 44 has an intrinsic viscosity and has a surface tension and, therefore, can be prevented from flowing out.

(Sixth Embodiment)

An example of an information recording and reproduction device provided with an outer rotor-type spindle motor is shown in Fig. 15. A cylindrical bearing part 65 with a bottom is engaged and secured to a base part 71. A stator 63 formed of an iron core 61 and a coil 62 is engaged and secured to the external peripheral surface of the bearing part 65. A rotational axis part 66 that is integrated with a disk part 67, on the surface of which a recording medium layer made of a magnetic material has been grown, is mounted to the bearing part 65 so as to be freely rotatable. An annular rotor yoke 68 is secured to the rear surface of the disk part 67 in the vicinity of the external periphery of the disk part 67 and a rotational magnet 69, which is magnetized to have a plurality of poles, is attached to the inner peripheral surface of

the rotor yoke 68. The rotational magnetic 69, the rotor yoke 68, the disk part 67 and the rotational axis part 66 form a rotor 70. The rotational magnetic 69 and the rotor yoke 68, which are the main parts of the rotor 70, are arranged outside of the stator 63 so as to provide an outer rotor-type spindle motor.

The same type of trust bearing parts as those shown in Fig. 13 are denoted as 50 and 52 while the same type of radial bearing part as that shown in Fig. 13 is denoted as 51. A lubricant 44 is filled in, in the same manner as in Fig. 13, into gaps within the thrust bearing parts 50 and 52 and the radial bearing part 51. In addition, a dynamic pressure generation trench is created in the same manner as in the above in, at least, one of the two surfaces that face each other in each of the thrust bearing parts 50 and 52 and in the radial bearing part 51.

Here, a thrust suction plate is denoted as 72 and a center axis is denoted as 73. The iron core 61 of the stator 63 may be attached to the base part 71.

(Seventh Embodiment)

The spindle motors of Figs. 12 and 15 are radial gap-type brushless motors. However, the present invention may be applied to an axial gap-type brushless motor in addition to the above. Fig. 16 shows a cross sectional structure of an axial gap-type brushless motor.

A cylindrical bearing part 82 with a bottom is engaged and secured to a base part 81. A rotational axis part 84, which is integrated with a disk part 83 on the surface of which a recording medium layer made of a magnetic material has been grown, is mounted to the bearing part 82 so as to be freely rotatable. A rotor yoke 85 is secured to the rear surface of the disk part 83 through adhesive, or the like, and an annular rotational magnet 86, which is magnetized to have a plurality of poles, is secured to the lower surface of the rotor yoke 85 through adhesive, or the like. A stator 89 formed of a printed circuit board 87 and a plurality of coils 88 is attached to the base part 81. The coils 88 are approximately in a triangular form. The rotational magnetic 86 and the coils 88 face each other separated by a small gap in the axis direction. The rotational magnet 86, the rotor yoke 85, the disk part 83 and the rotational axis part 84 form a rotor 90.

The same type of thrust bearing parts as those shown in Fig. 13 are denoted as 50 and 52 and the same type of radial bearing part as that shown in Fig. 13 is denoted as 51. A lubricant 44 is filled in, in the same manner as in Fig. 13, into gaps between the two surfaces in the thrust bearing parts 50 and 52 and in the radial bearing part 51. In addition, a dynamic pressure generation trench is created, in the same manner as above, in, at least, one of the two surfaces facing each other in each of the thrust bearing parts 50 and 52 and in the radial bearing part 51.

(Eighth Embodiment)

Fig. 17 shows the structure of a motor according to the eighth embodiment of the present invention. A bearing part 92 in a shaft form is secured to a base part 91. On the other hand, a cylindrical rotational axis part 94 is secured to a disk part 93 on the surface of which an information recording layer is formed so that the rotational axis part 94 is engaged with the bearing part 92 so as to be freely rotatable. A rotor yoke 95 is secured to the rear surface of the disk part 93 in the vicinity of the outside of the rotational axis part 94 and an annular rotational magnet 96 that is magnetized to have a plurality of poles is secured to the rotor yoke 95 by means of adhesive, or the like. On the other hand, a stator 99 formed of an iron core 97 and a coil 98 is secured to the base part 91 by being compressively inserted, or by another means. A lubricant 100 according to the present invention is filled in into a gap between the surfaces of the bearing part 92 in a shaft form and of the cylindrical rotational axis part 94, which face each other in the radial direction and in the thrust direction. The rotational magnet 96, the rotor yoke 95, a portion of the disk part 93 and the cylindrical rotational axis part 94 form a rotor 101. A thrust suction plate 102 is attached to the base part 91 so as to be opposed to the end surface of the rotational magnet 96.

The present embodiment differs from the other embodiments in a point that the cylindrical rotational axis part 94 is engaged from the outside to the bearing part 92 in a shaft form. A dynamic pressure generation trench (not shown) is created in, at least, one of the surfaces including the upper end surface of the bearing part 92 in a shaft form and the lower surface of the disk part 93 that is opposed to the upper end surface. In addition, the outer peripheral surface of the bearing part 92 in a shaft form and the inner peripheral surface of the cylindrical rotational axis part 94 face each other and a dynamic pressure generation trench (not shown) is created in, at least, one of these surfaces, the outer peripheral surface and the inner peripheral surface. Here, a dynamic pressure generation trench may be created in, at least, one of the surfaces including the end surface of the cylindrical rotational axis part 94 in the axis direction and the upper surface of the base part 91 that is opposed to this end surface. The bearing part 92 is not necessarily solid but, rather, may be hollow.

A metal material such as stainless steel, an aluminum alloy or a copper alloy, glass, a liquid crystal polymer or a thermoplastic material such as PPS (polyphenylene sulfide), for example, is utilized in the disk part 93, the cylindrical rotational axis part 94 and the bearing part 92 in a shaft form. In the case that an aluminum alloy or a copper alloy is utilized, it is preferable to harden the surface by applying nickel phosphorus plating, for example, in order to increase

the resistance to abrasion.

Fig. 18A is a bottom view from below of the.rotor 101 separated from the base part 91. A dynamic pressure generation trench 103 is created in the rear surface of the disk part 93 inside of the cylindrical rotational axis part 94.

Fig. 18B is a plan view from above of the base part 91 separated from the rotor 101. A dynamic pressure generation trench 104 is created in an annular region of the base part 91 opposed to the end surface of the cylindrical rotational axis part 94 in the axis direction.

In summary, the rotational axis part 94 is cylindrical and the bearing part 92 is in a shaft form in the spindle motor of Fig. 17, which, furthermore, is formed of an inner rotor-type and this spindle motor may be modified so as to be formed of an outer rotor-type. In addition, this spindle motor may be formed of an axial gap-type instead of a radial gap-type. In addition, a variety of additives, such as an oil agent, a metal corrosion inhibitor or a metal deactivator, for example, may, of course, be utilized in accordance with the structure or the utilization environment of the spindle motor. In any case, a very thin motor with a high rotational precision can be implemented. For example, an information recording and reproduction device, such as a disk device having a thickness of, for example, approximately no greater than 5 mm can be implemented.

Moreover, the present invention is applicable to an information recording and reproduction device to which an optical disk or an optical magnetic disk is mounted in addition to a hard disk device. Utilization in a dynamic pressure hydraulic bearing used in a motor for rotating a polygonal mirror or in other rotational parts is also possible.

[Examples and Reference Examples of the Fifth to Eighth Embodiments]

In the following, the examples and reference examples are described wherein the lubricants used in motors according to the fifth to eighth embodiments are examined.

(First Reference Example)

A lubricant of the first reference example is prepared as follows. This is a lubricant that includes a single ester base oil having the structure represented by (chemical formula 6), wherein the alkyl base R = $C_nH_{2n+1}$ is a straight chain saturated alkyl base of $C_7H_{15}$ in the case wherein n = 7. The above described base oil is made to include a mixed antioxidant formed of a hindered phenol-based antioxidant and a hindered amine-based antioxidant as an antioxidant. The content of each antioxidant is 1 wt.%. The hindered phenol-based antioxidant has four (3, 5-di-tert-butyl-4-hydroxyphenyl).

(Second Reference Example)

A lubricant of the second example is prepared as follows. The lubricant includes a single ester base oil wherein the alkyl base R = $C_nH_{2n+1}$ is a straight chain saturated alkyl base of $C_8H_{17}$ in the case wherein n = 8 in place of $C_7H_{15}$ in the case wherein n = 7. The other parts are the same as in the first reference example.

(Third Reference Example)

A lubricant of the third reference example is prepared as follows. The lubricant includes a single ester base oil wherein .the alkyl base R = $C_nH_{2n+1}$ is a straight chain saturated alkyl base of $C_9H_{19}$ in the case wherein n = 9 in place of $C_7H_{15}$ in the case wherein n = 7. The other parts are the same as in the first reference example.

(Fourth Example)

A lubricant of the fourth example is prepared as follows. The composition of the above described second reference example (n = 8) is made to additionally include 1 wt.% of triglyceride represented by (chemical formula 10). Here, R1, R2 and R3 have the same structure and, respectively, have the structure of CxHyOz wherein the value of x is 17, the value of y is 33 and the value of z is 1.

(First Comparative Example)

DOS (sebacic acid di-2-ethylhexyl), which is a di-ester-based lubricant conventionally used with dynamic pressure hydraulic bearings, is used as the first comparative example.

(Second Comparative Example)

A lubricant of which the base oil is a mixed ester wherein two types of esters, an ester of neopentylglycol and capryl acid and an ester of neopentylglycol and capric acid are mixed is used as the second comparative example.

Here, in the lubricants of from first to fourth examples and reference examples, the amount of the main component of the base oil is shown in Table 9. The main components of the base oils in the lubricants of the examples and reference examples are, respectively, 85 wt.% or more.

[Table 9]

| lubricant component | First Reference Example | Second Reference Example | Third Reference Example | Fourth Example |
|---|---|---|---|---|
| amount of main component of base oil (wt.%) | 98.5 | 85.3 | 97.7 | 96.7 |

The results of the measurements of the viscosity, the stability of the viscosity, the amount of vaporization, antioxidant properties, the stability at high temperatures and the friction coefficient of the above described lubricants of the four examples and reference examples and of the two comparative examples are shown in Table 10 and in Fig. 19 and are described in the following.

[Table 10]

| | First Reference Example | Second Reference Example | Third Reference Example | Fourth Example | First Comparative Example | Second Comparative Example |
|---|---|---|---|---|---|---|
| Viscosity at 0°C (mPa· s) | 27.3 | 36.13 | 46.71 | 36.52 | 52.72 | 37.39 |
| Viscosity at 40°C (mPa· s) | 6.44 | 7.72 | 9.50 | 7.75 | 10.42 | 8.00 |
| ratio of change according to temperature: B value | 2925 | 2757 | 2960 | 2914 | 3256 | 2975 |
| amount of vaporiz ation (wt.%) | 0.58 | 0.29 | 0.09 | 0.25 | 0.61 | 0.50 |
| total oxidation number (mgKOH/ g) | 0.00 | 0.91 | 0.00 | 0.37 | 18.16 | 1.00 |
| Friction coefficient | 0.15 | 0.14 | 0.14 | 0.12 | 0.16 | 0.15 |

The results of the measurement of the change in viscosity in an accelerated high temperature test carried out in the same manner as in the above described examples and reference examples of the first embodiment are shown in Fig. 19. The lubricant of the first reference example is denoted as P11, the lubricant of the second reference example is denoted as P12, the lubricant of the third reference example is denoted as P13, the lubricant of the fourth example is denoted as P14, the lubricant of the first comparative example is denoted as Q11 and the lubricant of the second comparative example is denoted as Q12. The explanations in from paragraph (§1) to paragraph (§2) in the descriptions of the above first embodiment correspond to the descriptions of the examples in this sixth embodiment.

Here, the lubricants from first to fourth examples and reference examples, which have not changed in viscosity, used with a bearing of a copper alloy material on which nickel phosphorus plating has been carried out also undergo

an accelerated high temperature test by being used with a bearing of copper alloy material on which nickel phosphorus plating has not been carried out. The results thereof are shown in. Fig. 20. The test conditions are the same as in the above described accelerated high temperature test.

Furthermore, the lubricants shown in the following reference examples are prepared in order to confirm the influence, according to amount, of antioxidants added to the lubricants.

(Fifth Reference Example)

The lubricant of the fifth reference example is prepared as follows. This is a lubricant that includes a single ester base oil having the structure represented by (chemical formula 6) wherein the alkyl base R = $C_nH_{2n+1}$ is a straight chain saturated alkyl base of $C_7H_{15}$ in the case wherein n = 7. An antioxidant has not been added to the fifth reference example.

(Sixth Reference Example)

The lubricant of the sixth reference example is prepared as follows. This is a lubricant that includes a single ester base oil having the structure represented by (chemical formula 6) wherein the alkyl base R = $C_nH_{2n+1}$ is a straight chain saturated alkyl base of $C_7H_{15}$ in the case wherein n = 7. A mixed antioxidant that includes 2 wt.% of a hindered phenol-based antioxidant having four (3, 5-di-tert-butyl-4-hydroxyphenyl) and 1 wt.% of a hindered amine-based antioxidant is added to the above described base oil as an antioxidant. This has an additional 1 wt.% of the hindered phenol-based antioxidant in comparison with the first reference example.

The viscosity, the stability of viscosity, the amount of vaporization and the antioxidant properties of the lubricants of the above described fifth reference example and the sixth reference example are measured so as to gain the results shown in Table 11.

[Table 11]

| | Fifth Reference Example | Sixth Reference Example |
|---|---|---|
| viscosity at 0°C (mPa · s) | 33.31 | 39.54 |
| viscosity at 40°C (mPa · s) | 7.41 | 8.12 |
| ratio of change according to temperature: B value | | |
| amount of vaporization (wt.%) | 0.39 | 0.30 |
| total oxidation number (mgKOH/g) | Measurement impossible | 0.50 |

As the amount of added hindered phenol-based antioxidant increases, the viscosity increases. Though the amount of vaporization is somewhat great when the content is 0 wt.%, there is almost no difference when the content is 1 wt.% or more. On the other hand, it was found in the accelerated high temperature test that a great fluctuation in viscosity occurred in an extremely short period of time when the added amount was 0 wt.% and the heat resistance stability increased when the added amount was increased and, thereby, the total oxidation number was reduced. That is to say, the heat resistance stability of the hindered phenol-based antioxidant increases together with the increase in the added amount of the hindered phenol-based antioxidant and, at the same time, the viscosity thereof also increases to a comparatively great degree.

In the case of the lubricant of the fifth reference example to which the hindered phenol-based antioxidant has not been added, though the total oxidation number could not be measured, the heat resistance stability increased and the viscosity at 0°C is low while, in contrast, the viscosity at 40°C is somewhat high in comparison with the lubricant of the second reference example to which 2 wt.% of hindered phenol-based antioxidant was added and, therefore, a change in viscosity due to the fluctuation in temperature is rather small. Accordingly, a reduction in the amount of power consumed can be implemented even at low temperatures and the fluctuation in the amount of power consumed can be restricted over a broad range of temperatures.

Though not concretely illustrated here, it was found from another experiment with respect to the heat resistance stability that the heat resistance stability is improved to a great extent by adding 0.1 wt.% or more of the hindered phenol-based antioxidant and the heat resistance stability was gradually improved in accordance with the amount added.

Next, the effect of the addition of triglyceride is described. When the lubricant of the second reference example is compared with the lubricant of the fourth example, wherein triglyceride is added to this lubricant of the second reference example, results are gained wherein the viscosity slightly increases and the amount of vaporization is slightly reduced

in the lubricant of the fourth example while the value of the total oxidation number of the lubricant of the fourth example is approximately 60% smaller than that of the lubricant of the second reference example, which indicates that the addition of triglyceride has improved the properties of the lubricant. It has become clear from the above that triglyceride has the effects of suppressing the decomposition of the base oil.'

A rotation start-up/stoppage cycle test of motors provided with dynamic pressure hydraulic bearings is carried out. This indicates the appearance of a change in a motor current with respect to the number of start-up/stoppage cycles. Fig. 21 shows the results of the case wherein the lubricant of the second example, which does not include triglyceride, is used and of the case wherein the lubricant of the fourth example, to which 1 wt.% of triglyceride is added. It was found that, as shown in this Fig. 21, the motor current clearly tended to increase, after passing approximately 600,000 cycles, when the lubricant of the second reference example, which does not include triglyceride; was used and the viscosity of the lubricant increased so that the characteristics of the lubricant deteriorated.

In addition, the results of the evaluation of the sliding characteristics when metal contact has occurred in the hydraulic lubricant part are described in order to confirm the results when triglyceride is added to the lubricant. The metal contact occurs when the lubricant film is broken immediately after start-up or immediately before the stoppage of the motor to which the dynamic pressure hydraulic bearing is mounted and the friction coefficient becomes great so that, therefore, a large amount of friction occurs. An evaluation is carried out by measuring the friction coefficient using a pinion disk test device. In this test, a pin made of stainless steel, which is, in general, used in the rotational shaft, and a disk made of a copper alloy on nickel phosphorus plating film has been formed, which is used in the bearing part, are utilized. As for test conditions, the' relative speed between the pin and the disk is set at 0.16 m/sec and the load added to the pin is set at 624 mN. Test results are shown in Table 10.

As a result, a small friction coefficient was gained in the case of the lubricant of the fourth example, to which triglyceride was added, in comparison with the lubricants of the first to third reference examples and the first and second comparative examples, to which triglyceride was not added. Since the friction coefficient can be reduced, seizure, or the like, can be prevented from occurring due to the repeated turning on and off of the drive when the lubricant is used in a portable apparatus. Furthermore, the addition of triglyceride improves the amount of vaporization and the total oxidation number and, thereby, stability of the lubricant can be increased. Even in the case that 1 wt.% of triglyceride is added, the increase in viscosity thereby caused is small and the amount of vaporization and the total oxidation number are improved. As is seen from the above, the addition of up to 5 wt.% of triglyceride is considered to be allowable.

In the case that an aluminum alloy or a copper alloy is utilized as a material of the part that forms the bearing part of a motor provided with a dynamic pressure hydraulic bearing, resistance to abrasion is increased by carrying out nickel phosphorus plating, for example, on the surface so that the surface is hardened.

Next, the relationship between nickel phosphorus plating and the lubricant including the above described single ester base oil is described.

In the case that an aluminum alloy or a copper alloy is utilized as a material for forming a rotor made of a disk part and a rotational axis part of a motor and a bearing part, nickel phosphorus plating is carried out by means of electroless plating. Concretely, an alloy plating bath is used and an aging process is carried out at from 200°C to 350°C so as to gain a nickel phosphorus film having a thickness of from approximately 10 $\mu$m to 20 $\mu$m and a hardness of from 5 GPa to 15 GPa. In the experiment wherein brass, which is a type of copper alloy, was used, it became clear that a film of a great hardness was formed when the phosphorus composition of the nickel phosphorus film was from 1 wt.% to 15 wt.%. When the relationship between the phosphorus component and hardness was examined, the hardness of the film started increasing in tandem with the increase in the amount of the phosphorus composition and a peak of hardness was found at 4 wt.%. The condition wherein the hardness is the greatest continues to the vicinity of 5 wt.% and a great hardness was indicated after exceeding 5 wt.% up to the vicinity of 15 wt.%. Accordingly, it is preferable for the phosphorus composition to be 5 wt.% or less and the vicinity of 4 wt.%, wherein the value of the hardness indicates a peak, is considered to be most desirable.

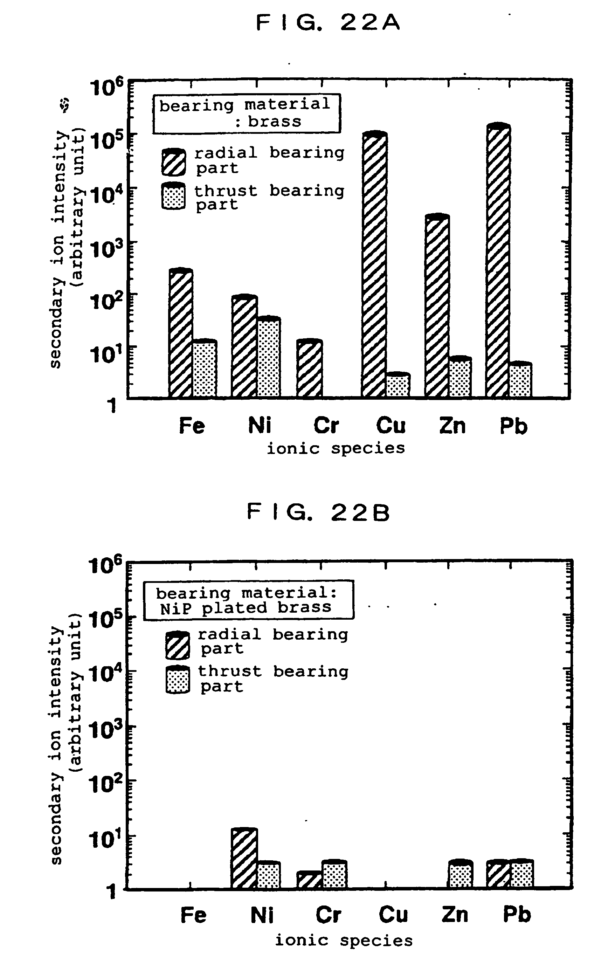

A bearing part is formed of brass, which is one type of copper alloy. In addition, a bearing part, wherein nickel phosphorus film is created by means of electroless plating on the surface of the brass, is formed. The lubricants made of ester-based base oil represented by (chemical formula 6) are utilized with the respective dynamic pressure hydraulic bearing parts and a rotation start-up/stoppage cycle test of motors is carried out. The lubricants are extracted after the completion of 1,000,000 cycles and metal components in the lubricants are measured by means of time of flight secondary ion mass spectrometry (TOF-SIMS method). The results are shown in Fig. 22.

After the test of the bearing part on which nickel phosphorus plating has been carried out, the metal components in the lubricant were found to be of a lowered amount and indicated that the bearing part was not worn. Accordingly, in the case that a lubricant made of a single ester base oil indicated by (chemical formula 6) is utilized in the dynamic pressure hydraulic bearing of a motor having a bearing part made of an aluminum alloy or a copper alloy, the carrying out of nickel phosphorus plating has a great effect from the point of view of resistance to abrasion.

In addition, it is known that when Cu or Pb precipitates out of the lubricant due to wear of the axis part, they become catalysts so that the deterioration of the lubricant is accelerated. A lubricant of a low viscosity, in particular,

easily deteriorates. Then, the carrying out of nickel phosphorus plating eliminates the influence of the above described catalysts and is considered to have a great effect of prevention of deterioration of the lubricant.

Here, though an example is shown above wherein a nickel phosphorus film is formed by carrying out an aging process, sufficient hardness can be gained without carrying out a heat treatment. Hardness is increased when the microscopic crystallization of the formed nickel phosphorus plating film has advanced. Meticulous attention is required because heat treatment may influence the dimensional precision of a motor. In addition, depending on the type of metal used for the rotational axis part and for the bearing part, the surface of the metal becomes corroded through reaction with the lubricant component. In such a case it is preferable to add a metal corrosion inhibitor or a metal deactivator to the lubricant.

Based on the above described results, the results of a comparison of the performances of the lubricants from first to sixth examples with those of the lubricants of the first and second comparative examples are summarized in the following.

That is to say, the lubricant of the first example having a single ester base oil, wherein R represented by (chemical formula 6) is formed of $C_7H_{15}$, has a low viscosity and an excellent heat resistance while having an amount of vaporization slightly greater than that of the lubricant of the second comparative example of which the base oil a mixed ester. As is seen from Fig. 19 and Table 10, however, the fluctuation in viscosity and the total oxidation number is small and stable even in the accelerated high temperature test and the heat generation due to friction of the lubricant itself is small due to its low viscosity. Therefore, heat generation is suppressed in comparison with the second comparative example and, therefore, the lubricant of the first example has greater stability, as a whole, at high temperatures than the lubricant of the second comparative example, though having a slightly greater amount of vaporization. Accordingly, though deterioration of the lubricant does not occur when a low torque is implemented, utilization conditions wherein the environments are not at very high temperatures are preferable.

The lubricants of the second and fourth examples and reference examples having single ester base oils of which the alkyl base R, represented by (chemical formula 6), is formed of $C_8H_{17}$ have characteristics wherein viscosity and amount of vaporization are both superior to those of the lubricants of the first and second comparative examples, which are conventional lubricants. In addition, they are found to be lubricants having great heat resistance and small bearing loss. Furthermore, as shown in Fig. 19, the fluctuation in viscosity is hardly seen in the accelerated high temperature test. The total oxidation number is 0.91 and this is slightly superior to that of the lubricant of the second comparative example, which is a conventional lubricant. The base oil,' of which the alkyl base R is $C_8H_{17}$ is the most well-balanced from the point of view of the overall characteristics from among the structures represented by (chemical formula 6) -and, in the case that the total oxidation number is further reduced, a lubricant having even better characteristics can be gained.

On the other hand, the lubricant o.f the third reference example, having a base oil of which the alkyl base R is formed of $C_9H_{19}$, has a higher viscosity than that of the lubricant of the second comparative example made of mixed ester while having better viscosity than that of the lubricant of the first comparative example and the fluctuation in viscosity and the total oxidation number both indicate good characteristics in the accelerated high temperature test. In addition, the amount of vaporization is very small and heat resistance is considered to be excellent because the value of total oxidation number is 0. As a result of this, even in the case that heat generation occurs because of friction of the lubricant itself, due to its great viscosity, oxidation, decomposition, or the like, does not occur and a dynamic pressure hydraulic bearing of a high reliability can be gained.

The lubricant of the first reference example is favorable for a motor for driving a rotary drum-shaped head of a type of a video recorder in which a camera is integrated or for a spindle motor for a mobile apparatus in the same manner as in the above description. In addition, the lubricant of the second reference example or of the fourth example is favorable for a motor utilized in a portable apparatus that is frequently started up or stopped. The lubricant of the third reference example, having a small vaporization loss and an excellent resistance to oxidation, decomposition and heat, is favorable for a dynamic pressure hydraulic bearing wherein high reliability and long lifetime are required.

As described above, the lubricants of the present examples have a low viscosity, in particular at low temperatures, have a small fluctuation according to temperature so as to have excellent lubricating characteristics and, thereby, have excellent reliability at high temperatures while having a small vaporization loss in comparison with the comparative examples, which are conventional lubricants. Accordingly, these lubricants do not require replenishment for a long period of time, the heat resistance stability can be improved and the characteristics of a dynamic pressure hydraulic bearing can be improved. Therefore, the lubricants having a long lifetime and excellent overall characteristics can be implemented.

Here, though the respective embodiments described above all relates to specific devices, the present invention is not limited to these but, rather, applications to other devices are also possible.

## Claims

1. A lubricant composition comprising a base oil which is a single ester having the following chemical structure :

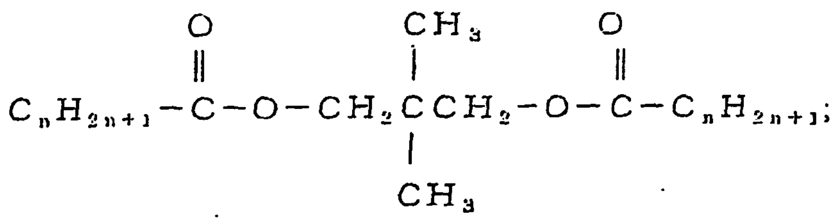

wherein n is any of 7, 8 or 9, and
wherein a hindered phenol-based antioxidant including at least one (3, 5-di-tert-butyl-4-hydroxyphenyl) in the structure thereof is further included, and
wherein a triglyceride represented by the following chemical structure of:

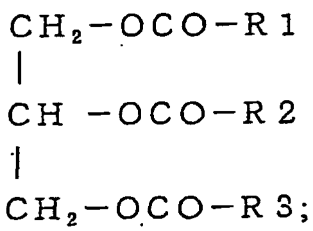

is further included;
wherein R1, R2, and R3 are unsaturated or saturated straight chain structures or branched structures formed of CxHyOz having respective integer values of x in a range from 15 to 21, y in a range from 29 to 43 and z in a range from 0 to 1.

2. The lubricant composition according to claim 1, wherein the secondary ion intensity has peaks at "127," "213" and "357" of the mass numbers of positive secondary ions per unit charge when a base oil included in the lubricant is analyzed by means of secondary ion mass spectrometry.

3. The lubricant composition according to claim 1, wherein the secondary ion intensity has peaks at "141," "227" and "385" of the mass numbers of positive secondary ions per unit charge when a base oil included in the lubricant is analyzed by means of secondary ion mass spectrometry.

4. The lubricant composition according to claim 1, wherein the secondary ion intensity has peaks at "155, " "241" and "413" of the mass numbers of positive secondary ions per unit charge when a base oil included in the lubricant is analyzed by means of secondary ion mass spectrometry.

5. The lubricant composition according to claim 1, wherein the content of said triglyceride is no greater than 5 wt.%, preferably no greater than 3 wt.%.

6. The lubricant composition according to any of claims 1 and 2 to 4. wherein the viscosity characteristics are no greater than 48 mPa • s at 0°C and no greater than 10 mPa • s at 40°C.

7. The lubricant composition according to.any of claims 1 and 2 to 4, which is filled into a gap between the surfaces facing each other of two members that shift in relation to each other.

8. The lubricant composition according to any of claims 1 and 2 to 4, which is filled into a gap between the surfaces facing each other of a rotational axis part and a bearing part in a dynamic pressure hydraulic bearing wherein said rotational axis part is engaged with said bearing part so as to be freely rotatable and wherein a dynamic pressure generation trench is created in at least one of the two surfaces facing each other of said rotational axis part and said bearing part.

**9.** A dynamic pressure hydraulic bearing, comprising: a bearing part and a rotational axis part engaged with each other so as to be freely rotatable; a dynamic pressure generation groove created in at least one of the two surfaces facing each other that form a gap between the bearing part and the rotational axis part; and a lubricant composition filling the gap, wherein a lubricant composition according to any of claims 1-8 is used as said lubricant composition.

**10.** A motor, comprising:

a base part;
a stator for generating a magnetic field secured to the base part;
a rotor having a rotational magnet opposed to the stator;
a rotational axis part provided in the rotor;
a bearing part which is provided in the base part and to which the rotational axis part is engaged so as to be freely rotatable;
a dynamic pressure generation groove created in at least one of the two surfaces facing each other that form a gap between the bearing part and the rotational axis part; and
a lubricant composition filling the gap, wherein a lubricant composition according to any of claims 1-8 is used as the lubricant composition.

**11.** The motor according to claim 10, wherein said bearing part on the base part side is of a cylindrical form and said rotational axis part on the rotor side is engaged with the inside of said bearing part.

**12.** The motor according to claim 10, wherein said rotational axis part on the rotor side is of a cylindrical form and said rotational axis part is engaged with the outside of said bearing part on the base part side.

**13.** The motor according to claim 10, which has a radial bearing part wherein,said bearing part and said rotational axis part face each other in the radial direction and said lubricant composition is filled in the gap between said bearing part and said rotational axis part and wherein a dynamic pressure generation trench is created in at least one of the two faces that form the gap in said radial bearing part.

**14.** The motor according to claim 10, which has a thrust bearing part wherein said bearing part and said rotational axis part face each other in the axis direction and said lubricant composition is filled in the gap between said bearing part and said rotational axis part and wherein a dynamic pressure generation trench is created in at least one of the two faces that form the gap in said thrust bearing part.

**15.** The motor according to claim 11, wherein a thrust bearing part is formed of an end surface on the side having an opening of said cylindrical bearing part and of an annular region in said rotor that is opposed to the end surface on the side having the opening of said bearing part and wherein a dynamic pressure generation trench is created in at least one of said end surface on the side having the opening and said annular region opposed to this end surface in said thrust bearing part.

**16.** The motor according to claim 12, wherein a thrust bearing part is formed of an end surface on the side having an opening of said cylindrical rotational axis part and of an annular region in said base part that is opposed to the end surface on the side having the opening of said rotational axis part and wherein a dynamic pressure generation trench is created in at least one of said end surface on the side having the opening and said annular region opposed to this end surface in said thrust bearing part.

**17.** The motor according to claim 10, wherein nickel phosphorous plating film is formed on the two surfaces facing each other that form the gap between said bearing part and said rotational axis part.

**18.** The motor according to claim 17, wherein said nickel phosphorous plating film is an electroless plating film of which the phosphorous concentration is no greater than 15 wt.%.

**19.** A motor integrated device to which a motor according to claim 10 is mounted.

**Patentansprüche**

**1.** Schmiermittelzusammensetzung, welche ein Basisöl umfasst, das einen Einfachester mit der folgenden chemischen

Struktur darstellt:

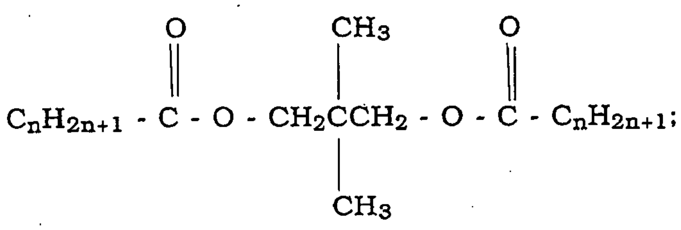

worin n irgendeins aus 7, 8 oder 9 ist, und
worin weiterhin ein gehindertes Antioxidationsmittel auf Phenolbasis, das mindestens ein (3,5-Di-tert-butyl-4-hydroxyphenyl) in der Struktur davon enthält, enthalten ist, und
worin ein durch die folgende chemische Struktur

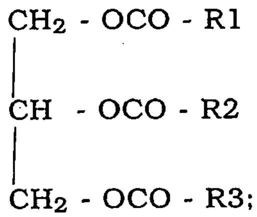

dargestelltes Triglycerid weiterhin enthalten ist;
worin R1, R2 und R3 ungesättigte oder gesättigte geradkettige Strukturen oder verzweigte Strukturen, die aus CxHyOz gebildet sind, mit jeweiligen ganzzahligen Werten von x in dem Bereich von 15 bis 21, von y in dem Bereich von 29 bis 43 und von z in dem Bereich von 0 bis 1 darstellen.

2. Schmiermittelzusammensetzung nach Anspruch 1, worin die sekundäre Ionenintensität Peaks bei "127", "213" und "357" der Massenzahlen von positiven sekundären Ionen pro Einheitsladung aufweist, wenn ein in das Schmiermittel eingefügtes Basisöl mittels sekundärer Ionenmassenspektrometrie analysiert wird.

3. Schmiermittelzusammensetzung nach Anspruch 1, worin die sekundäre Ionenintensität Peaks bei "141", "227" und "385" der Massenzahlen von positiven sekundären Ionen pro Einheitsladung aufweist, wenn ein in das Schmiermittel eingefügtes Basisöl mittels sekundärer lonenmassenspektrometrie analysiert wird.

4. Schmiermittelzusammensetzung nach Anspruch 1, worin die sekundäre Ionenintensität Peaks bei "155", "241" und "413" der Massenzahlen von positiven sekundären Ionen pro Einheitsladung aufweist, wenn ein in das Schmiermittel eingefügtes Basisöl mittels sekundärer Ionenmassenspektrometrie analysiert wird.

5. Schmiermittelzusammensetzung nach Anspruch 1, worin der Gehalt an Triglycerid nicht größer als 5 Gew.-%, vorzugsweise nicht größer als 3 Gew.-% ist.

6. Schmiermittelzusammensetzung nach einem der Ansprüche 1 und 2 bis 4,
worin die Viskositätseigenschaften nicht größer als 48 mPa·s bei 0°C und nicht größer als 10 mPa·s bei 40°C betragen.

7. Schmiermittelzusammensetzung nach einem der Ansprüche 1 und 2 bis 4, welche in einen Spalt zwischen den gegenüberliegenden Oberflächen zweier Elemente gefüllt ist, die sich relativ zueinander verschieben.

8. Schmiermittelzusammensetzung nach einem der Ansprüche 1 und 2 bis 4, welche in einen Spalt zwischen zwei gegenüberliegenden Oberflächen eines Drehachsenteils und eines Lagerteils in einem Staudruck-Hydrauliklager gefüllt ist, wobei der Drehachsenteil mit dem Lagerteil so in Eingriff steht, dass er frei drehbar ist, und wobei eine Staudruck-Erzeugungsrille in zumindest einer der zwei gegenüberliegenden Oberflächen des Drehachsenteils und des Lagerteils gebildet ist.

**9.** Staudruck-Hydrauliklager mit: einem Lagerteil und einem Drehachsenteil, die miteinander so in Eingriff stehen, dass sie frei drehbar sind; einer Staudruck-Erzeugungsrille, die in zumindest einer der gegenüberliegenden Oberflächen gebildet ist, welche einen Spalt zwischen dem Lagerteil und dem Drehachsenteil bilden; und einer Schmiermittelzusammensetzung, die den Spalt füllt, wobei eine Schmiermittelzusammensetzung gemäß einem der Ansprüche 1-8 als Schmiermittelzusammensetzung verwendet wird.

**10.** Motor mit:

- einem Basisteil;
- einem Stator zum Erzeugen eines magnetischen Feldes, der an dem Basisteil befestigt ist;
- einem Rotor mit einem dem Stator gegenüberliegenden Rotationsmagneten;
- einem Drehachsenteil, der in dem Rotor vorgesehen ist;
- einem Lagerteil, der in dem Basisteil vorgesehen ist, und mit dem der Drehachsenteil in Eingriff steht, um frei drehbar zu sein;
- einer Staudruck-Erzeugungsrille, die in zumindest einer der zwei gegenüberliegenden Oberflächen gebildet ist, welche einen Spalt zwischen dem Lagerteil und dem Drehachsenteil bilden; und
- einer Schmiermittelzusammensetzung, die den Spalt füllt, wobei eine Schmiermittelzusammensetzung nach einem der Ansprüche 1-8 als Schmiermittelzusammensetzung verwendet wird.

**11.** Motor nach Anspruch 10, wobei der Lagerteil auf der Basisteilseite von zylindrischer Form ist und der Drehachsenteil auf der Rotorseite in Eingriff mit dem Inneren des Lagerteils steht.

**12.** Motor nach Anspruch 10, wobei der Drehachsenteil auf der Rotorseite von zylindrischer Form ist und der Drehachsenteil mit der Außenseite des Lagerteils auf der Basisteilseite in Eingriff steht.

**13.** Motor nach Anspruch 10, welcher ein Radiallagerteil aufweist, wobei der Lagerteil und der Drehachsenteil sich einander in radialer Richtung gegenüberliegen und die Schmiermittelzusammensetzung in den Spalt zwischen dem Lagerteil und dem Drehachsenteil gefüllt ist, und wobei eine Staudruck-Erzeugungsrille in zumindest einer der zwei Flächen gebildet ist, welche den Spalt in dem Radiallagerteil bilden.

**14.** Motor nach Anspruch 10, welcher ein Axiallagerteil aufweist, wobei sich der Lagerteil und der Drehachsenteil in Axialrichtung gegenüberliegen und die Schmiermittelzusammensetzung in den Spalt zwischen dem Lagerteil und dem Drehachsenteil gefüllt ist, und wobei eine Staudruck-Erzeugungsrille in zumindest einer der zwei Flächen gebildet ist, welche den Spalt in dem Axiallagerteil bilden.

**15.** Motor nach Anspruch 11, wobei ein Axiallagerteil aus einer Endoberfläche auf der Seite mit einer Öffnung des zylindrischen Lagerteils und aus einem Ringbereich in dem Rotor gebildet ist, der der Endoberfläche auf der Seite mit der Öffnung des Lagerteils gegenüberliegt, und wobei eine Staudruck-Erzeugungsrille in der Endoberfläche auf der Seite mit der Öffnung und/oder dem Ringbereich gegenüberliegend zu dieser Endoberfläche in dem Axiallagerteil gebildet ist.

**16.** Motor nach Anspruch 12, wobei ein Axiallagerteil aus einer Endoberfläche auf der Seite mit einer Öffnung des zylindrischen Drehachsenteils und aus einem Ringbereich in dem Basisteil, der der Endoberfläche auf der Seite mit der Öffnung des Drehachsenteils gegenüberliegt, gebildet ist, und wobei eine Staudruck-Erzeugungsrille in der Endoberfläche auf der Seite mit der Öffnung und/oder dem Ringbereich gegenüberliegend zu dieser Endoberfläche in dem Axiallagerteil gebildet ist.

**17.** Motor nach Anspruch 10, wobei ein Nickel-Phosphor-Plattierfilm auf den zwei einander gegenüberliegenden Oberflächen gebildet ist, welche den Spalt zwischen dem Lagerteil und dem Drehachsenteil bilden.

**18.** Motor nach Anspruch 17, wobei der Nickel-Phosphor-Plattierfilm ein stromlos abgeschiedener Film mit einer Phosphorkonzentration von nicht größer als 15 Gew.-% ist.

**19.** Motorintegrierte Vorrichtung, an die ein Motor nach Anspruch 10 montiert ist.

## Revendications

**1.** Une composition lubrifiante comprenant une huile de base qui est un ester unique ayant la structure chimique suivante

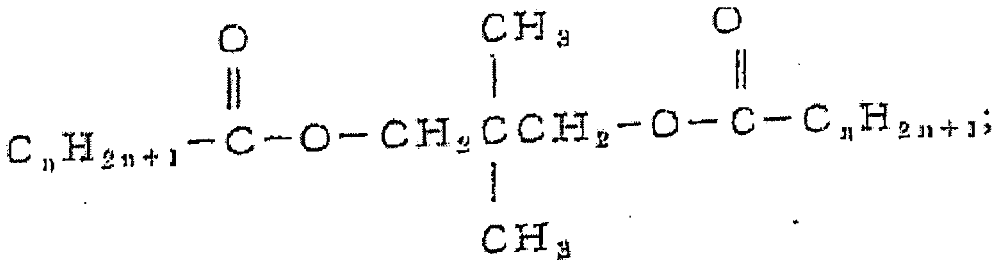

dans laquelle n est l'un quelconque de 7, 8 ou 9 et
dans laquelle un antioxydant à base de phénol inhibé comprenant au moins un (3,5-di-tert-butyl-4-hydroxyphenyl) qui est en outre inclut dans sa structure, et
dans laquelle un triglycéride représenté par la structure

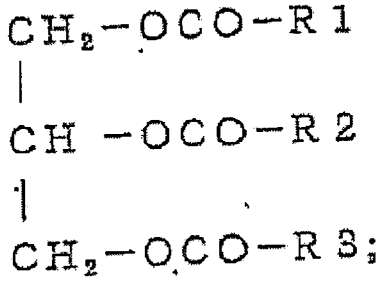

est également inclut ;
dans laquelle R1, R2 et R3 sont des structures en chaîne rectiligne ou des structures ramifiées insaturées ou saturées formées de CxHyOz ayant des valeurs entières de x dans l'intervalle de 15 à 21, y dans l'intervalle de 29 à 43 et z dans l'intervalle de 0 à 1.

**2.** La composition lubrifiante selon la revendication 1, dans laquelle, lorsqu'une huile de base contenue dans le lubrifiant est analysée au moyen de spectrométrie de masse à ion secondaire, l'intensité d'ion secondaire a des pics à « 127 » « 213 » et « 357 » des nombres de masse d'ions positifs secondaires par unité de charge.

**3.** La composition lubrifiante selon la revendication 1, dans laquelle, lorsqu'une huile de base contenue dans le lubrifiant est analysée au moyen de spectrométrie de masse à ion secondaire, l'intensité d'ion secondaire a des pics à « 141 » « 227 » et « 385 » des nombres de masse d'ions positifs secondaires par unité de charge.

**4.** La composition lubrifiante selon la revendication 1, dans laquelle, lorsqu'une huile de base contenue dans le lubrifiant est analysée au moyen de spectrométrie de masse à ion secondaire, l'intensité d'ion secondaire à des pics à « 155 » « 241 » et « 413 » des nombres de masse d'ions positifs secondaires par unité de charge.

**5.** La composition lubrifiante selon la revendication 1, dans laquelle le contenu dudit triglycéride n'est pas supérieur à 5% en poids de préférence pas supérieur à 3% en poids.

**6.** La composition lubrifiante selon l'une des revendications 1 et 2 à 4, dans laquelle les caractéristiques de viscosité ne sont pas supérieures à 48 mPa·s à 0°C et ne sont pas supérieures à 10 mPa·s à 40°C.

**7.** La composition lubrifiante selon l'une des revendications 1 et 2 à 4, qui remplit un espace entre les surfaces en regard l'une de l'autre de deux éléments se déplaçant l'un relativement à l'autre.

**8.** La composition lubrifiante selon l'une des revendications 1 et 2 à 4, qui remplit un espace entre les surfaces en regard l'une de l'autre d'une partie d'un axe rotatif et une partie d'un palier dans un palier dynamique à pression hydraulique dans lequel ledit axe rotatif est en contact avec ladite partie de palier pour être librement rotatif et dans lequel une tranchée à génération dynamique de pression est crée dans au moins l'une des deux surfaces en regard l'une de l'autre de ladite partie d'axe rotatif et ladite partie de palier.

**9.** Palier hydraulique à pression dynamique comprenant une partie de palier et une partie d'axe rotatif engagés l'un dans l'autre pour être librement rotatifs, une gorge à génération de pression dynamique créée dans au moins l'une des deux surfaces en regard l'une de l'autre qui forme un intervalle entre la partie de palier et la partie d'axe rotatif et une composition lubrifiante remplissant l'intervalle, dans lequel une composition lubrifiante selon l'une quelconque des revendications 1 à 8 est utilisée en tant que composition lubrifiante.

**10.** Un moteur comprenant :

une partie de base,
un stator ayant un aimant rotatif opposé au stator ;
une partie d'axe rotatif prévue dans le rotor ;
une partie de palier qui est prévue dans la partie de base et avec laquelle la partie d'axe rotatif est en engagement pour être librement rotative ;
une gorge à génération de pression dynamique créée dans au moins une des deux surfaces en regard l'une de l'autre qui forment un intervalle entre la partie de palier et la partie d'axe rotatif ; et une composition lubrifiante remplissant l'intervalle, dans lequel une composition lubrifiante selon l'une des revendications 1 à 8 est utilisée en tant que composition lubrifiante.

**11.** Moteur selon la revendication 10, dans lequel ladite partie de palier du côté de la partie de base est de forme cylindrique et ladite partie d'axe rotatif du côté du rotor est engagée à l'intérieur de ladite partie de palier.

**12.** Moteur selon la revendication 10, dans lequel ladite partie d'axe rotatif du côté du rotor est de forme cylindrique et ladite partie d'axe rotatif est engagée sur l'extérieur de ladite partie de palier sur le côté de la partie de base.

**13.** Moteur selon la revendication 10 qui a une partie de palier radiale, dans lequel ladite partie de palier et ladite partie d'axe rotatif sont en regard l'une de l'autre dans la direction radiale et ladite composition lubrifiante remplit l'intervalle entre ladite partie de palier et ladite partie d'axe rotatif et dans lequel une tranchée à génération de pression dynamique est créée dans l'un au moins des deux faces qui forment l'intervalle dans ladite partie de palier radiale.

**14.** Moteur selon la revendication 10, qui a une partie de palier de poussée, dans lequel ladite partie de palier et ladite partie d'axe rotatif sont en regard l'une de l'autre et dans lequel une tranchée à génération de pression dynamique est créée dans l'une au moins des deux faces qui forment l'intervalle dans ladite partie de palier de poussée.

**15.** Moteur selon la revendication 11, dans lequel une partie de palier de poussée est formée par une surface d'extrémité sur le côté ayant une ouverture de ladite partie cylindrique du palier et par une région annulaire dans ledit rotor qui est opposée à la surface d'extrémité sur le côté ayant l'ouverture de ladite partie de palier et dans lequel une tranchée à génération de pression dynamique est créée dans au moins l'une de ladite surface d'extrémité sur le côté ayant une ouverture et ladite région annulaire opposée à cette surface d'extrémité dans ladite partie de palier de poussée.

**16.** Moteur selon la revendication 12, dans lequel une partie de palier de poussée est formée par une surface d'extrémité sur le côté ayant une ouverture de ladite partie cylindrique de l'axe rotatif et par une région annulaire dans ladite partie de base qui est opposée à la surface d'extrémité sur le côté ayant l'ouverture de ladite partie d'axe rotatif et dans lequel une tranchée à génération de pression dynamique est créée dans au moins l'une de ladite surface d'extrémité sur le côté ayant une ouverture et ladite région annulaire opposée à cette surface d'extrémité dans ladite partie de palier de poussée.

**17.** Moteur selon la revendication 10, dans lequel un film de placage de nickel phosphoreux est formé sur les deux surfaces en regard l'une de l'autre qui forment un intervalle entre ladite partie du palier et ladite partie d'axe rotatif.

**18.** Moteur selon la revendication 17, dans lequel ledit film de placage de nickel phosphoreux est un dépôt de film de placage non électrique dont la concentration en phosphore n'est pas supérieure à 15% en poids.

**19.** Dispositif intégrant un moteur dans lequel est monté un moteur selon la revendication 10.

F I G. 1

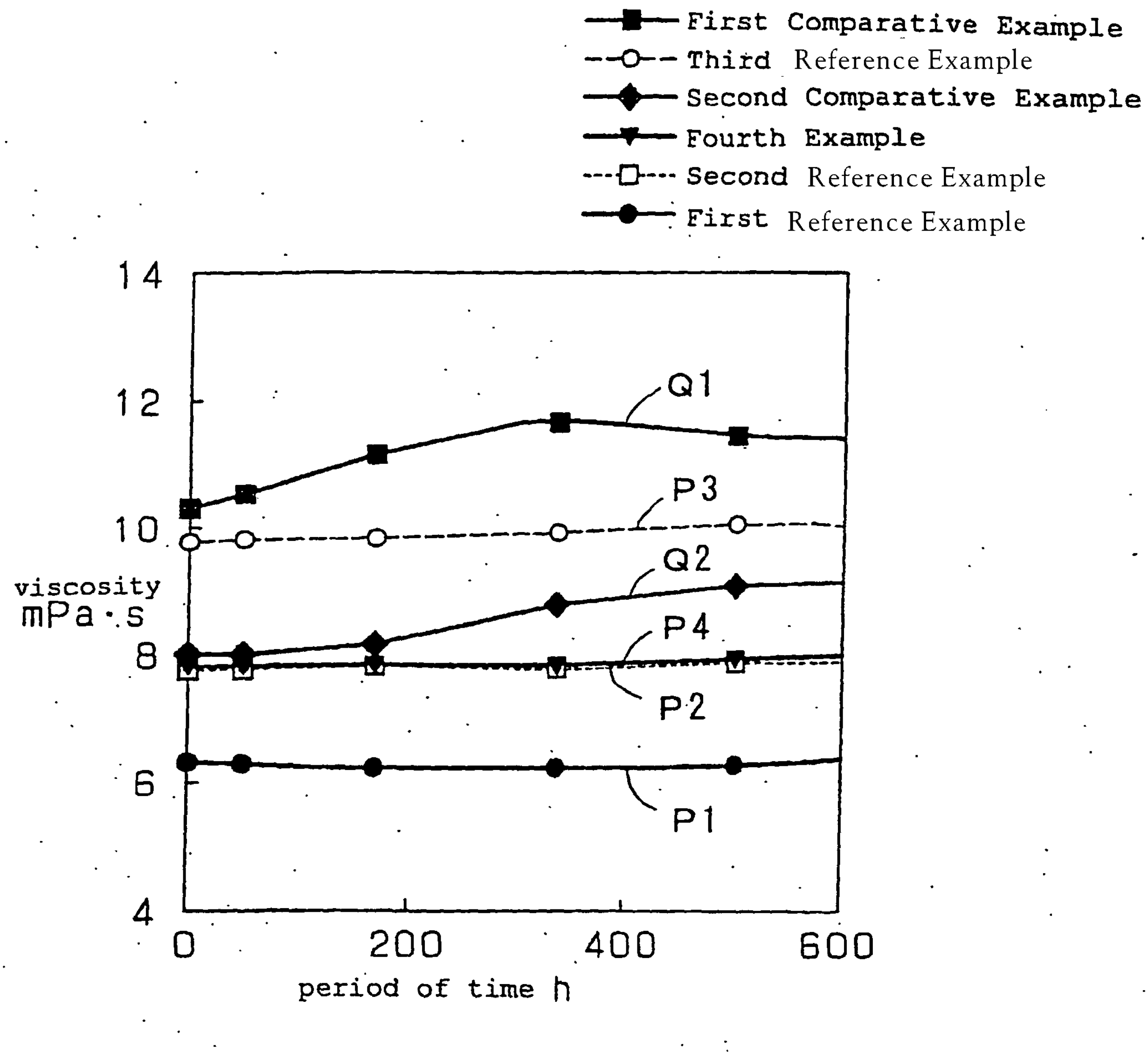
First Comparative Example
Third Reference Example
Second Comparative Example
Fourth Example
Second Reference Example
First Reference Example
14
12
10
8
6
4
viscosity
mPa·s
0
200
400
600
period of time h
Q1
P3
Q2
P4
P2
P1

FIG. 2

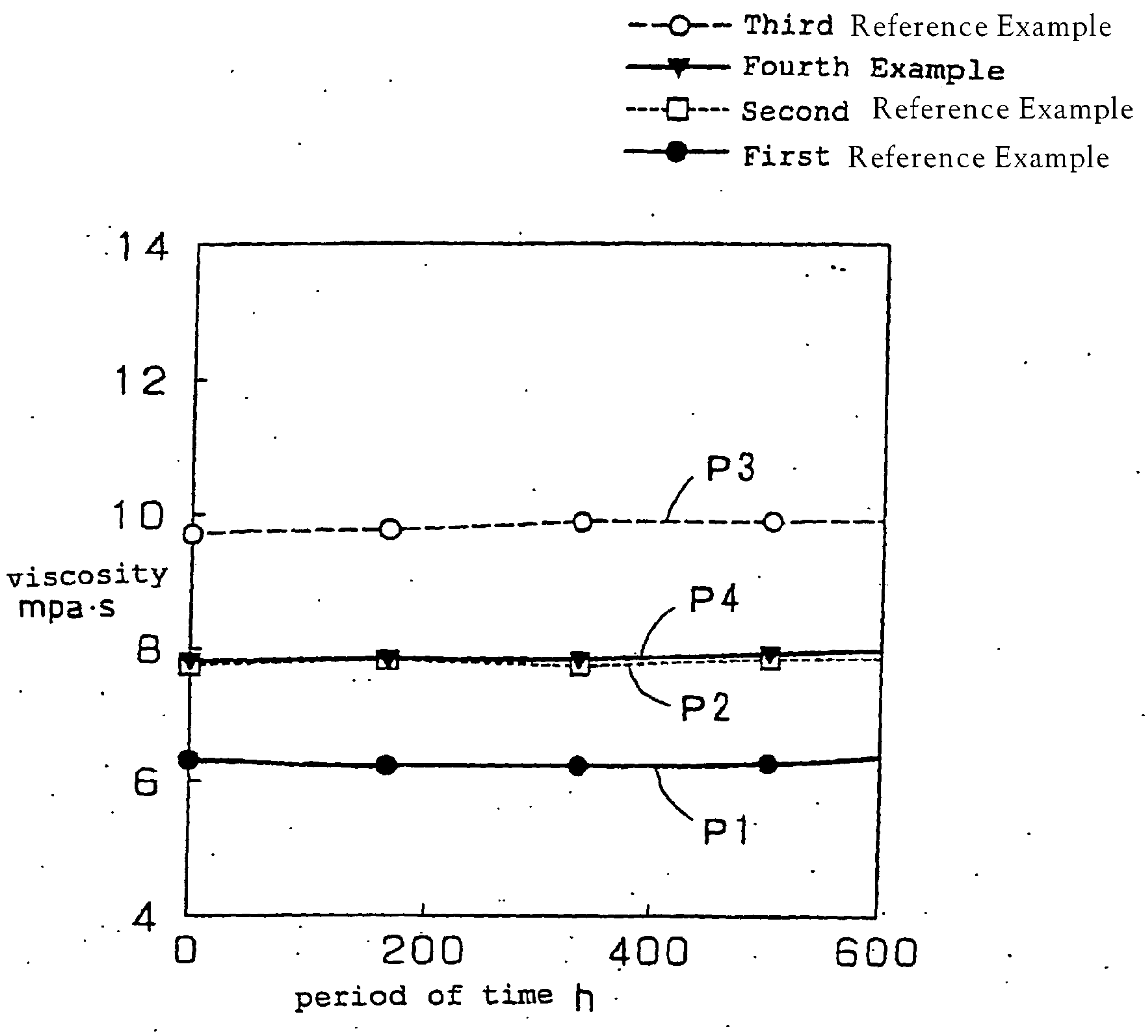
Third Reference Example
Fourth Example
Second Reference Example
First Reference Example
14
12
10
8
6
4
viscosity
mpa·s
P3
P4
P2
P1
0
200
400
600
period of time h

F I G. 3

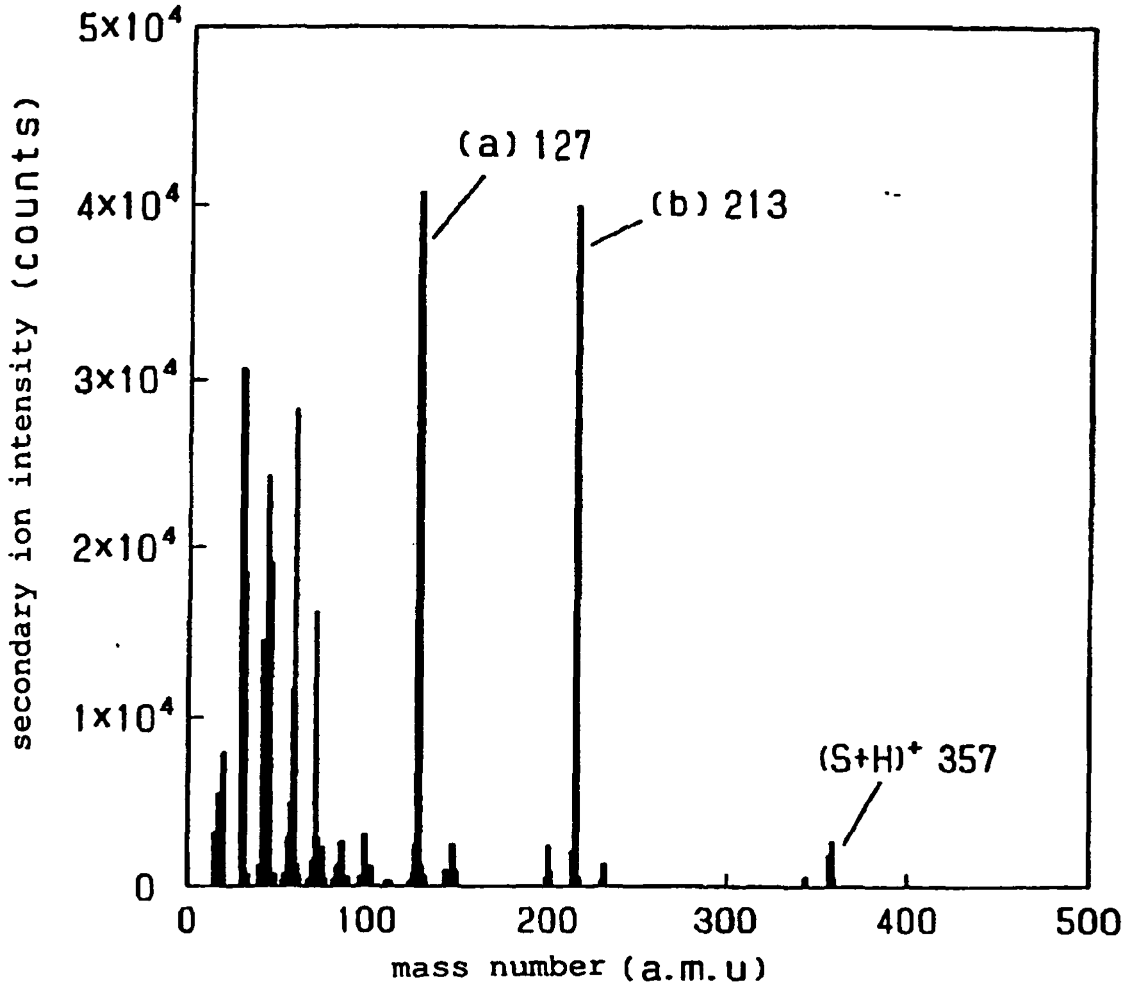

secondary ion intensity (counts)
5x10^4
4x10^4
3x10^4
2x10^4
1x10^4
0
(a) 127
(b) 213
(S+H)+ 357
0
100
200
300
400
500
mass number (a.m.u)

FIG. 4

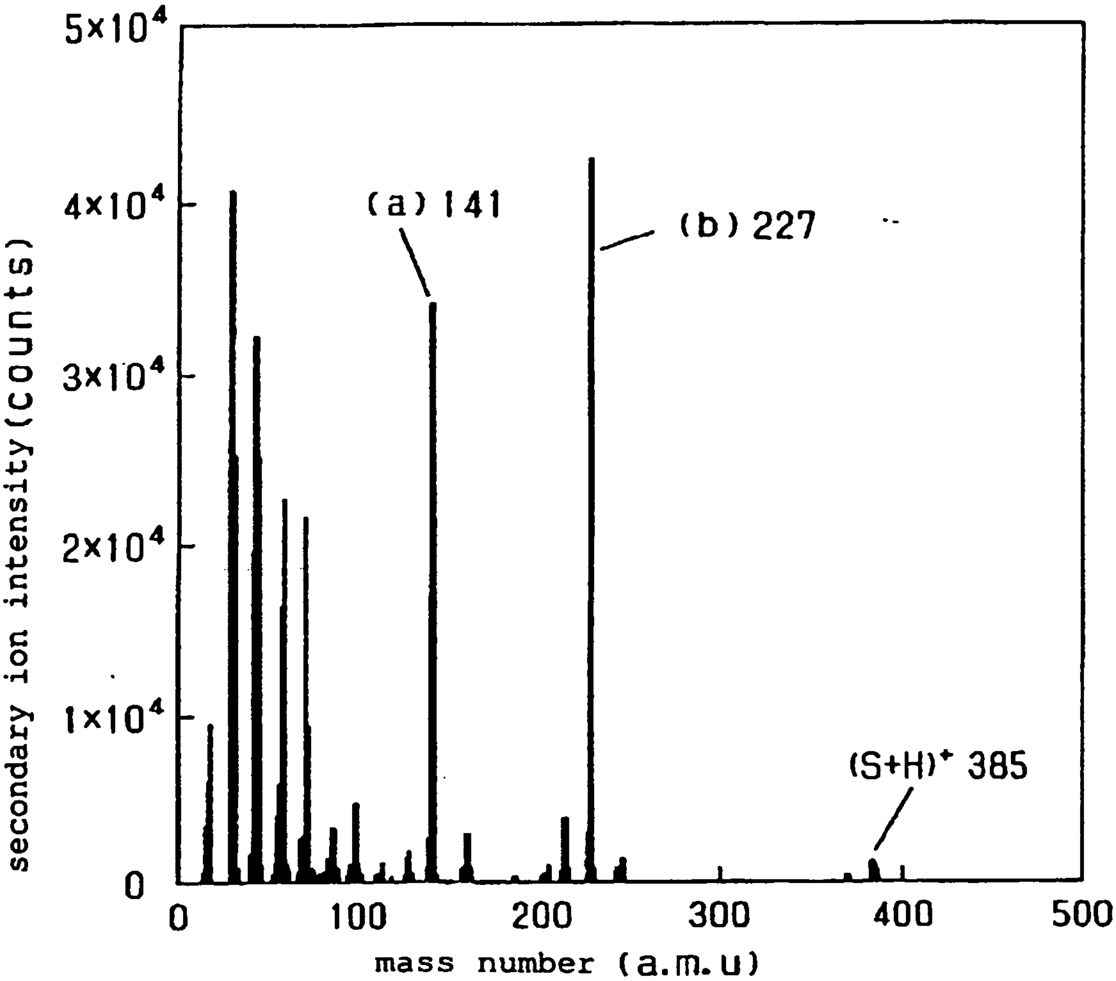
5×10^4
4×10^4
3×10^4
2×10^4
1×10^4
0
secondary ion intensity (counts)
(a) 141
(b) 227
(S+H)+ 385
0
100
200
300
400
500
mass number (a.m.u)

FIG. 5

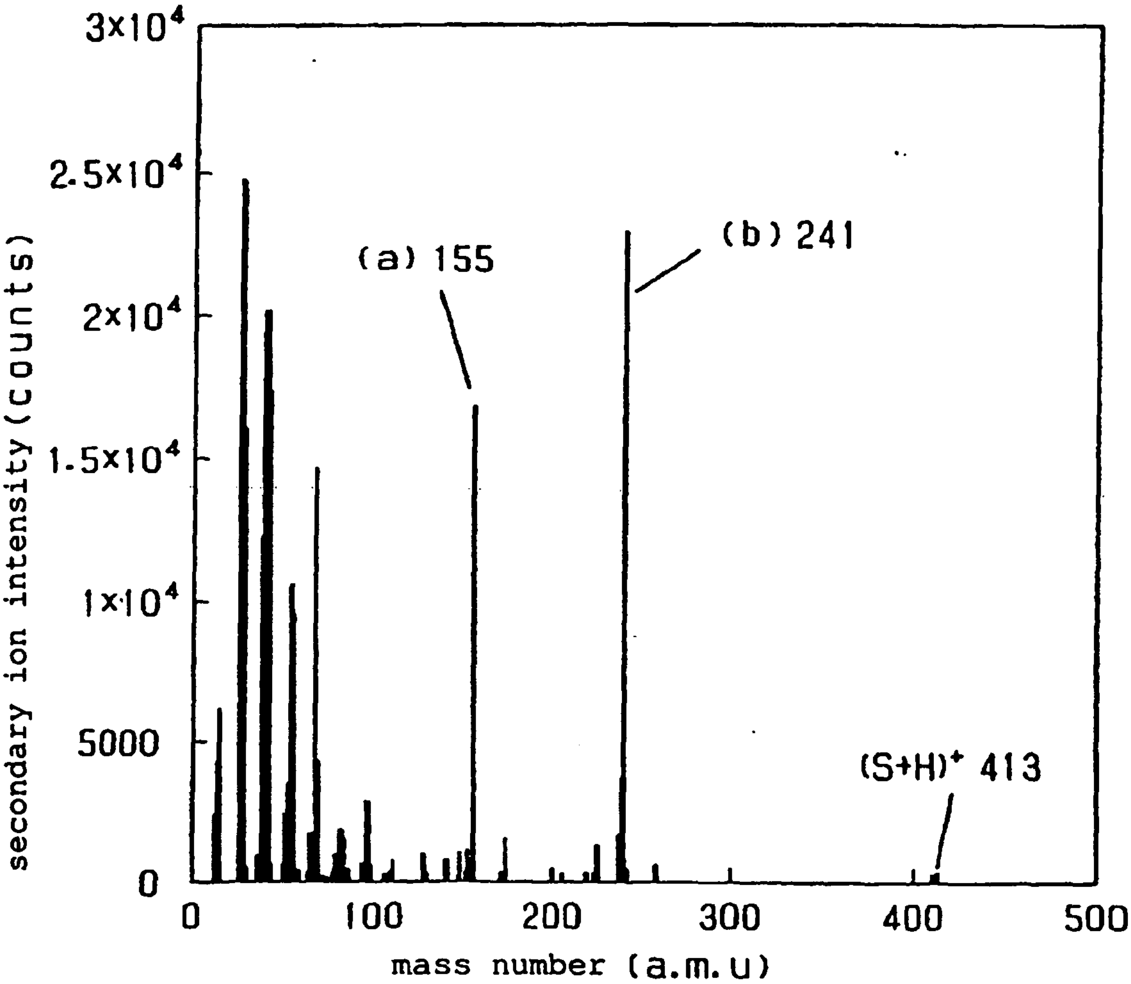
3x10⁴
2.5x10⁴
2x10⁴
1.5x10⁴
1x10⁴
5000
0
secondary ion intensity (counts)
(a) 155
(b) 241
(S+H)⁺ 413
0
100
200
300
400
500
mass number (a.m.u)

FIG. 6

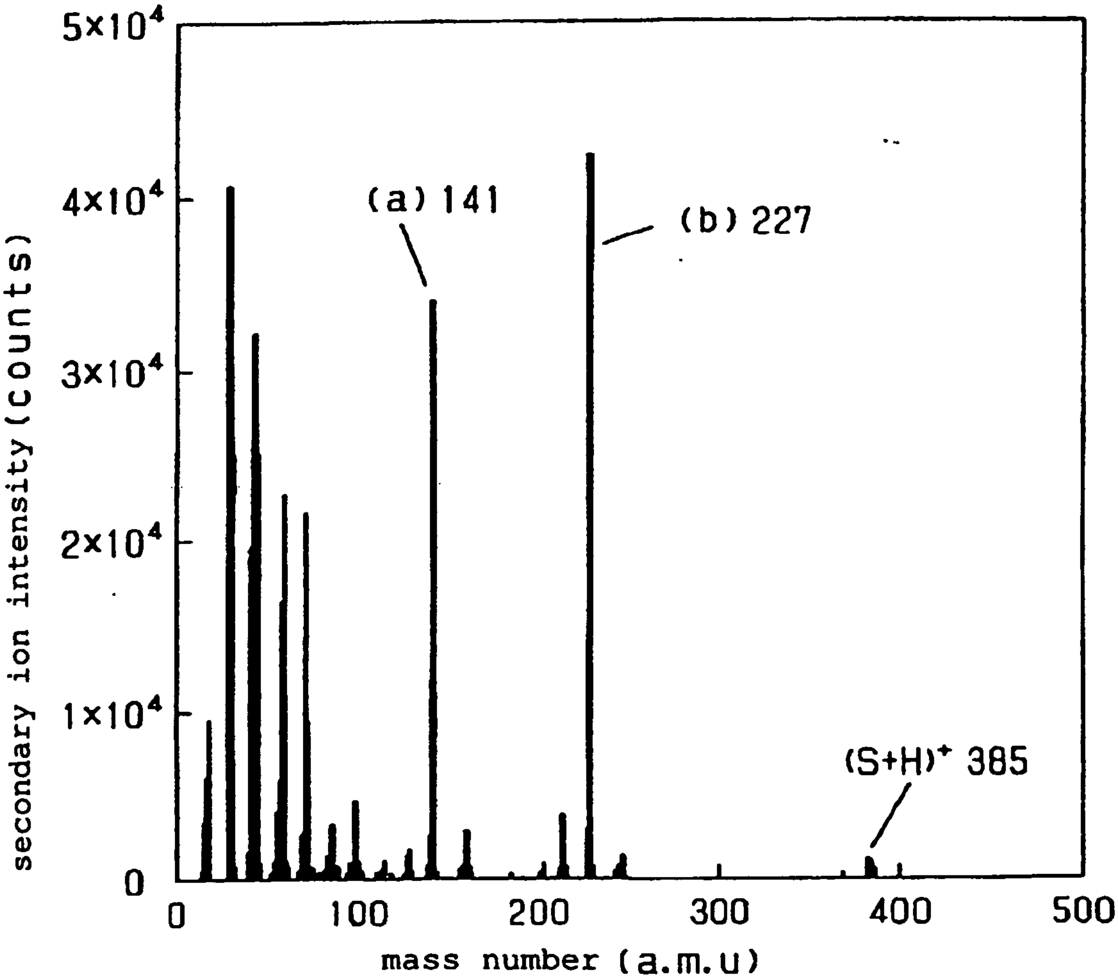
5×10⁴
4×10⁴
3×10⁴
2×10⁴
1×10⁴
0
secondary ion intensity (counts)
(a) 141
(b) 227
(S+H)⁺ 385
0
100
200
300
400
500
mass number (a.m.u)

FIG. 7

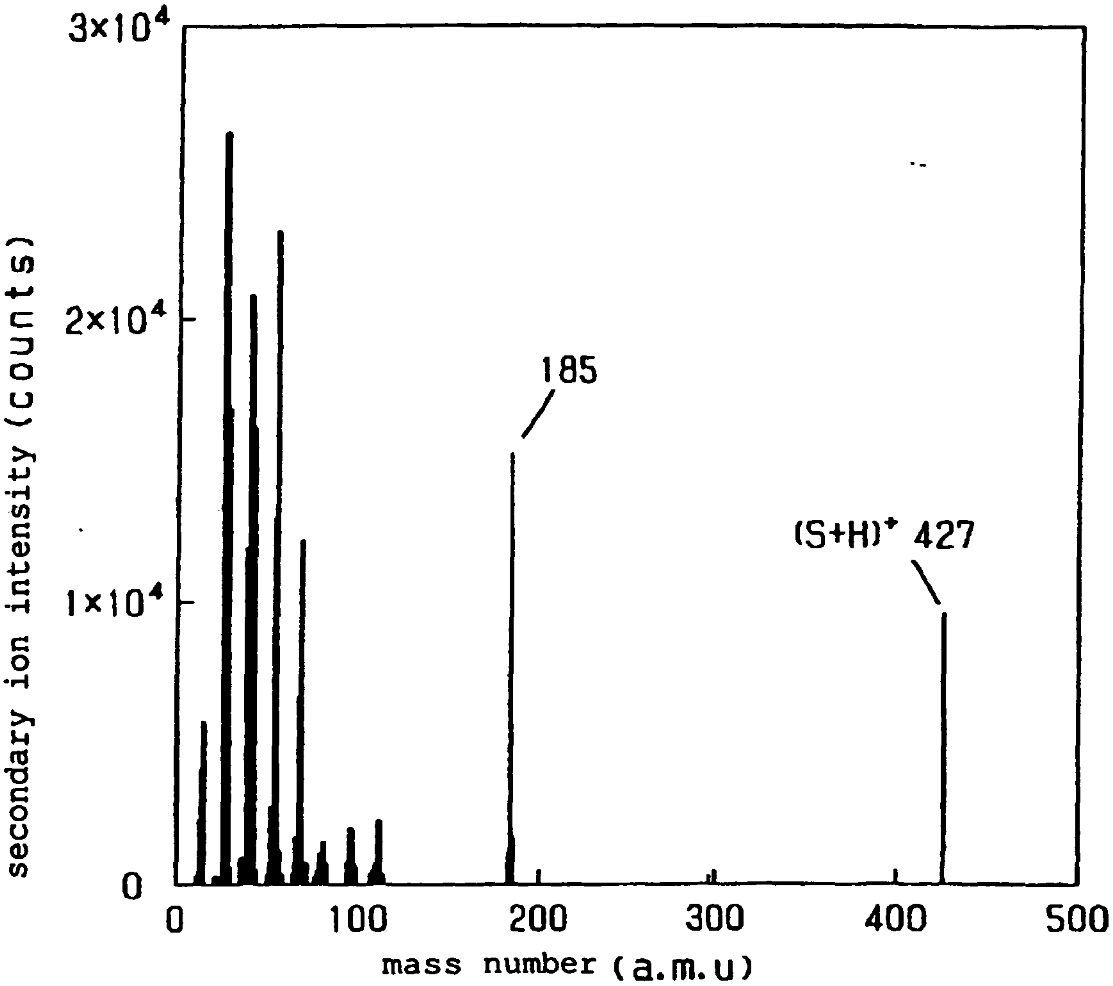
3×10^4
2×10^4
1×10^4
0
secondary ion intensity (counts)
185
(S+H)+ 427
0
100
200
300
400
500
mass number (a.m.u)

F I G. 8

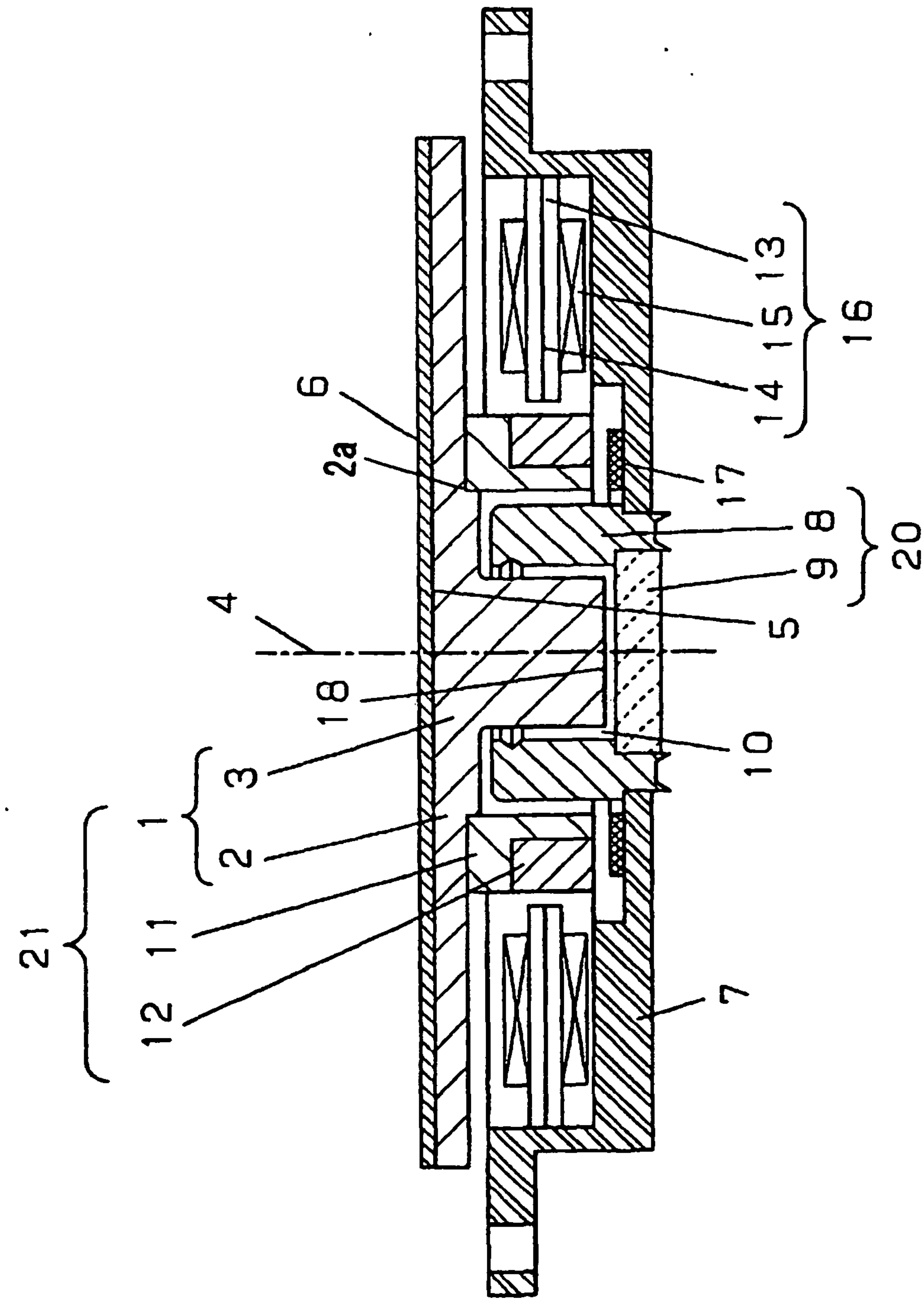

FIG. 9

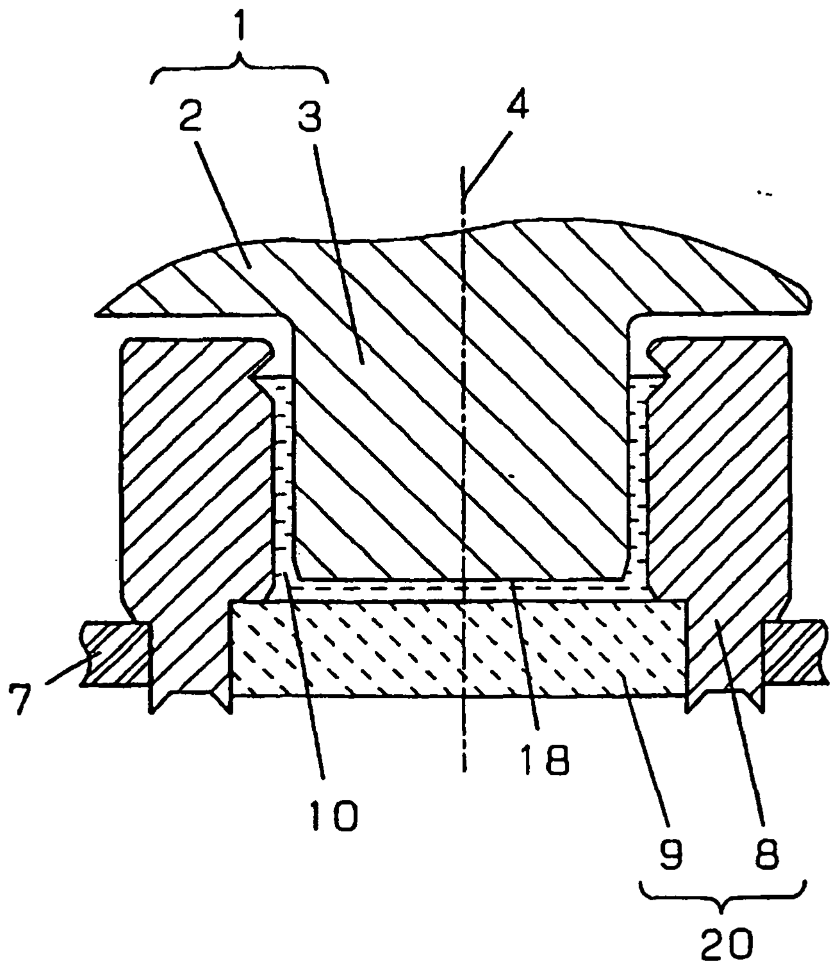

FIG. 10

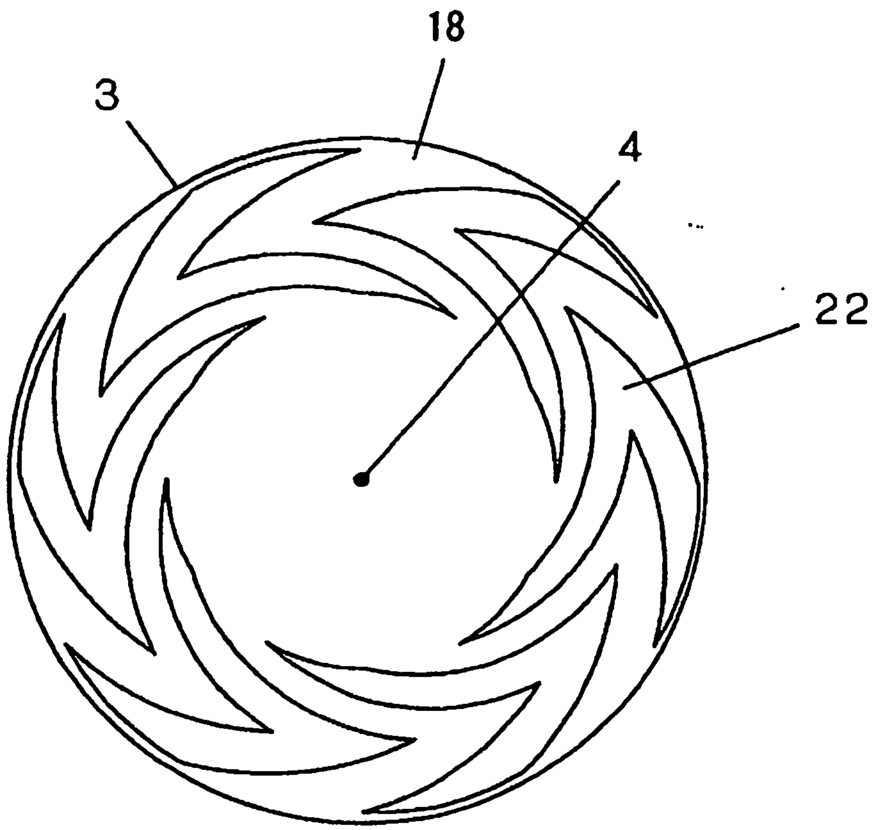

FIG. 11

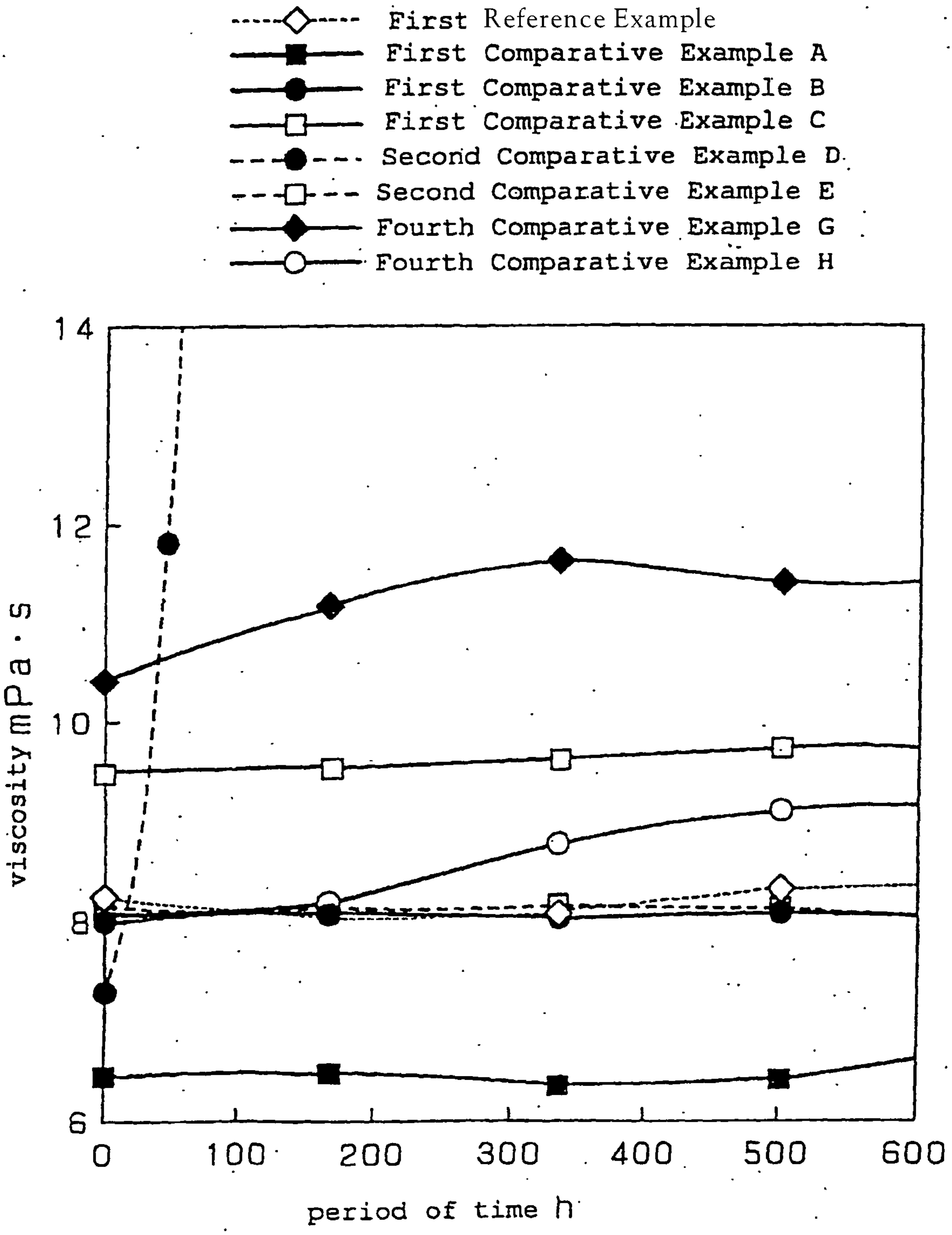
First Reference Example
First Comparative Example A
First Comparative Example B
First Comparative Example C
Second Comparative Example D
Second Comparative Example E
Fourth Comparative Example G
Fourth Comparative Example H
14
12
10
8
6
viscosity mPa・s
0
100
200
300
400
500
600
period of time h

FIG. 12A

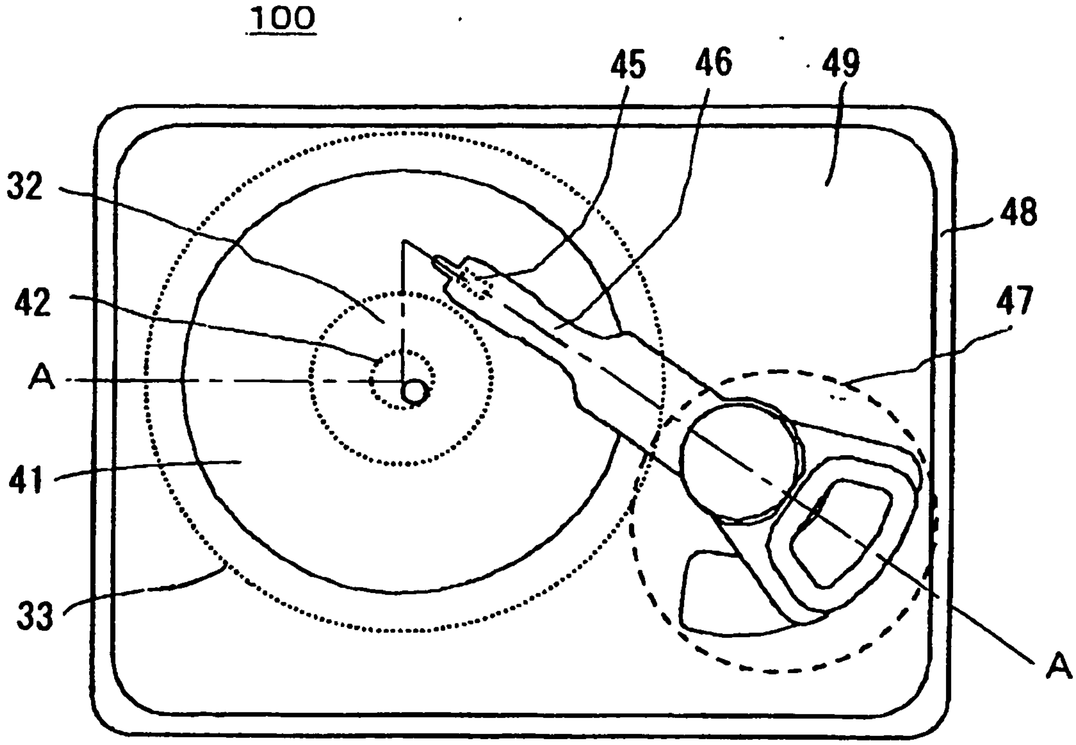


FIG. 12B

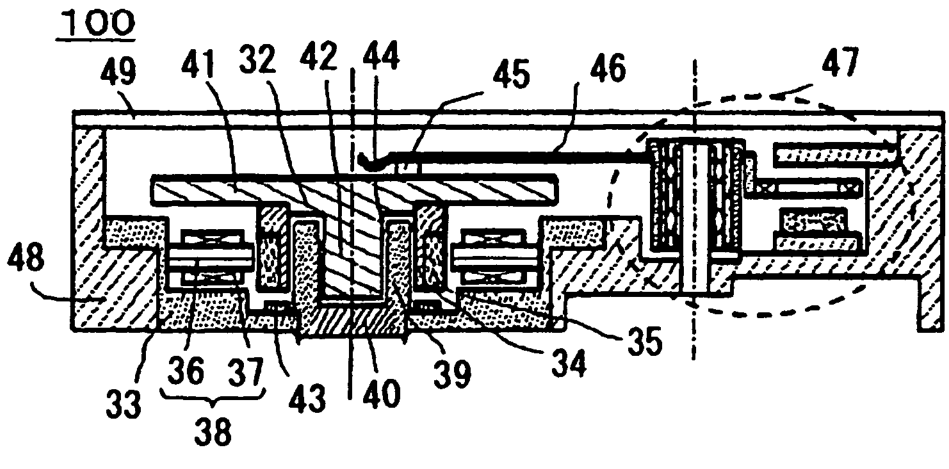

FIG. 13

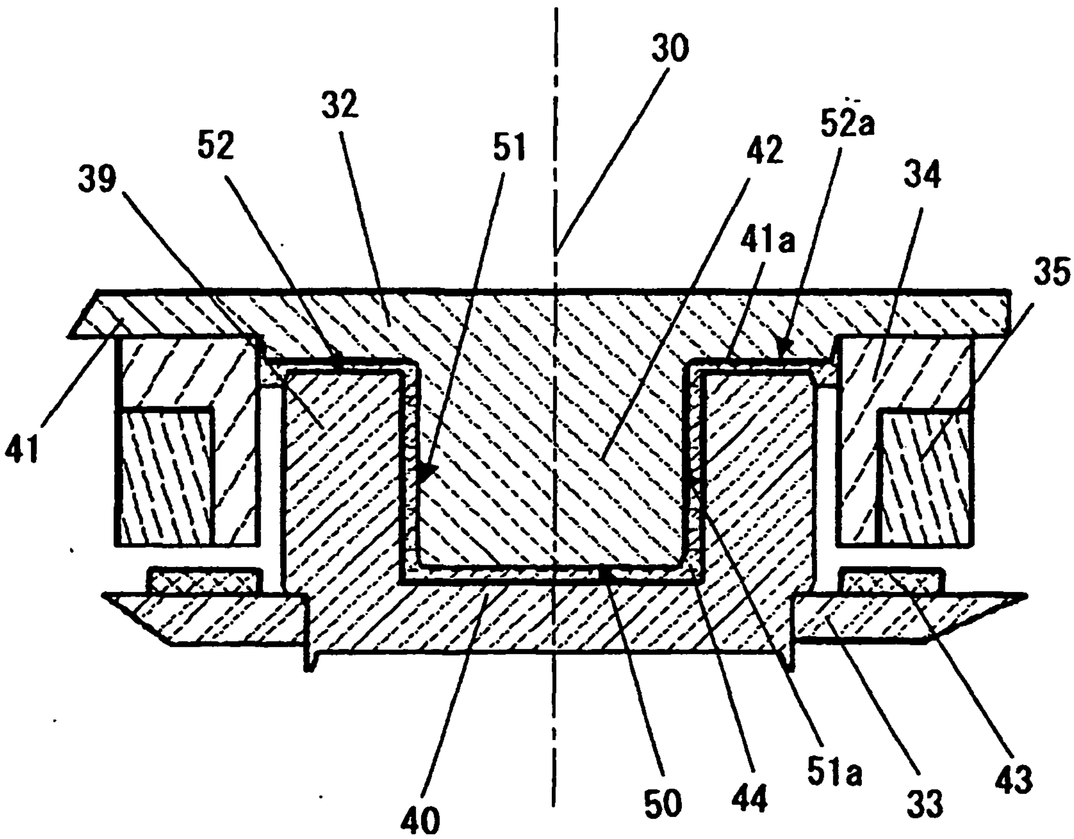

FIG. 14A

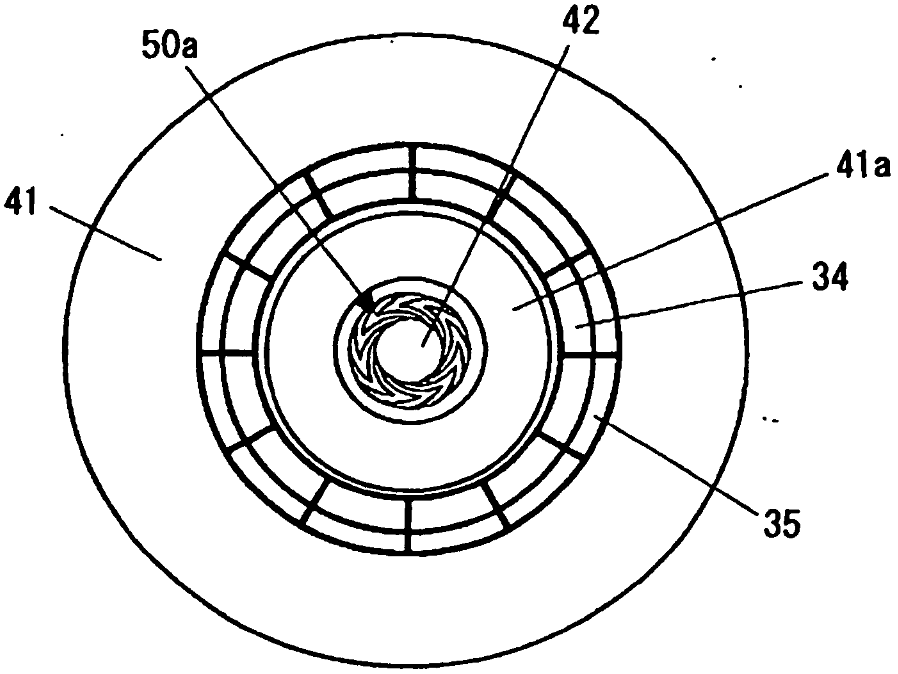


FIG. 14B

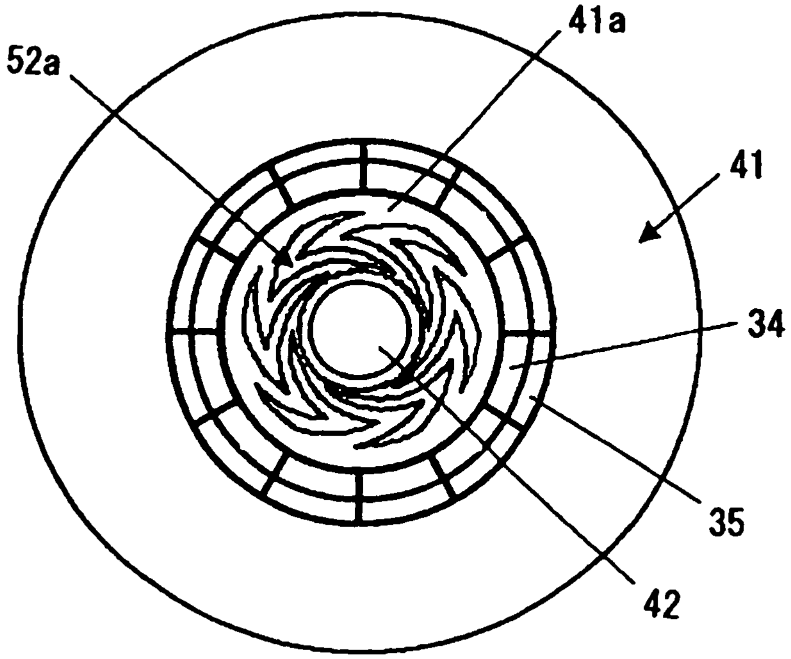

FIG. 15

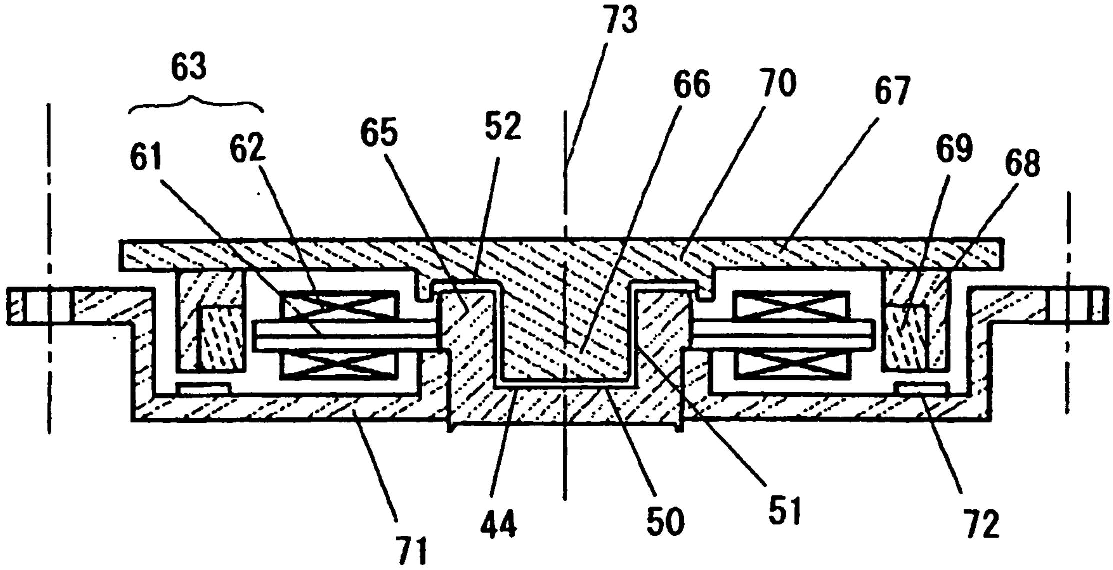
63
61
62
65
52
73
66
70
67
69
68
71
44
50
51
72

FIG. 16

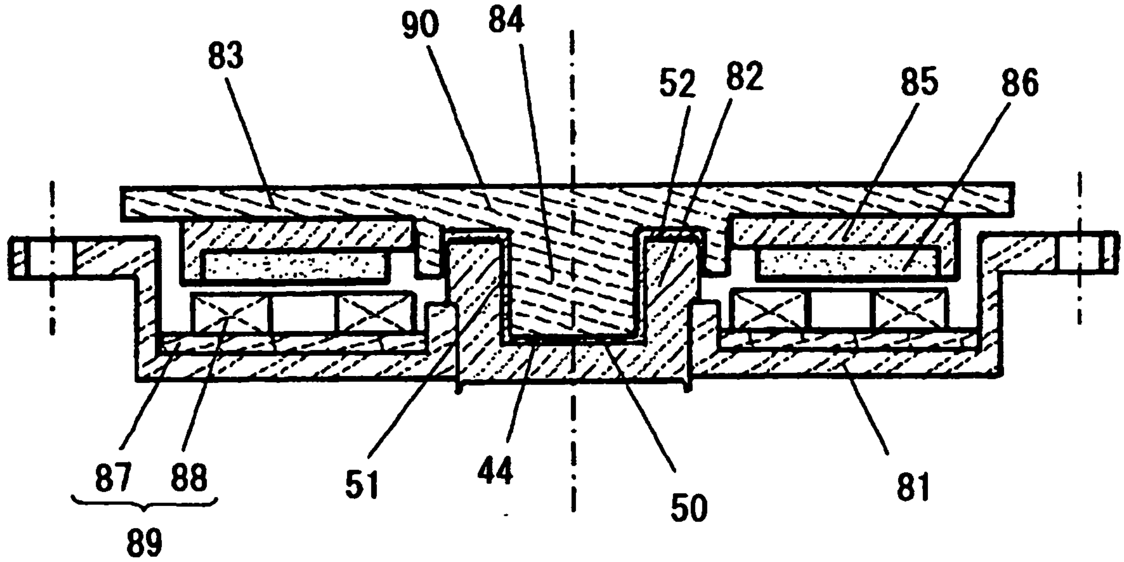
83
90
84
52
82
85
86
87
88
89
51
44
50
81

FIG. 17

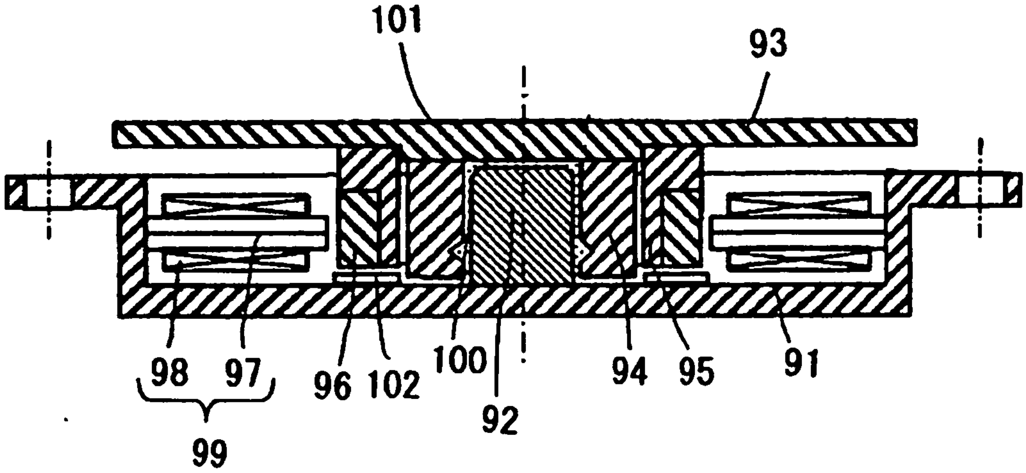

FIG. 18A

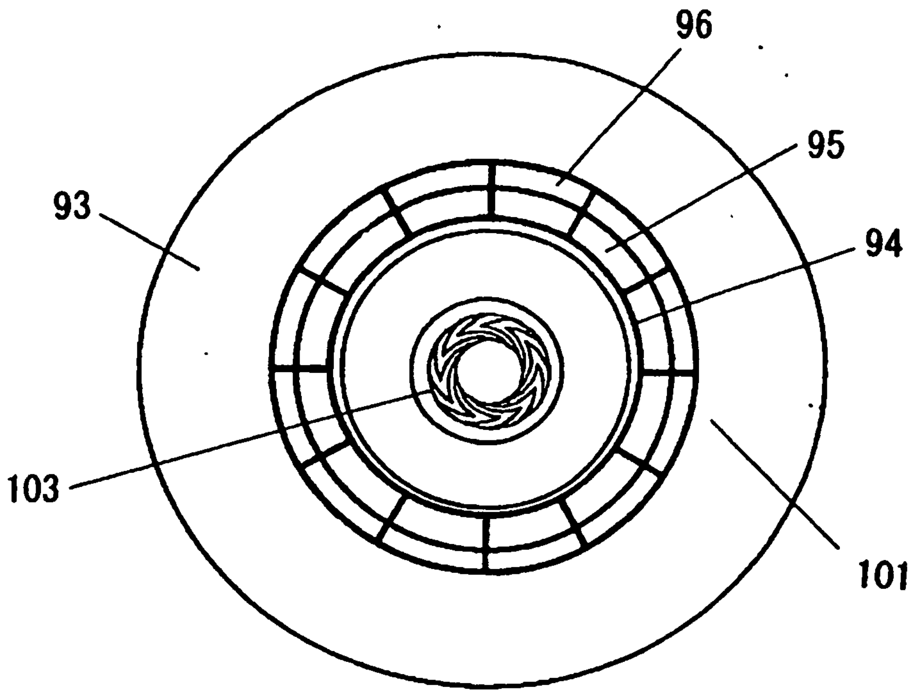


FIG. 18B

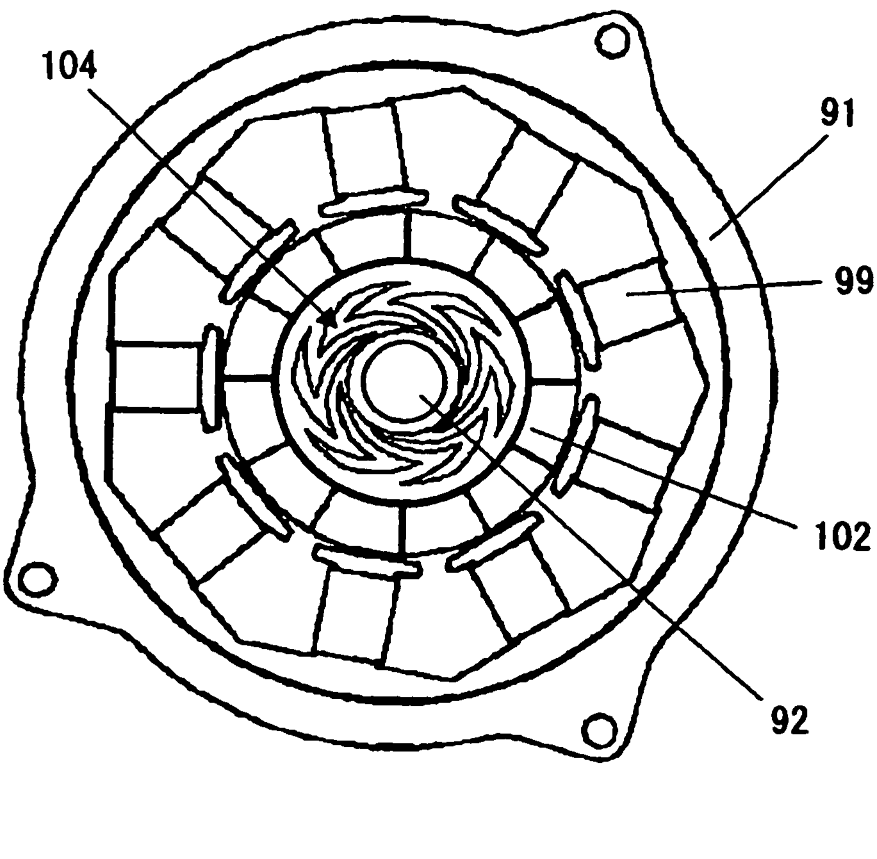

FIG. 19

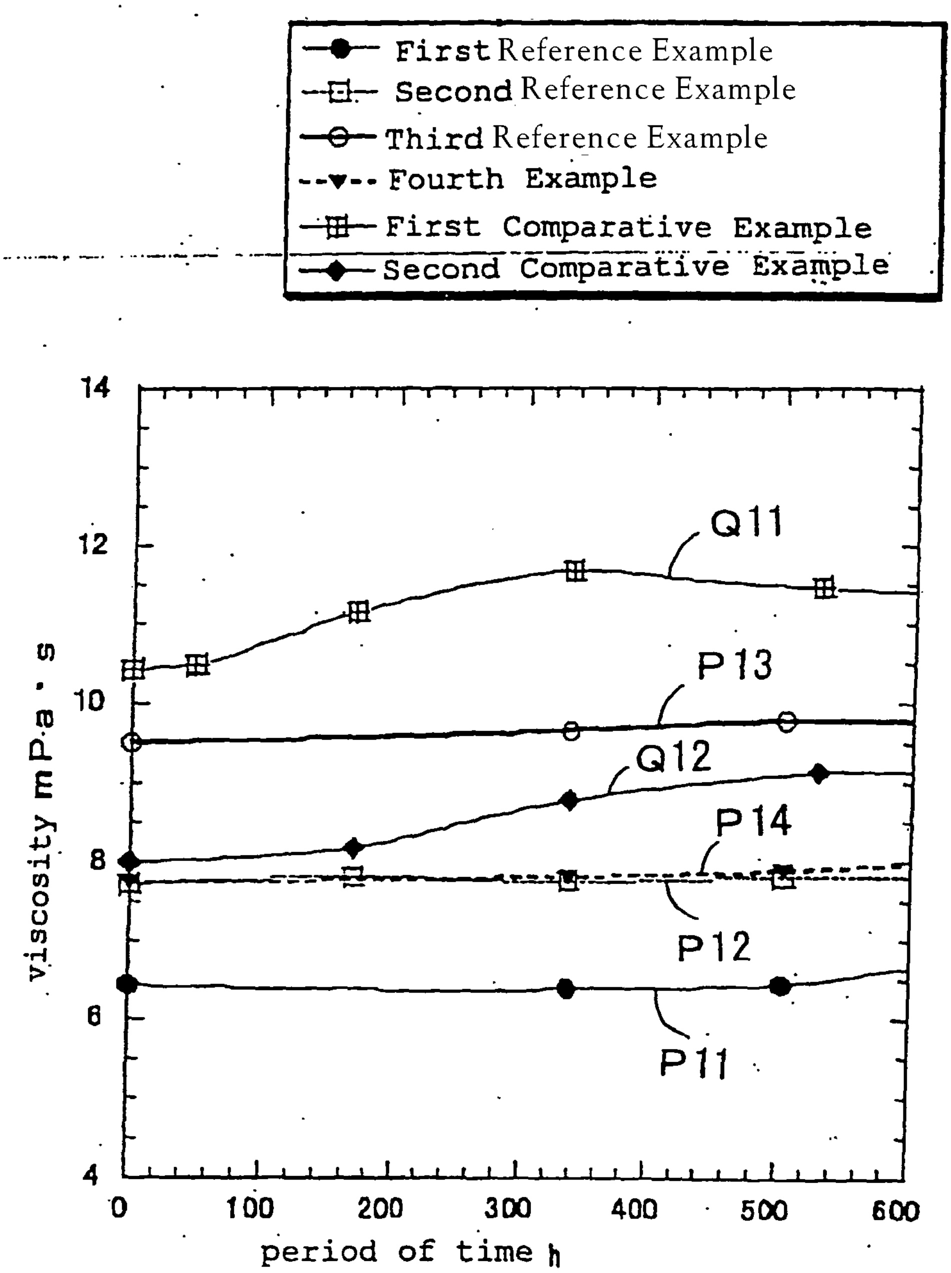
First Reference Example
Second Reference Example
Third Reference Example
Fourth Example
First Comparative Example
Second Comparative Example
14
12
10
8
6
4
0
100
200
300
400
500
600
viscosity mPa・s
period of time h
Q11
P13
Q12
P14
P12
P11

FIG. 20

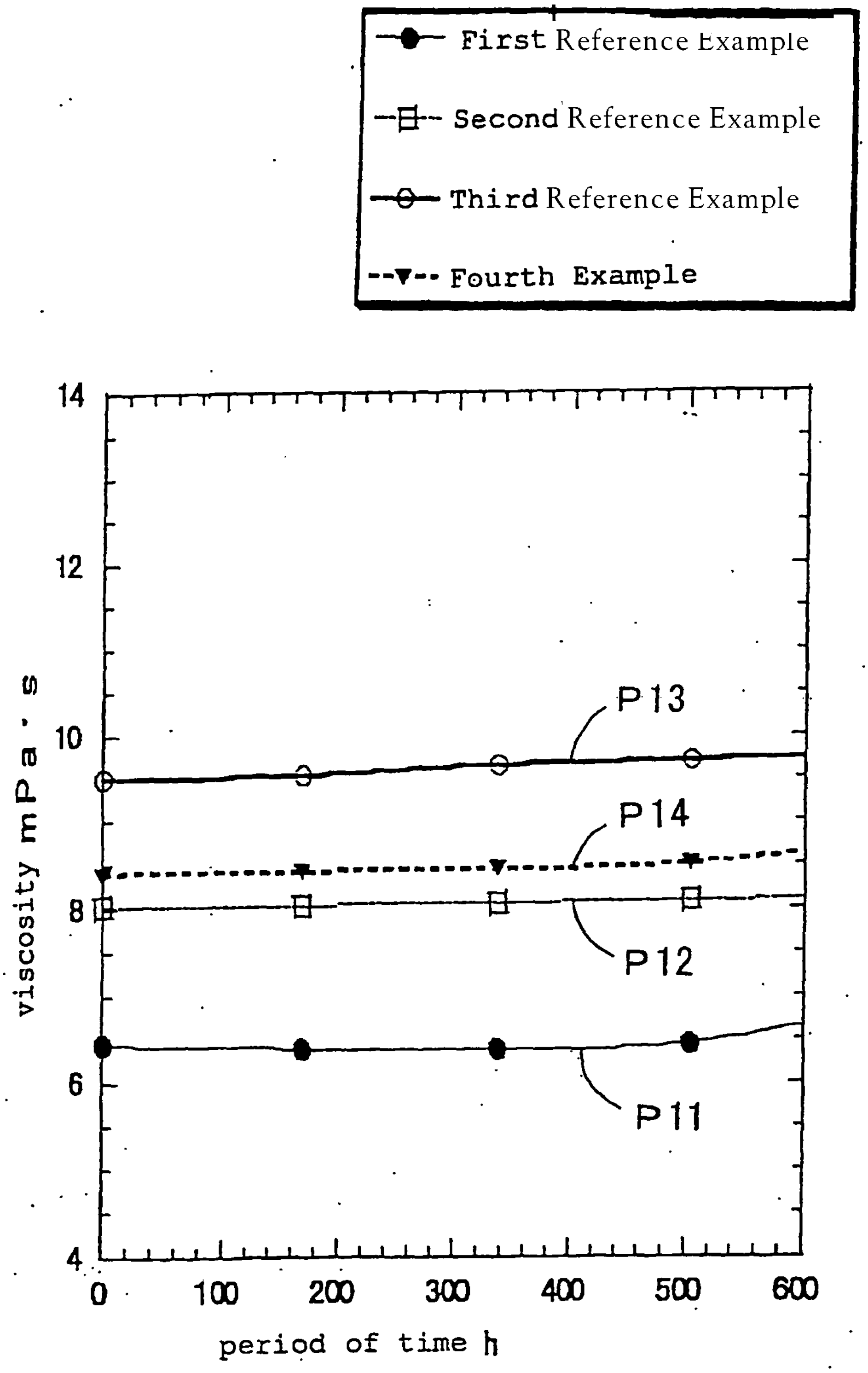
First Reference Example
Second Reference Example
Third Reference Example
Fourth Example
14
12
10
8
6
4
0
100
200
300
400
500
600
viscosity mPa · s
period of time h
P13
P14
P12
P11

FIG. 21

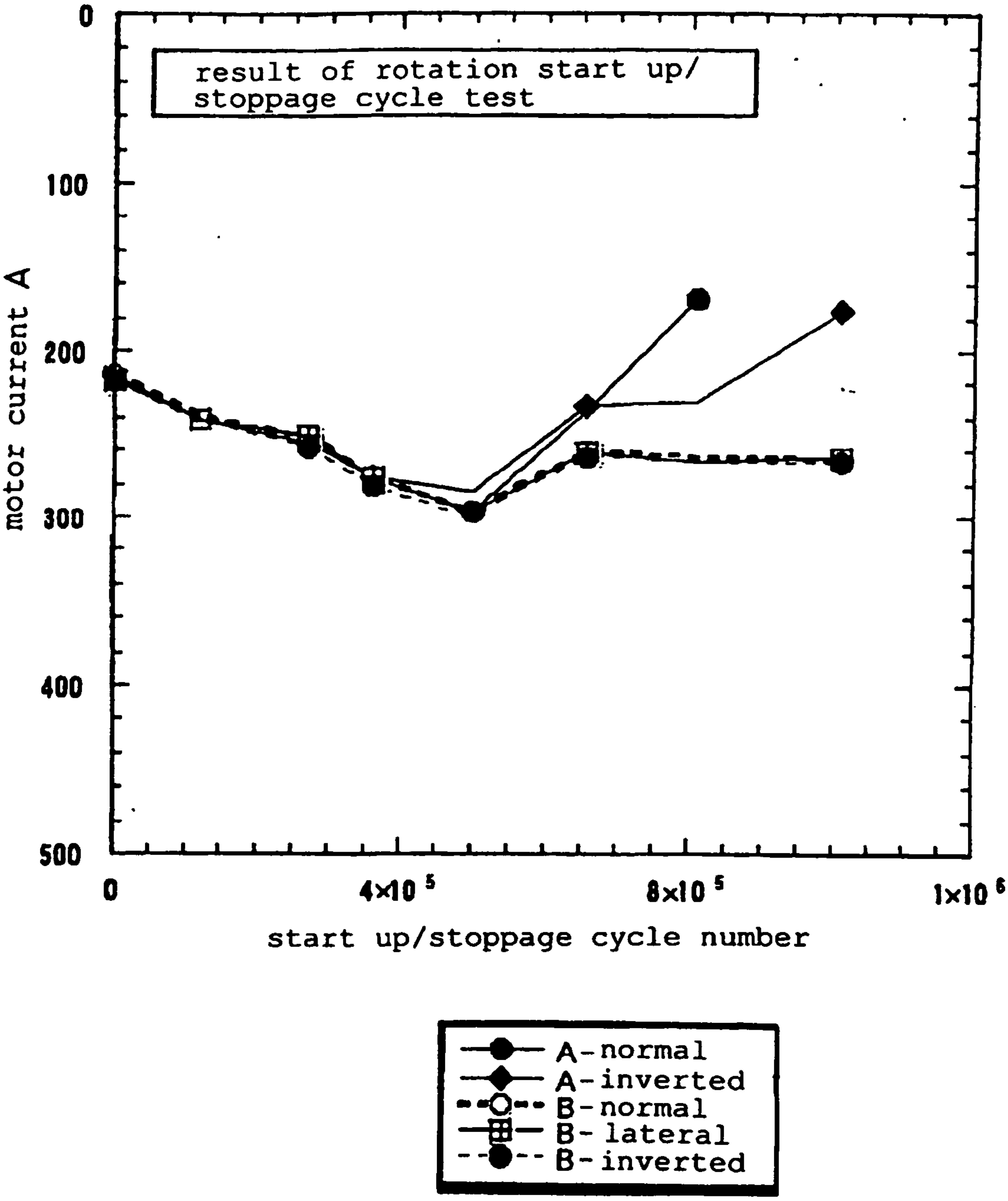


A: no triglyceride added
B: 1 % of triglyceride added
C: normal, lateral and inverted indicate positions in which motor is placed

FIG. 22A

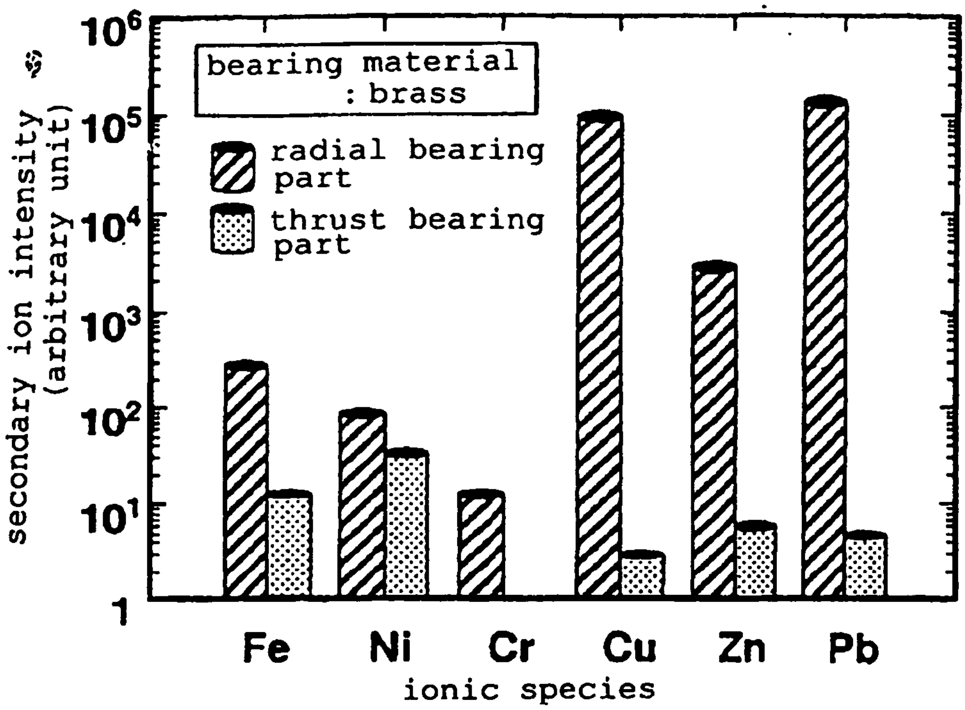


FIG. 22B